(19) Europäisches Patentamt
European Patent Office
Office européen des brevets

(11) EP 2 204 136 B1

(12) **EUROPEAN PATENT SPECIFICATION**

(45) Date of publication and mention of the grant of the patent:
**28.08.2013 Bulletin 2013/35**

(21) Application number: **10004337.1**

(22) Date of filing: **13.04.2001**

(51) Int Cl.:
***A61C 7/00*** *(2006.01)*
***A61C 7/20*** *(2006.01)*
***A61C 7/02*** *(2006.01)*

(54) **Orthodontic archwire**

Orthodontischer Bogendraht

Arc orthodontique

(84) Designated Contracting States:
**AT BE CH CY DE DK ES FI FR GB GR IE IT LI LU MC NL PT SE TR**

(30) Priority:
**19.04.2000 US 552189**
**19.04.2000 US 552190**
**28.04.2000 US 560127**
**28.04.2000 US 560128**
**28.04.2000 US 560129**
**28.04.2000 US 560130**
**28.04.2000 US 560131**
**28.04.2000 US 560132**
**28.04.2000 US 560133**
**28.04.2000 US 560134**
**28.04.2000 US 560583**
**28.04.2000 US 560584**
**28.04.2000 US 560640**
**28.04.2000 US 560641**
**28.04.2000 US 560642**
**28.04.2000 US 560643**
**28.04.2000 US 560644**
**28.04.2000 US 560645**
**28.04.2000 US 560646**
**28.04.2000 US 560647**
**13.07.2000 US 616093**

(43) Date of publication of application:
**07.07.2010 Bulletin 2010/27**

(62) Document number(s) of the earlier application(s) in accordance with Art. 76 EPC:
**01928490.0 / 1 301 140**

(73) Proprietor: **OraMetrix, Inc.**
**Richardson, TX 75082-4434 (US)**

(72) Inventors:
- **Rubbert, Rüdger**
  **12101 Berlin (DE)**
- **Weise, Thomas**
  **36284 Hohenroda (DE)**
- **Riemeier, Friedrich**
  **10557 Berlin (DE)**
- **Sachdeva, Rohit**
  **Plano**
  **Texas 75093 (US)**
- **Butscher, Werner**
  **13467 Berlin (DE)**

(74) Representative: **Stevens, Jason Paul**
**Dehns**
**St Bride's House**
**10 Salisbury Square**
**London**
**EC4Y 8JD (GB)**

(56) References cited:
**EP-A2- 0 778 008**
**US-A- 4 043 364**
**US-A- 4 656 860**
**US-A- 4 945 747**
**US-A- 5 017 133**
**US-A- 5 100 316**
**US-A- 5 447 432**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 204 136 B1

**Description**

BACKGROUND OF THE INVENTION

A. Field of the Invention

This invention relates generally to the field of orthodontics. More particularly, the invention relates to a computerized, interactive method and associated system for orthodontic treatment. The system includes a hand-held optical scanner capturing 3-dimensional information of objects, interactive computer-based treatment planning using three-dimensional tooth objects and user specified simulation of tooth movement, and appliance manufacturing apparatus, including bending machines.

B. Description of Related Art

In orthodontics, a patient suffering from a malocclusion is typically treated by bonding brackets to the surface of the patient's teeth. The brackets have slots for receiving an archwire. The bracket-archwire interaction governs forces applied to the teeth and defines the desired, direction of tooth movement. Typically, the bends in the wire are made manually by the orthodontist. During the course of treatment, the movement of the teeth is monitored. Corrections to the bracket position and/or wire shape are made manually by the orthodontist.

The key to efficiency in treatment and maximum quality in results is a realistic simulation of the treatment process. Today's orthodontists have the possibility of taking plaster models of the upper and lower jaw, cutting the model into single tooth models and sticking these tooth models into a wax bed, lining them up in the desired position, the so-called set-up. This approach allows for reaching a perfect occlusion without any guessing. The next step is to bond a bracket at every tooth model. This would tell the orthodontist the geometry of the wire to run through the bracket slots to receive exactly this result. The next step involves the transfer of the bracket position to the original malocclusion model. To make sure that the brackets will be bonded at exactly this position at the real patient's teeth, small templates for every tooth would have to be fabricated that fit over the bracket and a relevant part of the tooth and allow for reliable placement of the bracket on the patient's teeth. To increase efficiency of the bonding process, another option would be to place each single bracket onto a model of the malocclusion and then fabricate one single transfer tray per jaw that covers all brackets and relevant portions of every tooth. Using such a transfer tray guarantees a very quick and yet precise bonding using indirect bonding.

However, it is obvious that such an approach requires an extreme amount of time and labour and thus is too costly, and this is the reason why it is not practiced widely. The normal orthodontist does not fabricate set-ups; he places the brackets directly on the patient's teeth to the best of his knowledge, uses an off-the-shelf wire and hopes for the best. There is no way to confirm whether the brackets are placed correctly; and misplacement of the bracket will change the direction and/or magnitude of the forces imparted on the teeth. While at the beginning of treatment things generally run well as all teeth start to move at least into the right direction, at the end of treatment a lot of time is lost by adaptations and corrections required due to the fact that the end result has not been properly planned at any point of time. For the orthodontist this is still preferable over the lab process described above, as the efforts for the lab process would still exceed the efforts that he has to put in during treatment. And the patient has no choice and does not know that treatment time could be significantly reduced if proper planning was done.

U.S. Patent 5,431,562 to Andreiko et al. describes a computerized, appliance-driven approach to orthodontics. In this method, first certain shape information of teeth is acquired. A uniplanar target archform is calculated from the shape information. The shape of customized bracket slots, the bracket base, and the shape of an orthodontic archwire, are calculated in accordance with a mathematically-derived target archform. The goal of the Andreiko et al. method is to give more predictability, standardization, and certainty to orthodontics by replacing the human element in orthodontic appliance design with a deterministic, mathematical computation of a target archform and appliance design. Hence the '562 patent teaches away from an interactive, computer-based system in which the orthodontist remains fully involved in patient diagnosis, appliance design, and treatment planning and monitoring.

More recently, in the late 1990's Align Technologies began offering transparent, removable aligning devices as a new treatment modality in orthodontics. In this system, a plaster model of the dentition of the patent is obtained by the orthodontist and shipped to a remote appliance manufacturing centre, where it is scanned with a laser. A computer model of the dentition in a target situation is generated at the appliance manufacturing centre and made available for viewing to the orthodontist over the Internet. The orthodontist indicates changes they wish to make to individual tooth positions. Later, another virtual model is provided over the Internet and the orthodontist reviews the revised model, and indicates any further changes. After several such iterations, the target situation is agreed upon. A series of removable aligning devices or shells are manufactured and delivered to the orthodontist. The shells, in theory, will move the patient's teeth to the desired or target position.

The art has lacked an effective, computer-based interactive orthodontic treatment planning system that provides the necessary tools to allow the orthodontist to quickly and efficiently design a treatment plan for a patient. The art has also lacked a treatment planning system in which the orthodontist-derived parameters for the treatment can be translated into a design of an orthodontic appliance in real time, while the patient is in the chair. Real-time appliance design as described herein also allows for real-time communication of the treatment plan or appliance design to occur with the patient, or transmitted over a communications link and shared with a colleague or remote appliance manufacturing facility. Alternatively, the treatment planning can be performed remotely and a digital treatment plan sent to the orthodontist for review, interactive modification, or approval.

Scanners are devices for capturing and recording information from a surface of an object. Scanners for obtaining information from a two-dimensional surface, such as reading bar codes or characters printed on a piece of paper, are widely known. Several scanners have been proposed for recording three-dimensional information as well, including the field of dentistry.

U.S. Patent 4,837,732 and U.S. Patent 4,575,805 to Brandestini and Moermann propose a scanning system for *in vivo*, non-contact scanning of teeth. The patents describe a procedure for optically mapping a prepared tooth with a non-contact scan-head. The scan-head delivers the contour data, converted to electrical format, to be stored in a memory. A computer reads the memory following a line scan pattern. A milling device is slaved to follow this pattern by means of position control signals and mills an implant for the prepared tooth cavity.

The scan-head of the ’732 and ’805 patents includes a light emitting diode, with integral lens that radiates light onto the cavity. Before reaching the object, the rays of light are reflected by a mirror and pass through a ruling consisting of a plurality of parallel slits, or an alternating pattern of parallel opaque and transparent stripes. The reflected light is focused by a lens onto a charge-coupled device (CCD) sensor. Depth information is determined in accordance with a principle known as "active triangulation", using parameters shown in Figure 9 of this document and described subsequently. Basically, the object is viewed under an angle different from the incident rays due to a parallax effect. Each light stripe will have an apparent positional shift and the amount of the shift at each point along each light stripe is proportional to the vertical height of the corresponding portion of the surface on the object.

U.S. Patent No. 5,372,502 to Massen et al. describes an optical probe for measuring teeth that works on a similar principle. As noted in the Massen et al. patent, the Brandestini et al. technique is difficult to use when there are large variations in surface topography since such large jumps displace the pattern by an amount larger than the phase constant of the pattern, making it difficult to reconstruct the pattern of lines. Furthermore, precise knowledge of the angle of incidence and angle of reflection, and the separation distance between the light source and the detector, are needed to make accurate determinations of depth. Furthermore, the scanner has to be rather carefully positioned with respect to the tooth, and would be unable to make a complete model of the dentition.

U.S. Patent No. 5,027,281 to Rekow et al. describes a scanning method using a three axis positioning head with a laser source and detector, a rotational stage and a computer controller. The computer controller positions both the rotational stage and the positioning head. An object is placed on the rotational stage and the laser beam reflects from it. The reflected laser beam is used to measure the distance between the object and the laser source. X and Y coordinates are obtained by movement of the rotational stage or the positioning head. A three-dimensional virtual model of the object is created from the laser scanning. The ’281 patent describes using this scanning method for scanning a plaster model of teeth for purposes of acquiring shape of the teeth to form a dental prosthesis. The system of the ’281 patent is not particularly flexible, since it requires the object to be placed on the rotational stage and precise control of the relative position of the object and the positioning head is required at all times. It is unsuited for *in vivo* scanning of the teeth.

U.S. Patent 5,431,562 to Andreiko et al. describes a method of acquiring certain shape information of teeth from a plaster model of the teeth. The plaster model is placed on a table and a picture is taken of the teeth using a video camera positioned a known distance away from the model, looking directly down on the model. The image is displayed on an input computer and a positioning grid is placed over the image of the teeth. The operator manually inputs X and Y coordinate information of selected points on the teeth, such as the mesial and distal contact points of the teeth. An alternative embodiment is described in which a laser directs a laser beam onto a model of the teeth and the reflected beam is detected by a sensor. The patent asserts that three-dimensional information as to teeth can be acquired from this technique but does not explain how it would be done. Neither of the techniques of Andreiko have met with widespread commercial success or acceptance in orthodontics. Neither technique achieves *in vivo* scanning of teeth. Moreover, the video technique does not produce complete three-dimensional information as to the teeth, but rather a limited amount of two-dimensional information, requiring significant manual operator input. Even using this technique, additional equipment is required even to describe the labial surface of a tooth along a single plane.

Further, the prior art includes machines for bending wires to produce orthodontic wires. One such known machine, disclosed in US 4656860 (Orthuber et al), is a dental apparatus for bending and twisting wire pieces, for use as orthodontic wires. However, the apparatus disclosed in this document can only produce wires comprising straight segments separated by bends or twists; it cannot produce a wire where straight segments are separated by a curved segment comprising a combination of a bend and a twist.

## SUMMARY OF THE INVENTION

An interactive, orthodontic care system is disclosed based on scanning of teeth. The system includes a hand-held scanner and associated processing system for capturing images of the dentition of the patient and processing the images to generate a full, virtual, three-dimensional model of the dentition. A data conditioning system processes the virtual, three-dimensional model and responsively generates a set of individual, virtual three-dimensional tooth objects representing teeth in the dentition of the patient. A workstation is provided having a user interface for display of the set of individual, virtual three-dimensional tooth objects. Interactive treatment planning software is provided on the workstation permitting a user to manipulate the virtual three-dimensional tooth objects to thereby design a target situation for the patient in three dimensions and parameters for a customized orthodontic appliance for the teeth.

Specifically, the invention provides a manufactured orthodontic archwire, comprising: a wire, wherein the wire, in a relaxed state, has a generally arcuate configuration corresponding to an archform for a particular patient to receive the wire; wherein the wire further comprises a series of straight slot segments having precision lengths, each of the segments for placement into a slot of a bracket bonded to a tooth of the particular patient and, as needed, to allow for sliding of the bracket along such straight segments, the straight slot segments connected to each other by curved segments, comprising bends and/or twists, each forming a precision geometrical relationship between adjacent straight segments of the wire; and wherein at least one pair of adjacent straight slot segments are connected to each other by a curved segment comprising a combination of a bend and a twist forming a precision geometrical relationship between the adjacent straight segments in the wire.

The hand-held scanner and associated processing system, data conditioning system, and workstation may all be installed in an orthodontic clinic. Alternatively, the hand-held scanner and workstation are installed in an orthodontic clinic, and the data conditioning system is installed in a general purpose computer at a remote location from orthodontic clinic. Further, the treatment planning software can be either installed at the clinic, and/or at a remote location, and/or at a precision appliance manufacturing centre that manufactures a custom orthodontic appliance.

The treatment planning apparatus can be considered an interactive, computer-based computer aided design and computer aided manufacturing (CAD/CAM) system for orthodontics. The apparatus is highly interactive, in that it provides the orthodontist with the opportunity to both observe and analyze the current stage of the patient's condition and develop and specify a target or desired stage. A shortest direct path of tooth movement to the target stage can also be determined. Further, the apparatus provides for simulation of tooth movement between current and target stages.

In its broader aspects, the apparatus comprises a workstation having a processing unit and a display, and a memory storing a virtual, complete three-dimensional model representing the dentition of a patient. The virtual three-dimensional model can be obtained from one of several possible sources; in the preferred embodiment it is arrived at from a scanning of the dentition. The apparatus further includes software executable by the processing unit that accesses the model and displays the model on the display of the workstation. The software further includes navigation tools, e.g., typed commands, icons and/or graphical devices superimposed on the displayed model, that enables a user to manipulate the model on the display and simulate the movement of at least one tooth in the model relative to other teeth in the model in three-dimensional space, and quantify the amount of movement precisely. This simulation can be used, for example, to design a particular target situation for the patient.

The development of a unique target situation for the patient has utility in a variety of different orthodontic appliances, including an approach based on off-the-shelf or generic brackets and a custom orthodontic archwire. The scope of the invention is sufficient to encompass other types of appliances, such as an approach based on customized brackets, retainers, or removable aligning devices. In a bracket embodiment, the memory contains a library of virtual, three-dimensional orthodontic brackets. The software permits a user to access the virtual brackets through a suitable screen display, and place the virtual brackets on the virtual model of the dentition of the patient. This bracket bonding position can be customized on a tooth by tooth basis to suit individual patient anatomy. Because the tooth models, brackets and archwire are individual objects, and stored as such in memory, the treatment planning apparatus can simultaneously display the virtual brackets, the archwire and the virtual model of the dentition, or some lesser combination, such as just the brackets, just the dentition, or the brackets and the archwire but not the teeth. The same holds true with other appliance systems.

In a preferred embodiment, the virtual model of teeth comprises a set of virtual, individual three-dimensional tooth objects. A method of obtaining the tooth objects from a scan of teeth, and obtaining other virtual objects of associated anatomical structures, e.g., gums, roots and bone is described. When the teeth are separated from each other and from the gums, they can be individually manipulated. Thus, the individual tooth objects can be individually selected and moved relative to other teeth in the set of virtual tooth objects. This feature permits individual, customized tooth positioning on a tooth by tooth basis. These positioning can be in terms or angular rotation about three axis, or translation in transverse, sagittal or coronal planes. Additionally, various measurement features are provided for quantifying the amount of movement.

One of the primary tools in the treatment planning apparatus is the selection and customization or a desired or target archform. Again, because the teeth are individual tooth objects, they can be moved independently of each other to define an ideal arch. This development of the target archform could be calculated using interpolation or cubic spline algorithms. Alternatively, it can be customized by the user specifying a type of archform (e.g., Roth), and the tooth are moved onto that archform or some modification of that archform. The archform can be shaped to meet the anatomical constraints of the patient. After the initial archform is designed, the user can again position the teeth on the archform as they deem appropriate on a tooth by tooth basis. The treatment planning software thus enables the movement of the virtual tooth objects onto an archform which may represent, at least in part, a proposed treatment objective for the patient.

Numerous other features are possible with the treatment planning software, including movement of the teeth with respect to the other teeth in the archform, changing the position of the virtual brackets and the teeth with respect to each other, or opposing teeth with respect to the selected archform. Custom archwire bends can be simulated to provide additional corrections. Bonding corrections at the bracket-tooth interface are also possible.

In another aspect, a method for digital treatment planning for an orthodontic patient on a workstation having a processing unit, a user interface including a display and software executable by the processing unit comprises the steps of obtaining and storing a three-dimensional virtual model of teeth representing the dentition of the patient in a current or observed situation. The virtual model is displayed on the display. The method further includes the step of moving the position of teeth in the virtual model relative to each other so as to place the teeth of the virtual model into a target situation and displaying the virtual model with the teeth moved to the target situation to the user. Parameters for an orthodontic appliance to move the patient's teeth from the current situation to the target situation can be derived from the virtual model and the target situation. For example, if virtual brackets are placed on the teeth, their location in the target situation can dictate the design of an archwire to move the teeth to the target situation.

The scanner system is provided for capturing three-dimensional information of an object. The object can be virtually any object under scrutiny, however the present document will describe an application in which the object is the dentition of a patient suffering from a malocclusion.

The scanning system enables three-dimensional surface information to be obtained with a very high decree of precision. Moreover, the scanning system can be used without requiring precise movement of the scanner, or requiring the object under scrutiny to be fixed in space. Surprisingly, the scanner is able to generate precise three dimensional surface information by simply moving the scanner over the surface of the object, such as by hand, in any manner that is convenient for the user, even if the object moves in any random direction during the scanning within reasonable limits. Thus, the scanner can be used to capture the surface of a patient's dentition in a minute or two, even if the patient moves their head or jaw while the scanning is occurring. Precise knowledge of the spatial relationship between the scanner and the object is not required.

The scanner obtains a set of images, which are processed in a computer to calculate the surface configuration of the object in three dimensions of space automatically, quickly, with high precision, and with essentially no human involvement other than the act of scanning. The precision or accuracy will be dictated largely by the extent to which the object under scrutiny tends to have undercut or shadowed features which are difficult to detect, necessitating a narrow angle between the projection and imaging axes. For teeth, an accuracy of under 20 or 30 microns is possible. This accuracy can be further improved depending on the nature of the surface, such as if the surface does not have a lot of undercut or shadowed features, by increasing the angular separation of the projection axis and the imaging axis.

Each image captured by the scanner is converted to a virtual, three-dimensional point cloud or "frame". The illustrated embodiment has a relatively coarse resolution for any single frame, due to a coarse projection pattern, but a fine resolution is obtained by obtaining multiple images and performing a registration procedure on the frames, as described below. Since each point on the surface of the object is captured in a plurality of images (such as five or six in a typical example of scanning), the registration of frames results in a fine resolution. An even finer resolution can be obtained by scanning slower and capturing more images of the surface of the object from different perspectives and registering the resulting frames to each other.

This surface configuration of the object in three dimensions of space can be represented as a mathematical model, i.e., a virtual model of the object, which can be displayed on any workstation or computer using available software tools. The mathematical model can be viewed in any orientation in space, permitting detailed analysis of the surface. The model can be compared to template objects stored in a computer. Deviations in the object from the template can be quantified and analyzed. Further, the virtual model can be transported from one computer and another computer anywhere in the world essentially instantaneously over communications links such as the Internet. The model can be replicated in a computer and thus shared and used by multiple users simultaneously.

The scanner system is useful for a wide variety of industrial, medical, archaeological, forensic, archival, or other purposes. Furthermore, the scanner can be scaled down in size such that it can be hand-held and used to scan small objects, e.g., teeth or small machined parts, or scaled up in size so that it can be used to make mathematical models of larger scale objects such as works of art, sculptures, archaeological sites (e.g., the caves at Lascaux, France or the dwellings or kivas in Mesa Verde National Park), rooms or building facades.

In accordance with a preferred embodiment, the scanner system includes a projection system that projects a pattern onto the object along a first optical axis. The pattern may consist of parallel lines, parallel lines separated by shapes or colours, such as coloured dots, or other suitable pattern. The projected pattern is used to gather information as to the surface characteristics of the object in accordance with the methods and procedures described in more detail below.

The scanner further includes an electronic imaging device, preferably in the form of a charge-coupled device comprising a two-dimensional array of photo-sensitive pixels. The electronic imaging device is oriented along a second optical axis different from the first optical axis. The electronic imaging device forms an image of the pattern after reflection of the pattern off of the object under scrutiny. The surface configuration of the object will cause the projection pattern to become distorted and changed, and thereby provide information as to the surface configuration. This information as to the surface is captured by the imaging device as two-dimensional images of the reflection pattern. These images are processed in accordance with procedures described herein to derive three-dimensional information as to the surface of the object.

The scanning system, in a preferred embodiment, further includes a memory that stores data representing a three axis (X, Y, Z) calibration relationship for the scanner. The calibration relationship can be in the form of a table or in the form of a mathematical function (e. g., polynomial, spline, or other function). The calibration relationship identifies two properties of the scanner: (1) pixel coordinates for the electronic imaging device for numerous portions of the pattern, said pixel coordinates associated with distance information from the projection system in a Z direction at at least two different Z distances, and (2) distance information in X and Y directions, for the numerous portions of said pattern, at the at least two different Z distances. A method of obtaining the calibration relationship is described in detail below. While the simplest form of the relationship is a table, as described in detail below, these calibration relationships could be equivalently represented by one or more mathematical functions as will be apparent to those skilled in art.

The calibration relationship is used to derive three-dimensional coordinates for points on the surface of an object imaged by the imaging device. The generation and use of the preferred calibration table is also explained in further detail below. Other calibration tables or procedures are also possible. The use of the calibration relationship allows the scanner to operate without precise knowledge of the optical and mechanical properties of the scanner, as is required in prior art systems.

The scanning system further includes at least one data processing unit, e.g., the central processing unit of a computer or a digital signal processor, which processes the images of the projection pattern after reflection from the surface of the object. The processing unit compares data from the image (e.g., pixel locations where certain points in the projection pattern are imaged) to the calibration relationship to thereby derive spatial information, in three dimensions, of points on the object reflecting the projected pattern onto the electronic imaging device. Multiple processing units can be used to reduce the amount of time it takes to process the two-dimensional images, calculate three-dimensional coordinates for points in each image, and register frames of three-dimensional coordinates relative to each other to generate a complete virtual model of the object.

The scanning system thus derives three-dimensional information of the object from the images generated by the imaging device and from the calibration relationship stored in memory. Precise knowledge of the optical characteristics of the scanner, the angles between the first and second optical axes, the angle between the optical axis and the point in the imaging device, or the separation distance of the projection device from the imaging device are not necessary. The projection system and the imaging device can be even uncoupled relative to each other. The calibration relationship automatically and completely compensates for these issues, as well as manufacturing variations and tolerances in the scanner optics, in a highly reliable manner. Moreover, knowledge of the distance from the scanner to the object is not required. Additionally, control over the distance between the scanner and the object is not required, within reasonable limits dictated by the depth of focus of the imaging optics. Ideally, during scanning the distance from the scanner to the object is maintained within the limits of the Z distances used during calibration, and that distances is within the combined depth of focus of the projection optics and the imaging optics.

The scanning system can be constructed such that the memory, processing unit, and optical elements are in a single unit. Alternatively, the processing unit and memory can be located at a separate location, such as a scanning workstation or "scanning node", in order to reduce the size of the scanner per se. In such an embodiment, the projection system and the image-recording device can be miniaturized into a hand-held scanner device. A suitable cable connects the scanner device to the workstation to thereby supply the processing unit with scan data, and to receive commands (illumination commands, start/stop commands, etc.) from the workstation.

Preferred appliance manufacturing systems are also described, including bending apparatus that bends medical devices, such as orthodontic appliances like archwires, fixation plates and other devices. Bracket placement trays are also described. As one embodiment, an orthodontic appliance manufacturing system is provided comprising a machine readable memory storing digital data representing a three-dimensional virtual model of a malocclusion of a patient and digital information representing the location of orthodontic brackets to, be placed on the malocclusion, said memory further storing digital data representing a three-dimensional virtual model of the patient's teeth and the dentition and location of orthodontic brackets at a target situation in three dimensions. The apparatus further includes a wire bending robot. A rapid prototyping instrument such as SLA (stereolithography) is provided for generating a three-dimensional physical model of the malocclusion with the brackets placed on the malocclusion. A forming device forms a bracket placement tray matching the geometry of the physical model of the malocclusion with the brackets. The bracket placement tray may be removed from the physical model, said tray formed with spaces for placement of brackets and enabling bonding of the brackets to the teeth at a desired location based on the virtual model of the dentition and the placement of orthodontic brackets on the malocclusion.

Numerous other features of the appliance manufacturing apparatus, the treatment planning software, the scanning system and related features will be more apparent from the following detailed description.

BRIEF DESCRIPTION OF THE DRAWINGS

Figure 1 is an illustration of an orthodontic care system incorporating a hand-held scanner system. The hand-held scanner is used by the orthodontist or the assistant to acquire three-dimensional information of the dentition and associated anatomical structures of a patient and provide a base of information to diagnose and plan treatment for the patient.

Figure 2 is a block-diagram of a scanning system, suitable for use in the orthodontic care system of Figure 1.

Figure 3 is a perspective view of a hand-held scanner used to acquire information of an object under scrutiny, suitable for use in the orthodontic care system of Figure 1.

Figure 4 is an illustration of a patient being scanned with the hand-held scanner of Figure 3.

Figure 5 is a block diagram of the back office server of Figure 1 showing the elements used to calculate the digital model of the patient's dentition and display the digital model on a screen display of the server.

Figure 60 is a screen shot from an orthodontic workstation showing the computer model of the patient's teeth positioned in a target or desired condition, as a result of the user selecting an archform for the patient and the computer placing the teeth along the arch selected by the user. Figure 60 also shows the various parameters by which the orthodontist can adjust the shape of the arch, the distance between the teeth, the distance between the molars, and other parameters, so as to provide a unique and customized target situation for the patient.

Figure 61 is another screen shot showing the computer model of the patient's teeth in a target situation, also showing the numerous parameters available to the orthodontist to customize the tooth position, orientation, angulation, torque, and other parameters on a tooth by tooth basis for the target archform.

Figure 62 is another screen shot showing a front view of the target situation and additional parameters available to the orthodontist for moving teeth relative to each other in planning treatment for the patient.

Figure 63 is a screen shot of a target situation for the patient showing the virtual tooth in a target position, a set of virtual brackets placed on the teeth, and a virtual archwire.

Figure 66 is a screen shot of a workstation user interface display showing a patient records screen which the user access various information regarding the patient, including dental history, X-ray photographs, medical history, photographs of the face, teeth and head and the three-dimensional model of the malocclusion.

Figure 67 is an illustration of a series of icons that appear on a screen display that provide some tools for viewing the three-dimensional model of the patient's dentition.

Figure 68 is an illustration of a set of icons which are part of the screen displays which act as a navigational tool and allow the user to manipulate the three-dimensional models of teeth and brackets on the display.

Figure 69 is a screen shot from the treatment planning software showing a set of individual tooth objects representing the observed stage of a patient suffering from a malocclusion.

Figure 70 is another screen shot from the treatment planning software, showing the observed stage and the placement of virtual three-dimensional brackets on the surfaces of the teeth.

Figure 71 is another screen shot from the treatment planning software, showing several views of the observed stage and the fields by which an orthodontist can enter values to alter the relation of the upper jaw to the lower jaw as an initial step of planning treatment.

Figure 72 is another screen shot showing several views of the malocclusion displayed simultaneously, similar to Figure 71.

Figure 73 is a screen show showing a cross-section or "clipping plane" view through the upper arch in a target situation.

Figure 74 is a screen shot illustrating of a portion of a target arch, showing a vertical cross-section or clipping plane taken through the teeth. This view is helpful in adjusting the relation between the upper and lower jaw.

Figure 75 is a screen shot showing the placement of the virtual brackets on the teeth at the malocclusion, showing the user clicking on an icon to establish an initial archform for the upper arch.

Figure 76 is a screen shot showing the computer model of the patient's teeth positioned in a target or desired stage, as a result of the user selecting an archform for the patient and the computer placing the teeth along the arch selected by the user. Figure 76 also shows the various parameters by which the orthodontist can adjust the shape of the arch, the distance between the teeth, the distance between the molars, and other parameters, so as to provide a unique and customized archform for the patient.

Figure 77 is another screen shot showing the computer model of the patient's teeth in a target stage, also the brackets and the orthodontic archwire, and showing the numerous parameters available to the orthodontist to customize the tooth position, orientation, angulation, torque, and other parameters on a tooth by tooth basis for the target archform.

Figure 78 is another screen shot showing a view of the target situation, with brackets and archwire, showing fields allowing the orthodontist to moving the teeth objects relative to each other in planning treatment for the patient.

Figure 79 is a screen show showing a bracket offset correction being entered to move tooth number 16 into an improved occlusal relationship with the opposing jaw.

Figure 80 is a screen shot showing the tooth movement that occurs with tooth number 16 when the bracket offset correction is made.

Figure 81 is another screen show showing the target stage, with the brackets and archwire, showing a tooth moved in the buccal and coronal directions by an amount indicated by the orthodontist, and the correction incorporated into the archwire.

Figure 82 is another screen shot showing a space management feature by which the target situation can be adjusted by specifying spaces between teeth or by extraction of teeth.

Figure 83 is a screen shot illustrating the simulation of an extraction of a tooth number 41.

Figure 84 is a screen shot showing the user modifying the mesial gap between the teeth and showing how the user-specified mesial gap is instantaneously represented on the screen.

Figure 85 is a screen shot showing the user modifying tooth position in a target stage on a tooth by tooth basis using a bonding correction feature.

Figure 86 is a screen shot showing a wire tab, which allows the user to make changes in the shape of an archwire without changing bracket position.

Figure 87 is a screen shot showing a wire offset tab, which allows the user to change the bend size, location, etc., in the wire.

Figure 88 is a schematic representation of an archwire manufacturing system shown in Figure 1.

Figure 88A is a block diagram of the system of Figure 88.

Figure 89 is a perspective view of a moveable robot arm used in the manufacturing system in Figure 88; in Figure 89 the gripping tool at the distal end of the arm is omitted for sake of clarity to show the various other aspects of the arm.

Figure 90 is perspective view of the robot arm of Figure 89, showing the movement of one of the arm joints and the corresponding motion of the arm.

Figure 91 is a detailed perspective view of the gripping tool that is mounted to the distal end of the moveable robot arm of Figure 89 and Figure 90.

Figure 92 is a perspective view of the fixed gripping tool of Figure 88 and the gripping tool of Figure 91, in which an orthodontic archwire is gripped by the tools.

Figure 93 is a perspective view of a magazine of Figure 88 that holds a plurality of straight archwires.

Figure 94 is a detailed perspective view of the moveable gripping tool grasping one of the archwires from the magazine of Figure 93.

Figure 95 is a perspective view of an alternative arrangement of a moveable robot arm.

Figures 96A and 96B are perspective views of alternative magazine constructions to the magazine of Figure 93.

Figure 97 is a schematic illustration of a conveyor system that carries archwires to the robot arm of Figure 88.

Figure 98 is a diagram illustrating the robot software as it relates to the production flow in producing orthodontic archwires.

Figure 99 is a simplified illustration of a set of teeth showing the origin of a coordinate system that is used to calculate bracket location for a set of brackets, in three dimensions, for a patient. The bracket location for the teeth in a target situation determines the shape of an orthodontic archwire.

Figure 100 is an illustration showing the vectors drawn from the origin of the coordinate system to the centre of the brackets.

Figure 101 is a perspective view of an orthodontic bracket.

Figure 102 is an illustration of a vector drawn from the origin of the coordinate system to the bracket, a normal vector N perpendicular to the slot surface of the bracket, and a tangential vector T extending in the direction of the slot of the bracket.

Figure 103 shows the normal vector Y for a particular bracket, the tangential vector X, the tangential distance $T_d$ and antitangential distance $AT_d$.

Figure 104 shows in matrix form the values for an individual bracket which describe the location of the bracket and its orientation, which are used to generate the commands for the robot to form the orthodontic archwire.

Figure 105 is an illustration of a set of points P1, P2, P3,... PN which represent a set of bending points associated with individual brackets for a patient in a target situation. The location of the points in the three-dimensional coordinate system is known.

Figure 106 is an illustration of a section of wire between points P1 and P4 in which a bend is placed between points P2 and P3.

Figure 106A is an illustration of four points and a curve defined by a Bezier spline, a technique used to calculate the shape of the bend in the wire between points P2 and P3 in Figure 106.

Figure 106B is a flow chart of an algorithm to calculate the Bezier spline of Figure 106A and the length of the curve.

Figure 107 is a graph of force as a function of deflection for a workpiece such as a wire. The graph illustrates that that when a certain amount of force, F1, is applied to the workpiece and then released, a deflection D2 results. When the force is released, the amount of remaining deflection, D1, is less than the deflection observed when the force is applied to the wire, D2, since the wire has elastic properties.

Figure 108 is an illustration of the stresses found in a wire when it is bent.

Figure 109 is an elevational view of the gripping fingers of the fixed gripping tool of Figure 92, showing the origin of a coordinate system used by the robot in bending wire.

Figure 110 is a top view of the gripping fingers of Figure 109.

Figure 111 is flowchart illustrating a deformation-controlled overbending procedure, which may be used to compensate for the elastic properties of the wire demonstrated by Figure 107.

Figure 112 is an illustration showing the overbending method set forth in Figure 111.

Figures 113A-113E are a series of schematic drawings of the fixed and moveable gripping tools of Figure 92, showing how they moved relative to each other and grip and release the archwire to place the archwire in position to form a bend between points P2 and P3 of Figure 105.

Figure 114 is a schematic illustration showing how the movable gripping tool bends an archwire while maintaining a constant distance from the fixed gripping tool.

Figures 115A-115C illustrate how a bend may be formed in a series of steps.

Figures 116A-116D are a series of schematic drawings of the fixed and moveable gripping tools of Figure 92, showing how they move relative to each other to place the archwire in position to form a bend between points P4 and P5.

Figure 117 is an illustration of the points defining a portion of an archwire, and illustrating a technique for placing bends in wires where a substantial distance exists between the straight wire segments.

Figure 118 is an illustration of a portion of an archwire showing a bend formed therein to increase the forces applied by the wire when the wire has nearly straightened out, e.g., near the end of treatment.

Figure 119 shows the wire segment of Figure 118 installed between two teeth.

Figures 120 shows a wire with a loop in the wire.

Figures 121A-121B illustrate one possible method of forming the loop in the wire of Figure 120.

## DETAILED DESCRIPTION OF PREFERRED EMBODIMENT

### Part 1. Overview

Figure 1 is an illustration of an orthodontic care system 10 incorporating a scanner system 12. The scanner system 12 includes a hand-held scanner 14 that is used by the orthodontist or his assistant to acquire three-dimensional information of the dentition and associated anatomical structures of a patient. The images are processed in a scanning node or workstation 16 having a central processing unit, such as a general-purpose computer. The scanning node 16, either alone or in combination with a back-office server 28, generates a three-dimensional computer model 18 of the dentition and provides the orthodontist with a base of information for diagnosis, planning treatment, and monitoring care for the patient. The model 18 is displayed to the user on a monitor 20 connected to the scanning node 16.

As noted above, the scanner system 12 is optimized for *in vivo* scanning of teeth, or alternatively, scanning a plaster model of the teeth and/or an impression of the teeth. However, it will be apparent to persons skilled in the art that the scanning system 12 can by readily optimized for a variety of other diagnostic and/or treatment planning and/or monitoring uses in the medical arena. An example is scanning the face or head and planning plastic or orthopaedic surgery. It can be readily adapted to virtually limitless number of applications in industrial, manufacturing, forensic, archaeological, scientific, archival or other applications.

The orthodontic care system consists of a plurality of orthodontic clinics 22 which are linked via the Internet or other suitable communications medium 24 (such as the public switched telephone network, cable network, etc.) to a precision appliance service centre 26.

Each clinic 22 has a back office server work station 28 having its own user interface, including a monitor 30. The back office server 28 executes an orthodontic treatment planning software program. The software obtains the three-dimensional digital data of the patient's teeth from the scanning node 16 and displays the model 18 for the orthodontist. The treatment planning software includes features to enable the orthodontist to manipulate the model 18 to plan treatment for the patient. For example, the orthodontist can select an archform for the teeth and manipulate individual tooth positions relative to the archform to arrive at a desired or target situation for the patient. The software moves the virtual teeth in accordance with the selections of the orthodontist. The software also allows the orthodontist to selectively place virtual brackets on the tooth models and design a customized archwire for the patient given the selected bracket positions. When the orthodontist has finished designing the orthodontic appliance for the patient, digital information regarding the patient, the malocclusion, and a desired treatment plan for the patient are sent over the communications medium to the appliance service centre 26. A customized orthodontic archwire and a device for placement of the brackets on the teeth at the selected location is manufactured at the service centre and shipped to the clinic 22. The system is also applicable to other types of appliance systems ; brackets and archwires are shown in the illustrated embodiment but other types of appliance systems can benefit from the scanning system described herein, such as removable aligning devices, retainers, Herbst appliances, etc.

As shown in Figure 1, the precision appliance service centre 26 includes a central server 32, an archwire manufacturing system 34 and a bracket placement manufacturing system 36. These details are described later.

Figure 2 is a more detailed block-diagram of the scanning system 12, suitable for use in the orthodontic care system of Figure 1. The scanning system 12 is a mechanism for capturing three-dimensional information of an object 40, which in the present example is the dentition and surrounding anatomical structures of a human patient, e.g., gums, bone and/or soft tissue. The scanning system 12 includes a scanner 14 which is used for image capture, and a processing system, which in the illustrated example consists of the main memory 42 and central processing unit 44 of the scanning node or workstation 16.

The scanner 14 includes a projection system 46 that projects a pattern onto the object 40 along a first projection axis 48. The projected pattern is formed on a slide 50 which is placed in front of a light source 53. In the illustrated embodiment, the light source 53 comprises the terminus of a fibre-optic cable 51. The cable 51 carries a high intensity flash generated by a flash lamp 52 located in a base unit 54 for the scanner. A suitable flash lamp is the model FX-1160 flash unit available from Perkin Elmer. The illuminations of the flash lamp 52 cause the pattern contained in the slide 50 to be projected onto the three-dimensional surface of the object. Further details on the types of patterns suitable for the pattern are set forth in the following co-pending patent applications of Rudger Rubbert et al: serial no. 09/254,755 filed March 9, 1999; serial no. 09/1560,131 filed April 28, 2000, and serial no. 09/673,863 filed November 30, 2000 assigned to the assignee of the present invention.

The scanner 14 further includes an electronic imaging device 56 comprising an array of photo-sensitive pixels. A preferred embodiment is an off-the-shelf, colour-sensitive, charged-coupled device (CCD) of a size of 1,028 x 1,028 pixels arranged in an array of rows and columns. The Sony ICX205AK CCD chip is a suitable electronic imaging device.

The electronic imaging device 56 is oriented perpendicular to a second imaging axis 58, which is off-set from the projection axis 48. The angle $\Psi$ between the projection and imaging axes need not be known. Angle $\Psi$ is shown somewhat exaggerated in Figure 2, and will generally range between 10 and 30 degrees for most applications.

The electronic imaging device 56 forms an image of the projection pattern after reflection of the pattern off of the surface of the object 40. The reflected patterns imaged by the imaging device contain three-dimensional information as to the surface of the object, and this information needs to be extracted from the images. The scanning system therefore includes a processing subsystem which is used to extract this information and construct a three-dimensional virtual model of the object 40. In the preferred embodiment, this processing subsystem consists of a memory 42 storing calibration information for the scanner, and at least one processing unit, such as the central processing unit 44 of the scanning workstation 16. The location of the memory and the processing unit is not important. They can be incorporated into the scanner 14 per se. Alternatively, all processing of the images can take place in the back office server 28 or in another computer. Alternatively, two or more processing units could share the processing in order to reduce the amount of time required to generate the three-dimensional information.

The memory 42 stores a calibration relationship for the scanner 14. The calibration relationship, which can be in the form of a table or one more mathematical functions, comprises information used to compute three-dimensional coordinates of points on the object that reflected the projection pattern onto the imaging device. The information for the table is obtained during a calibration step, performed at the time of manufacture of the scanner 14. The calibration table includes an array of data storage locations that contain two pieces of information. Firstly, the calibration table stores pixel coordinates in X and Y directions for numerous portions of the projection pattern that are imaged by the electronic imaging device 56, when the pattern is projected onto a calibration surface at two different distances during a calibration procedure. Secondly, the table stores distance information, (e.g., in units of tenths of millimetres), in X and Y directions, for the portions of the projection pattern imaged at the two different distances. The scanning system requires at least one processing unit to perform image processing, three-dimensional calculations for each image, and registration of frames to each other. The processing unit 44 in the illustrated embodiment is the central processing unit (CPU) of the scanning work station 16. The CPU 44 processes the image of the pattern after reflection of the pattern off the surface of the object 40 and compares data from the image to the entries in the calibration table. From that comparison (or, more precisely, interpolation relative to the entries in the table), the processing unit 44 derives spatial information, in three dimensions, of points on the object that reflect the projected pattern onto the electronic imaging device.

Basically, during operation of the scanner to scan an object of unknown surface configuration, hundreds or thousands of images are generated of the projection pattern as reflected off of the object in rapid succession as the scanner and object are moved relative to each other. For each image, pixel locations for specific portions, i.e., points, of the reflected pattern are compared to entries in the calibration table. X, Y and Z coordinates (i. e., three dimensional coordinates) are obtained for each of these specific portions of the reflected pattern. For each picture, the sum total of all of these X, Y and Z coordinates for specific points in the reflected pattern comprise a three-dimensional "frame" or virtual model of the object. When hundreds or thousands of images of the object are obtained from different perspectives, as the scanner is moved relative to the object, the system generates hundreds or thousands of these frames. These frames are then registered to each other to thereby generate a complete and highly accurate three-dimensional model of the object 40.

Stray data points are preferably cancelled out in generating the calibration table or using the calibration table to calculate three-dimensional coordinates. For example, a smoothing function such as a spline can be calculated when generating the entries for the calibration table, and the spline used to cancel or ignore data points that deviate significantly from the spline.

Figure 2 also shows a few other features of the presently preferred scanning system 12. After the CCD imaging device 56 captures a single image, the analogue voltage signals from the device 56 are amplified in an amplifier 57 and fed along a conductor 59 to an analogue to digital converter 60. The digital signal is converted into a bitmap stream of digital image data. The data is formatted by a module 61 into an IEEE 1394 "firewire" format for transmission over a second conductor 62 to the main memory 42 of the scanner work station 16. The scanning system includes an optical scanner holder 64 for the user to place the scanner after the scanning of the dentition is complete. These details are not particularly important and can vary considerably from the illustrated embodiment. As noted earlier, preferably the scanning system is constructed to provide a minimum of equipment and clutter at the chair side. Hence, the scanning work station 16 is preferably located some distance away from the chair where the patient sits. The cable leading from the scanner 14 to the base station and/or workstation 16 could be suspended from the ceiling to further eliminate chairside clutter.

The scanning work station 16 also includes the monitor 20 for displaying the scanning results as a three-dimensional model 18 of the dentition in real time as the scanning is occurring. The user interface also includes a keyboard and mouse for manipulating the virtual model of the object, and for entering or changing parameters for the scanning, identifying sections or segments of scans that have been obtained, and other features. The scanning station may also include a foot switch, not shown, for sending a signal to the CPU 44 indicating that scanning is commencing and scanning has been completed. The base station may alternatively include a voice recognition module that is trained to recognize a small set of voice commands such as START, STOP, AGAIN, REPEAT, SEGMENT, ONE, TWO, THREE, FOUR, etc., thereby eliminating the need for the foot switch. Scanner start and stop commands from the CPU 44, in the form of control signals, are sent to the light source 52, thereby controlling the illumination of the lamp 52 during scanning.

The light source 52 operates at a suitable frequency, preferably at least greater than one flash per second, such as six flashes per second, and the frame rate of the CCD imaging device 56 is synchronized with the flash rate. With a frame rate of 6 frames per second, and a scanning motion of say 1-2 centimetres per second, a large of overlap between images is obtained. The size of the mirror at the tip 68 of the scanner influences the speed at which scanning is possible. The illustrated embodiment of the mirror at the tip 68 is 18 mm square. A larger mirror reflects more surface of the object and enables faster scanning. A smaller mirror requires slower scanning. The larger the mirror, the more difficult *in vivo* scanning becomes, so some trade-off between size and utility for *in vivo* scanning exists.

This overlap between images generated by the scanner 14, and resulting three dimensional frames, allows a smooth and accurate registration of frames relative to each other. The frame rate and permissible rate of scanner motion will depend on many factors and can of course vary. Flashing a high intensity flash lamp for a brief period of time is a preferred embodiment since it is desirable to reduce the exposure time of the CCD imaging device 56 to reduce blurring since relative motion exists between the scanner and the object. A high intensity lamp is desirable to achieve sufficient signal strength from the imaging device. A preferred embodiment uses 5 psec flash times with similar exposure periods. An alternative embodiment would use a constant illumination source of high intensity, and control exposure of the imaging device using a shutter, either a physical shutter or using electronic shutter techniques, such as draining charge accumulating in the pixels prior to generating an image. Scanning using longer exposures would be possible without image blur, using electronic image motion compensation techniques described in Lareau, et al., U.S. Patent 5,155,597.

Figure 3 is a perspective view of a hand-held scanner 14 used to acquire information of an object under scrutiny, suitable for use in the orthodontic care system of Figure 1. The projection system 46 and the electronic imaging device 56 of Figure 2 are contained in the housing 65 for the scanner. The housing 65 is sized and shaped to be held in a human hand. The scanner 14 includes an elongate distal portion 66 having a tip 68. The tip 68 is sized and shaped such that it can be inserted into and moved within an oral cavity of a human so as to enable scanning of anatomical structures inside the oral cavity. A heating mirror (not shown) is placed on the underside of the tip 68 to direct the projection pattern from the optics of the scanner onto the object and to direct the reflected pattern from the object towards the imaging optics 108 of Figure 7 associated with the electronic imaging device 56. The mirror housing has A/C conductive heating coil that heats the mirror. The mirror is heated to approximately 40 degrees to prevent fogging of the mirror while the scanner is used *in vivo.*

Figure 4 is an illustration of a patient 70 being scanned with the hand-held scanner 14 of Figure 3. The cheeks and lips are retracted from the teeth and the tip 68 of the scanner is moved over all the surfaces of the teeth in a sweeping motion at a velocity of perhaps 1-2 centimetres per second. The entire upper or lower jaw may need to be scanned in a series of scans, one for the left side, one for the right side, and one for the front. These individual scans are registered to each other as described below. Voice commands or activation of the foot switch (not shown) indicates when each scanning segment is initiated and terminated. The entire process takes just a few minutes. Depending on the colour and translucency of the object and the illumination intensity and frequency of the light source in the scanner, it may be necessary to apply a very thin coating of a bright reflective substance such as Titanium Dioxide to the object.

While Figure 4 illustrates *in vivo* scanning of a human patient, the scanner can of course be used to scan a plaster model of the dentition if that is preferred, or an impression taken from the patient. When scanning an impression or a plaster model, the scanning may be formed in a single pass, without the need for registering scan segments to each other. It is also possible that a scan of a patient may be partially taken *in vivo* and the remainder from a model or an impression.

Figure 5 is a block diagram of the back office server of Figure 1 showing the elements used to calculate the digital model of the patient's dentition. After the scanning workstation has processed all the images captured by the scanner and generated a set of three dimensional frames, the frame data is transmitted to the back office server 28. The back office server 28 performs a cumulative registration process for the frames and ultimately generates and displays the digital model on a screen display 30. The raw scanner data in the form of three-dimensional frames is stored in the main computer memory 72. The frame data for N captured images, i = 1... N from the scanner is stored in the hard disk 74. The hard disk also stores a set of (N-1) transformation matrices $[T]_i$, for i = 2 - N. The transformation matrices basically contain information as to how each frame of three-dimensional points needs to be translated and rotated in a three-axis Cartesian coordinate system in order to be registered with the other frames in a best-fit manner. One of the frames, such as the first frame in the series, is a starting point for registration and no transformation matrix is obtained for that frame.

As mentioned above, the invention is concerned with manufacture of a custom orthodontic archwire, which is produced to fit a specific treatment plan which is based on a virtual three-dimensional model representing the dentition of a patient. Thus, the details of the scanner and the scanning process are not relevant to the invention as now claimed, and so these aspects of the system will not be described in detail. Further, the exact details of how the virtual three-dimensional model representing the dentition of a patient is derived from the data obtained by the scanner are also not of relevance to the invention as now claimed, and so these will also not be described in detail.

## Part 2. Treatment Planning

### Introduction

Treatment planning software is provided on the workstation of the orthodontic clinic, and possibly at other remote locations such as the precision appliance centre of Figure 1. The treatment planning software can be considered an interactive, computer-based computer aided design and computer aided manufacturing (CAD/CAM) system for orthodontics. The apparatus is highly interactive, in that it provides the orthodontist with the opportunity to both observe and analyze the current stage of the patient's condition and develop and specify a target or desired stage. A shortest direct path of tooth movement to the target stage can also be determined. Further, the apparatus provides for simulation of tooth movement between current and target stages.

In its broader aspects, the apparatus comprises a workstation having a processing unit and a display, and a memory storing a virtual, complete three-dimensional model representing the dentition of a patient. The virtual three-dimensional model can be obtained from one of several possible sources; in the preferred embodiment it is arrived at from a scanning of the dentition. The apparatus further includes software executable by the processing unit that accesses the model and displays the model on the display of the workstation. The software further includes navigation tools, e.g., typed commands, icons and/or graphical devices superimposed on the displayed model, that enables a user to manipulate the model on the display and simulate the movement of at least one tooth in the model relative to other teeth in the model in three-dimensional space, and quantify the amount of movement precisely. This simulation can be used, for example, to design a particular target situation for the patient.

The development of a unique target situation for the patient has utility in a variety of different orthodontic appliances, including an approach based on off-the-shelf or generic brackets and a custom orthodontic archwire. The scope of the invention is sufficient to encompass other types of appliances, such as an approach based on customized brackets, retainers, or the removable aligning devices mentioned earlier. In a bracket embodiment, the memory contains a library of virtual, three-dimensional orthodontic brackets. The software permits a user to access the virtual brackets through a suitable screen display, and place the virtual brackets on the virtual model of the dentition of the patient. This bracket bonding position can be customized on a tooth by tooth basis to suit individual patient anatomy. Because the tooth models, brackets and archwire are individual objects, and stored as such in memory, the treatment planning apparatus can simultaneously display the virtual brackets, the archwire and the virtual model of the dentition, or some lesser combination, such as just the brackets, just the dentition, or the brackets and the archwire but not the teeth. The same holds true with other appliance systems.

In a preferred embodiment, the virtual model of teeth comprises a set of virtual, individual three-dimensional tooth objects. A method of obtaining the tooth objects from a scan of teeth, and obtaining other virtual objects of associated anatomical structures, e.g., gums, roots and bone is described. When the teeth are separated from each other and from the gums, they can be individually manipulated. Thus, the individual tooth objects can be individually selected and moved relative to other teeth in the set of virtual tooth objects. This feature permits individual, customized tooth positioning on a tooth by tooth basis. These positioning can be in terms or angular rotation about three axis, or translation in transverse, sagittal or coronal planes. Additionally, various measurement features are provided for quantifying the amount of movement.

One of the primary tools in the treatment planning apparatus is the selection and customization or a desired or target archform. Again, because the teeth are individual tooth objects, they can be moved independently of each other to define an ideal arch. This development of the target archform could be calculated using interpolation or cubic spline algorithms. Alternatively, it can be customized by the user specifying a type of archform (e. g, Roth), and the tooth are moved onto that archform or some modification of that archform. The archform can be shaped to meet the anatomical constraints of the patient. After the initial archform is designed, the user can again position the teeth on the archform as they deem appropriate on a tooth by tooth basis. The treatment planning software thus enables the movement of the virtual tooth objects onto an archform which may represent, at least in part, a proposed treatment objective for the patient.

Numerous other features are possible with the treatment planning software, including movement of the teeth with respect to the other teeth in the archform, changing the position of the virtual brackets and the teeth with respect to each other, or opposing teeth with respect to the selected archform. Custom archwire bends can be simulated to provide additional corrections. Bonding corrections at the bracket-tooth interface are also possible.

In another aspect, a method is provided for digital treatment planning for an orthodontic patient on a workstation having a processing unit, a user interface including a display and software executable by the processing unit. The method comprises the steps of obtaining and storing a three-dimensional virtual model of teeth representing the dentition of the patient in a current or observed situation. The virtual model is displayed on the display. The method further includes the step of moving the position of teeth in the virtual model relative to each other so as to place the teeth of the virtual model into a target situation and displaying the virtual model with the teeth moved to the target situation to the user. Parameters for an orthodontic appliance to move the patient's teeth from the current situation to the target situation can be derived from the virtual model and the target situation. For example, if virtual brackets are placed on the teeth, their location in the target situation can dictate the design of an archwire to move the teeth to the target situation.

In a preferred embodiment, the method includes the step of providing screen displays on the display enabling a user of the workstation to operate the user interface so as to place virtual three-dimensional objects representing orthodontic appliances, e.g., brackets, onto the surface of teeth in the virtual model. A library of the virtual brackets can be stored in memory and a landmarking procedure used to place the brackets on the teeth at the desired location. Anatomical considerations may dictate movement of the brackets from their originally selected. position to a new position. Accordingly, the software provides navigational tools enabling a user to change the position of the brackets relative to the teeth.

The treatment planning system is based on individual tooth objects which can be moved to any position in three dimensional space. They can be moved in several ways by direct user specified movement, and by adding an object comprising an orthodontic appliance and changing the configuration of the appliance to cause the teeth to move. For example brackets can be virtually bonded to the teeth and the position of the brackets changed in three dimensions to move the teeth. Alternatively, an archwire shape can be defined which fits into the slots in the brackets. Movement of the archwire can be simulated, resulting in a simulation of tooth movement.

The treatment planning software includes features enabling more accurate diagnosis. For one thing, the virtual model of the dentition can be manipulated in three dimensions at will, resulting in complete visual assessment of the model. Measurement tools are also provided by which the orthodontist can determine the distance between any two points on the model. This allows the user to quantify the patient's morphology both at initial and at target stages. Thus, treatment progress, proposed changes in appliance design, or tooth movement can be quantified precisely. By measuring the differences and changes in morphology during the care cycle, the orthodontist can quickly and accurately assess patient treatment. Changes in treatment can be made early on. The result is shorter treatment times (and the ability for the orthodontist to service more patients per year).

The treatment planning system incorporates virtual objects comprising orthodontic appliances that may be used to treat the patient. The system provides for design of complete appliance systems and simulation of various treatment scenarios prior to initiation of treatment.

A bite registration scan is obtained from the patient to spatially correlate the scans of the upper and lower jaws when the dentition is clenched. This scan is used to provide a registration of the upper and lower jaw to determine the correct relative position. This bite registration scan is usually needed at the beginning of treatment to set the relation between the upper and lower jaws.

Landmarks are then placed on the labial surfaces of all the teeth. The illustrated embodiment places landmarks manually, but this process could be automated. The landmarks are placed initially on the molars and the front teeth, and an estimated position for the landmarks on the other teeth can be made, such as in the same plane, based on the relative position of the landmark with respect to the gingival tissue and incisal edge of the tooth, or other factors.

The landmarks are placed at the location where the orthodontist expects to place an orthodontic bracket to correct the malocclusion. The bracket shape is shown on the monitor 30 (Figure 1). Three-dimensional templates for a variety of commercially available brackets are stored in memory and the software asks the orthodontist to select a particular manufacturer and style of bracket to use with the patient. Thus, as the landmarks are placed, virtual brackets appear in the computer model on the labial surfaces of the teeth where the orthodontist desires to place the brackets. The orthodontist can move the bracket position depending on the type of forces the orthodontist wishes to create on teeth to correct the malocclusion.

Figure 60 is a screen shot from an orthodontic workstation showing the computer model of the patient's teeth objects 312 positioned in a target or desired condition. The illustration is the result of the user selecting an archform for the patient from a known type of archform (e.g., Roth), and the computer placing the teeth along the arch selected by the user. This is executed by placing the virtual brackets the orthodontist placed on the teeth along the curve selected by the orthodontist. The brackets are omitted from Figure 60, but are shown in Figure 61. The software allows the orthodontist to change many variables in the target situation, simply by entering new values in the slide line area 320 of the screen display, by mouse operation of up and down arrows to scroll through available values, or by mouse operation of a bar to change the values, or other similar technique. Figure 60 shows some of the parameters by which the orthodontist can adjust the shape of the arch, the distance between the teeth, the distance between the molars, and other parameters, so as to provide a unique and customized target situation for the patient.

Figure 61 is another screen shot showing the computer model of the patient's teeth in a target situation, also showing the numerous parameters available to the orthodontist to customize the tooth position, orientation, angulation, torque, and other parameters on a tooth by tooth basis for the target archform. Virtual brackets 322 are positioned on the tooth objects 312 at the location where the user placed the landmarks. A virtual archwire 324 passes through the slots in each virtual bracket.

Figure 62 is another screen shot showing a front view of the target situation and additional parameters available to the orthodontist for simulating the movement and positioning of teeth relative to each other in planning treatment for the patient. For example, in Figure 62, the cursor is moved onto the virtual tooth 41 (in the tooth numbering convention) and the mouse is clicked. Tooth 41 is then highlighted. If the orthodontist wants to extract that tooth, they then click on the box 22 to extract the tooth in the simulation. Alternatively, tooth 41 could be rotated about any of three axis of rotation, moved in the X, Y or Z direction, or a larger or smaller gap could be created between teeth.

Figure 63 shows the target situation for the upper arch, with the virtual brackets 322 in place. The orthodontist can adjust the bracket 322 position, archwire shape 324, or tooth 312 position, on a tooth by tooth basis to thereby optimize treatment planning for the patient.

The result of the treatment planning is the generation of a set of bracket placement positions and the display on the monitor of the shape of a customized orthodontic archwire to treat the malocclusion. Information as to the location of the brackets, the three-dimensional model of the malocclusion, the three dimensional model of the target situation, and the type of archwire to use are sent to the precision appliance centre 26 of Figure 1. A customized orthodontic archwire is manufactured in accordance with the bracket location and type and the target situation for the patient. Additionally, a transfer tray is manufactured to assist the orthodontist to place the brackets at the proper location. The transfer tray, brackets and archwire are shipped to the orthodontist's clinic 22. The orthodontic appliance is then applied to the patient and treatment commences.

Because the hand-held scanner allows for scans of the dentition in a matter of minutes, the scanner becomes an important tool in monitoring treatment. As the treatment progresses, the movement and position of the teeth during treatment can be quantified with a high degree of precision. The orthodontist can discern during treatment that corrections in the wire need to be made, for example due to biological influences affecting tooth movement. The treatment planning software on the workstation displays the current situation, and also the target situation. A new customized archwire is designed on the computer. The relevant information for making the new archwire is sent to the precision appliance service centre and a new archwire is manufactured and shipped to the clinic.

Monitoring scans are taken during treatment to measure and quantify progress and detect deviations from the expected treatment. Since each of the tooth objects is already stored, the monitoring scan need not be of the entire dentition, but rather needs to only be of one surface, such as the occlusal surface, or the lingual surface, or some combination of the two. A bite scan with the teeth in a clenched condition is also taken to get the current upper and lower relation. The position of the rest of the teeth is obtained from the virtual tooth objects of the patient's teeth. After the monitoring scans are performed, a registration is performed of the scanned data relative to the tooth models to complete the teeth and derive a current virtual model of the patient at that point in treatment. Study of this virtual model and comparison to the target virtual model of the teeth and the virtual model at the beginning of treatment can indicate whether progress is as anticipated or if additional correction to the orthodontic appliance need to be made. These corrections will typically be carried out as wire changes in an archwire orthodontic treatment regime, in which new bends are placed in the orthodontic archwire.

Initial virtual bracket placement

With the individual teeth now cut from the three-dimensional model of the dentition and represented as tooth objects, they can be moved relative to each other in three dimensions. Since orthodontics assumes that a bracket is fixedly bonded to a tooth, by moving the bracket one moves the tooth. The next step in the process is thus selecting an initial location to bond the brackets to the tooth. As noted below, this initial location can be adjusted by the treatment planning software. The spatial location of the surfaces of the bracket and the surfaces of the corresponding tooth are known. Collision avoidance algorithms are used to keep the bracket positioned on the surface of the tooth and prevent the virtual bracket from entering the tooth itself, a clinically undesirable result. The user is able to move the bracket independently of the tooth by activating an icon (such as one shaped like a magnet to signify the mating of the bracket to the tooth). When the bracket is moved to the new location, the tooth matches up to the surface of the bracket.

The brackets are represented in the software as virtual three-dimensional objects, and the surface of all the brackets and the teeth are known in three-dimensional spatial coordinates. Accordingly, collision detection algorithms are employed to detect when simulated tooth or bracket movement would result in a collision between brackets and teeth. Similar collision algorithms are provided to prevent the adhesion surface of the bracket from migrating into the body of the virtual tooth object and to keep the brackets located on the surface of the teeth. If the user wishes to move the location of the brackets, the movement of the teeth follows the movement of the bracket. Also, again since the bracket is a three-dimensional virtual object with known spatial coordinates, the user is provided with a tool (such as an icon) which when activated allows the user to move the bracket about one plane or axis, and freeze the movement in the other directions.

Initial virtual bracket placement is done as follows. Landmarks are placed on the labial surfaces of all the teeth. The landmarks are placed at the location where the orthodontist expects to place an orthodontic bracket to correct the malocclusion. The bracket shape is shown on the monitor. Three-dimensional templates for a variety of commercially available brackets are stored in memory and the software asks the orthodontist to select a particular manufacturer and style of bracket to use with the patient. Thus, as the landmarks 302 are placed, virtual brackets appear in the computer model on the labial surfaces of the teeth where the orthodontist desires to place the brackets. The orthodontist can move the bracket position depending on the type of forces the orthodontist wishes to create on teeth to correct the malocclusion. Because the brackets are individual objects and stored in memory, when they are placed on the surface of virtual teeth complete position information is known in three dimensions. As such, the brackets can be displayed either alone, or in conjunction with teeth, or hidden from view, by means of appropriate user specified commands on the user interface. For example, the screen display showing the target or current stage can have an icon indicating hide brackets, or display brackets, and activating the icon causes the brackets to be hid or displayed. The same is true for other virtual objects that exist independently of other objects, such as tooth models and the archwire.

With the teeth now separated into individual tooth objects, the orthodontist can now view the current target stage, custom design a target situation for the patient, and design the appliance to treat the malocclusion. These aspects will now be described in further detail.

Viewing the observed (current) stage

Figure 69 is a screen shot showing a three-dimensional model 18 of a malocclusion, showing the teeth 312 in both the upper and lower arches 326 and 328, respectively. The screen 330 includes a row of icons 332 across the upper portion of the display, which are associated with various tools available to the user to view the dentition, virtual brackets, and current and target archforms. The lower portion 334 of the screen includes a set of tabs 336 that are accessed in various aspects of treatment planning. These tabs 336 include a patient tab 338, which accesses the screen of Figure 66. A limits tab 340 allows a user to breakdown the tooth movement between observed and target stages into stages, such as 30 percent, 50 percent and 75 percent, and display the tooth positions for both arches at these positions. A differences tab 342 quantifies the differences (in terms of translation and rotation) between the observed and target stages for each tooth. The space management tab 344 permits the user to simulate extraction of one or more teeth and adjust the spacing between teeth in either arch. A bonding correction tab 346 allows for adjustment of tooth position to be realized via bonding corrections. The technique tab 348 allows the user to select a bracket prescription and default settings for bracket height (distance from bracket slot to incisal edge of tooth). The tab also displays the parameters for the bracket prescription chosen by the user. The upper/lower (U/L) relations tab 327, selected in the screen shot of Figure 69, allows the user to modify the relation of the upper and lower jaws, by both translation in three axes (transversal, sagittal and vertical directions) and by rotation about these axes. The user manually enters values in the field 350 to change any parameter, and the change is immediately reflected in the view of the model of the dentition.

The tabs also include a bracket offset tab 352 that allows a user to reposition the bracket on a tooth and specifies numerical values for each bracket placement modification. A brackets tab 354 allows a user to enter information as to the type or manufacturer of brackets for each tooth in the both arches.

A further "morphing" tab could be provided which would animate the movement of the teeth from malocclusion to target situations based on treatment steps or limits defined by the user (explained in further detail below).

The screen shot of Figure 69 also includes a region 356 that allows the user to navigate between views of the observed stage and views of the target stage. Here, the user has highlighted or selected both arches in the observed stage, so the screen display shows the model of the dentition in the current or observed stage.

Referring to Figure 67, the treatment planning software preferably displays a plurality of icons 331, not all of which are shown in Figure 69, to enable the user to quickly and easily view the three dimensional model in various standard perspectives. For example, the icons 331 of Figure 67 include icons 333, 335, 337 and 339 for viewing the dentition in top, bottom, right hand side and left hand side views, respectively. An icon 341 is provided which zooms in or out. Icons 343 and 345 allow the user to select for viewing just the upper or lower arches, respectively, including virtual teeth, virtual brackets and virtual archwire. The icon 347 allows the user to show or hide the virtual dentition, excluding brackets and archwires. An icon 349 allows the user to select or deselect the virtual brackets. A marker icon 341 is used for measurement functions (described below) and an object navigation icon 353 is used for manipulating any of the virtual objects on the screen.

When positioning multiple objects in the three-dimensional view, such as shown in Figure 69, the camera navigation icons of Figure 67 move all the elements together. As shown in Figure 70, the initial placement of the virtual brackets 400 can be displayed along with the teeth. Further, the camera navigational tools allow the user to zoom in an zoom out in any desired degree. However, the virtual teeth 312 and virtual brackets 400 are individual three-dimensional objects which can be selected and moved independently. One way of moving objects is by entering new positional values (e.g., in terms of mm of displacement or angle of rotation, as described later). Another method provided by the software is using object navigational controls, activated by clicking the icon 353 or by accessing the function via a tools menu. The object navigation controls allow the user to move the object based on orthodontic judgment and visual feedback. The amount of movement is stored and can be displayed using numerical position information. As will be discussed in further detail below, the bracket position can be individually adjusted on a tooth by tooth basis. Furthermore, the camera navigation icons permit navigation of the archforms (i.e., the teeth placed on some-selected archform), navigation of the brackets, or navigation of the archwire.

The object navigation tools first require an object (e.g., tooth, bracket, archwire, etc.) to be selected. To select and deselect any object displayed on the screen, the user places the cursor over the object and double clicks the mouse. The selected object is highlighted in a separate colour. Additional objects are selected by pressing and holding down the <CTRL> button while double clicking additional objects. To magnify the object, the object is selected as described above and the icon 341 is clicked.

To move the selected objects, the software provides an object navigation icon 353. When the icon 353 is selected, object navigational tools appear on the screen 330. These navigational tools 355 are shown in Figure 68 and Figure 69. The object navigational tools 355 comprise a large circle 357, a small circle 359 and a small rectangle 361. First, the object is selected as described above. Then, the object navigation icon 353 is clicked, activating the tools 355 such that they are displayed. The user then positions the mouse pointer relative to the tools 355 and presses and drags as described below to position the object. When the mouse pointer is outside the large circle 357, when they start dragging the object is turned either clockwise or counterclockwise depending on the direction of dragging. When the mouse pointer is positioned within the large circle 357, when they start dragging they rotate the object in any direction. When they start dragging from inside the small circle 359, the object is moved in one plane. When they start dragging from inside the rectangle 361, by dragging down the object is moved closer, by dragging upward the object is moved farther away.

Measuring objects

Referring again to Figure 67, the icon 351 allows the user to establish a measurement marker on any portion of the virtual model of the dentition. The user uses the icon 351 to place markers at any two points on the dentition and the distance between the markers is displayed on the screen.

To use the icon 351, the user clicks on the icon, and then clicks anywhere in the 3-D view of the dentition to place markers. A straight line is drawn between two markers. The distance between the markers appears on the screen, e.g., at the upper left hand corner of the windowpane of the 3-D view. A tool in the Tools menu in includes a DELETE ALL MARKERS function to delete the markers.

The measurement function allows the user to measure tooth size, inter-molar width, inter-canine width, the arch length, curve of spee, and other important orthodontic and diagnostic parameters. The measurement aspect is particularly significant in that it permits precise quantification of simulated tooth movement, both in terms of establishing initial treatment plan as well as monitoring treatment.

Viewing cross-sections of model

The viewing options also include a clipping plane feature by which cross-sections of the teeth through any desired plane are possible. As shown in Figure 73, the clipping plane is shown through the upper jaw, but the user at can move this plane in three-dimensional space at will. If the teeth are magnified, this clipping plane feature is very useful for inspecting contact points between the upper and lower jaw, viewing and adjusting the upper and lower jaws in the initial bite registration, and adjusting the location of the occlusal plane. For example, in Figure 74 the clipping plane is shown through the upper and lower incisors 312A and 312B.

The clipping plane is manipulated like an object with the object navigational tools shown in Figure 68. The plane is accessed using a tools menu and the user highlights or selects SHOW CLIPPING PLANE. A plane appears on the screen. The user then clicks on the object navigation icon 353 (Figure 67). The object navigational controls 355 of Figure 68 then are displayed. The user then positions the mouse pointer over the navigational controls 353 to adjust the position of the clipping plane. When they start dragging in the region outside the large circle 357 (Figure 68, Figure 73), the plane is turned clockwise or counterclockwise. Then they start dragging inside the large circle 357, the plane is rotated in the direction indicated by the dragging. When they start dragging from inside the small circle 359, the clipping plane is moved in the direction of the dragging. When they start from inside the rectangle 361, if they drag up they cut less into the model, by dragging down they cut further into the model.

Viewing and adjusting initial bite registration

The first step in typical treatment planning is deciding where to place the teeth in three-dimensional space. This will ordinarily involve a definition or fixation of the vertical level of the teeth relative to the bones, and defining an occlusal plane, and adjusting the occlusal plane sagittally and transversely. This, in turn, will ordinarily involves an assessment of structural relationship between the teeth and the maxilla and mandible. The orthodontist performs this by accessing and studying X-ray, CAT scan, photographs or other two dimensional data stored in the patient records portion of the treatment planning software, and of course the three-dimensional model of the malocclusion, with the teeth either represented as individual tooth objects or in context with the surrounding anatomical tissue. The mid-sagittal profile of the incisors and molars is set up by superimposing the mid-sagittal plane of the teeth over the X-ray.

Figure 66 is a screen shot from the workstation running the treatment planning software showing a patient information screen. The screen includes a region 500 for storing various photographs 502 of the patient's head and face, and various views of the patients dentition. The photographs are taken with a digital camera and loaded into the workstation, and accessed from the treatment planning software. The patient information section of the software also includes separate screens for entering and displaying other pertinent information for the patient, accessed through a menu 504. These additional screens (not shown) include the patient demographics, patient medical, dental and orthodontic history, examination notes, and X-rays.

To assist this process, the treatment planning software provides the ability to view and adjust the initial bite registration. The initial bite registration between the upper and lower arches can be modified using the U/L relation tab 327 of Figure 69. The user can move or rotate the lower jaw relative to the upper jaw by entering values in the field 366. The user can also simulate a chewing motion of the upper and lower jaws by moving the slide bar 368 down. During this simulation the lower jaw moves from side to side and up and down to simulate a chewing motion.

Figure 71 shows how the three-dimensional viewing area on the screen on the workstation can be broken up into separate screens using known windowpane techniques. Here, in windowpane 360, the area of the molar in the observed stage is displayed, with the orthodontist able to assess the upper and lower relation and change values for the upper and lower relation in three planes of space. Simultaneously, windowpane 362 shows the upper and lower jaws as seen from above. Windowpane 364 shows the dentition looking from the molars out towards the incisors, a view the orthodontist would otherwise not be able to access without the three-dimensional virtual model. These various views, plus the clipping plane tool, and the X-Ray and patient photograph data in the patient records portion of the software, provide a complete suite of tools for effective orthodontic diagnosis, treatment planning, and appliance design, including initial bite registration.

Figure 72 is another screen shot showing the three-dimensional model of the dentition in the observed stage. No values have been entered in the malocclusion field 366 in the U/L Relations tab. By inspection of the upper and lower jaws (using magnification or clipping plane features if needed), the user can set numerical values in field 366 and immediately simulate the corresponding tooth movement to arrive at a desired upper and lower initial bite registration.

Design of Target Archform

Referring to Figure 75, after the user has set the initial level of the occlusal plane and inspected the initial observed situation, the next step is selection of a desired or target archform for the patient and the midline. The orthodontist will have previously selected or indicated a general type of archform for the patient, e.g., Roth. The treatment planning software allows the user to generate a target arch based on this archform for the individual patient. The user highlights the observed stage in region 356 and then right clicks the mouse. The pop-up menu 370 appears and the user selects INSERT TARGET STAGE. The target stage is arrived at by creating a flat virtual wire that runs through the virtual bracket slots to create a virtual arch line. The arch shape is based on the user's selected preference for the arch shape, based on the patient's facial anatomy, bone structure, malocclusion, and other parameters using the orthodontist's judgment. The wire target shape has a best fit through the malocclusion bracket slot positions. In one embodiment this wire is flat. It is also possible to design the wire to adapt to the malocclusion in the vertical direction to create a Curve of Spee if desired. The geometry in the transverse direction can also be changed, such as the transverse curve of Monson establishing an inclination of the teeth in the coronal plane.

Figure 76 is a screen shot from an orthodontic workstation showing the computer model of the patient's teeth objects 312 positioned in a target or desired condition. The illustration is the result of the user selecting an archform for the patient from a known type of archform and the computer placing the virtual brackets along the arch selected by the user. This is executed by placing or threading the virtual brackets along the archform or curve selected by the orthodontist. The brackets are omitted from Figure 76, but are shown in Figure 77. When the initial target archform is created, the slide line tab shown in Figure 67 is activated.

The initial target archform presented to the user in Figure 76 is only an initial archform. The treatment planning software allows the orthodontist to change many variables in the target situation, simply by entering new values in the slide line area 402 of the screen display. Figure 19 shows some of the parameters by which the orthodontist can adjust the shape of the arch, including the distance between the cuspids, the distance between the rearmost molars, the centre line offset, and the radius of curvature at the front of the teeth. Slide line area also permits the user to select a symmetrical archform or an asymmetrical archform, and apply corrections on the right and left quadrants as indicated. As values are entered in area 402, the shape of the archform is instantaneously modified on the screen display, allowing the user to simulate various potential archform configurations for the patient.

In generating the archforms shown in Figures 76 and 77, the user will ordinarily set up the lower arch first. The upper arch is automatically derived from the lower arch. The user can view the arch forms in several ways using three arch icons 390, 392 and 394. The arch icon 390 is for the malocclusion arch, which causes a blue line to appear on the screen which exhibits the curvature of the observed arch or malocclusion. The line passes through the slots on the virtual brackets, as placed on the teeth. The arch icon 392 is for the target arch, which represents a custom archwire passing through the bracket slots of the teeth in the target situation. The line 396 in Figure 76 represents this arch. The arch icon 394 if for an ideal spline or curve generated by the software to represent an optimal shape. The arch retains a parabolic shape as the user manipulates the target arch using the entries in the slide line. The numerical values in region 398 of the slide line tab represent checkpoints and boundary conditions on the ideal spline associated with the icon 394. These values can be edited as indicated.

Since the software allows for generation and display of a malocclusion archform and a planned archform, the difference between the two archforms indicates the space needed to control arch length inadequacy; i.e., to identify the need for interproximal reduction, extraction, or control of gap size. Interproximal reduction can be achieved by the clipping plane feature, and the simulation of shaping of individual tooth objects in three planes of space. The simulation of extractions and control of gap is provided for as explained next.

Space Management -- Management of Extractions and Gaps Between Teeth

The treatment planning software provides a space management tab 344 that is used for managing space between teeth, including tooth extraction. Figure 82 is a screen shot showing the target situation of both the upper and lower jaws. The user clicks on the space management tab 344, and the region 420 of the tab appears. The region 420 allows the user to simulate the extraction of a tooth in either the current or target stage. Here the user is simulating the extraction of tooth number 41 by clicking on the appropriate cell for that tooth in the rows for current and target stages. The result is shown in Figure 83. The extraction of tooth 41 is simulated. The region 422 also allows the user to enter values for the mesial gap size between teeth. A default value of 0.1 mm is provided. However, the user can change these values. For example, the user can enter any values in the row of cells for mesial gap size as shown in Figure 84 (in this example 2 mm). Note also in Figure 84 that the simulation of the extraction of tooth 41 is not being performed since the cell 423 is not checked. The tooth moves distally if a positive number is typed in the cell for that tooth in the mesial gap size row 424. If a negative number is typed into the cell, the tooth moves mesially.

Adjusting virtual bracket position

After the upper and lower archforms have been optimized, the user may wish to adjust the position of the virtual brackets 400 on the teeth. The step of adjusting the virtual bracket position can also be performed prior to the design of the archform.

The vertical position of the virtual brackets relative to the incisal edge of the virtual teeth is one adjustment that can be made. This distance can be measured using the measurement, tool described earlier. Bracket manufacturers have recommendations for this distance. If the initial landmarking has placed the brackets outside of the recommended distance, this distance can be adjusted by using the object navigational tools. Alternatively, the user can select preferred values for the archform and the bracket set (bracket manufacture, bracket technique and wire type, e.g., straight) and the landmarks and virtual brackets will be placed on the teeth at the recommended distance.

The bracket placement can also be performed interactively by the user. The user looks at every tooth 312 one by one (using a screen such as the screen shot of Figure 75) to see if they are basically satisfied with the bracket 400 position, e.g., angulation, side to side position, and its relation to teeth in the opposing jaw. The user may be performing this step while viewing the malocclusion, or the target stage. To improve the position of the virtual bracket 400, the user selects the bracket, zooms in if necessary, and adjusts the position of the bracket on the tooth surface using the navigational tools as described earlier.

Correction of Individual Tooth Position In Target Archform

After the archform has been designed and the bracket placement optimized, the user can adjust the individual tooth position on a tooth by tooth basis in the target arch. There are three ways this can be performed. First, the user can use the tables in the Target Correction tab. See, for example, Figure 78, in which the user has entered a value of -15 degrees for rotation of tooth number 41, and the tooth is rotated by that amount. The correction is realized by a bend in the archwire 396. The bracket position on the tooth does not change in this example. The target corrections tab 410 permits any values to be entered for mesial, buccal and coronal translation in three planes of space, and torque, angulation and rotation movements about three orthogonal axis. Thus, independent tooth position corrections are available in 6 degrees of freedom for every tooth, merely by entering values in the tables in the target corrections tab 410. Another example is shown in Figure 81, in which a new target position is shown for tooth number 41.

Secondly, the user can move teeth using the Bonding Corrections tab 346 of Figure 69. An example is shown in Figure 85. The bonding corrections tab 346 allow the user to enter new values for tooth position for any tooth in the arch by merely entering values in the appropriate cell. The selected tooth 312A (tooth number 41) is moved as indicated (here, a new rotation value of -15 degrees is entered). The virtual bracket remains in the same location in space and the gap 412 between the pad of the bracket and the surface of the tooth is taken up by a bonding adhesive when the bracket is bonded to the tooth.

Examples of common tooth movements that can be simulated using the bonding corrections tab or the target corrections tab are moving the lower incisors buccally or lingually, inclining the incisors axially, and levelling the incisors.

Thirdly, the user can simulate tooth position corrections interactively using the navigational tools. The user displays a target stage as needed. A tooth object is selected as explained above. The user clicks on the zoom icon 341 of Figure 67 to zoom in or out as needed. The user then clicks on the object navigation icon 353 to display the object navigation controls. The user then uses the navigation controls to move the tooth as desired. The movement of the tooth is recorded as new values in the bonding correction and target correction tables, in case the user wants to quantify the movement or use those tables for further modification of the tooth position. After the user has moved the tooth to the new position, they click one of two check mark icons 414, 416 (Figure 77, 78) that are highlighted on the screen. The blue check mark 414 realized the new tooth position via a bonding correction. The red check mark 416 realizes the new tooth position via a wire correction.

Another example of correction of individual tooth position is shown in Figures 79 and 80. In Figure 80, the target situation is shown, both with the virtual tooth objects 312 and the virtual brackets 400. Note that with tooth 16 (312A), there is a gap between the rearmost cusp of the tooth and the opposing tooth. The orthodontist can correct this gap by building in a bracket offset, basically repositioning the location of the bracket 400 on the tooth 312A by entering an amount in the table 450 in the angulation cell for tooth number 16 (here -15 degrees). The result is shown in Figure 80. The change in the angulation value causes tooth number 16 to rotate back into a more desirable occlusion with the opposing tooth.

Wire Tab

The wire tab 432 of Figure 86 allows the user to make wire configuration changes, while not changing bracket position. Note that in Figure 86, the virtual wire 396 is shown isolated from the teeth and brackets. The virtual wire can be scaled to actual size and printed out and provide a template for a manual bending of a wire if the orthodontist chooses not to obtain a wire from the precision appliance manufacturing centre. The tab includes a section 430 where the user can view the distance in mm in which the wire will slide relative to the brackets, on a tooth by tooth basis, when the teeth are moved from the current situation to the target situation. Note that a collision is detected for tooth 22 in movement of the tooth from the current situation to the target situation. This can be resolved in several possible ways in accordance with the teachings of U.S. Patent ....... to Sachdeva et al., serial no. 09/451,609 allowed December 7, 2000.

Additional Wire Bending Corrections

The wire offsets tab 426 (see Figure 73 and Figure 87) allows the user to simulate bending a wire 396 to the corrected tooth position while simultaneously retaining the original bracket position. Note that in Figure 87 the wire 396 is flat with no corrections indicated. The user highlights one of the teeth in the virtual model and enters new values for tooth position. The change is reflected in a new shape for the wire 396. The tab also allows the user to build in over/ under compensation into the archwire. These settings do not affect the bracket position. The characteristics of the wire bends, such as the radius of curvature, can be controlled by accessing a sidings tab (not shown). Another tab, forces 428, displays approximations of the forces the wire applies to each tooth to displace it from its initial position to the target position.

Treatment Stages

Since the difference between the current situation and the target situation is quantifiable in terms of millimetres of movement or degrees of rotation about three axes, the treatment for the patient can be broken up into segments or stages with each stage defined arbitrarily. For example, the orthodontist can specify a first treatment stage as the movement of the teeth from the initial position half-way to the final position. The software includes a screen that permits the user to view the position of the teeth when moved to the half-way position. Basically, the simulation simply multiplies the differences in tooth position between initial and target stages by 0.5 and the resulting tooth positions are displayed on the workstation. Additionally, the user can specify further intermediate positions, such as one fourth or three fourths. With this feature, the orthodontist can monitor treatment and compare the progress of the treatment with the limits or stages that have been set. When the patient comes in for a visit during treatment, the patient's dentition is scanned. The three-dimensional model of the now current situation is compared with the defined stages and perhaps with the target situation. Difference between actual progress and the planned treatment can be quantified. Changes to the archwire can be designed using the treatment planning software to move the teeth in the desired direction to account for unexpected biological influences that may be observed.

The above description of treatment planning has been predicated on the use of a three-dimensional model of the dentition from the scanner. However, it is possible to perform digital treatment planning by importing into the software three-dimensional software from other sources. It is known today that three-dimensional models can be exchanged with different software programs in different file formats, similar to the translation programs that convert text documents from one type of file (e.g., Microsoft Word to WordPerfect). Most three-dimensional applications have several import filters for different 3D formats.

However, there are generally two different ways to describe three-dimensional objects: by surface representations and by solid representations. A 3D file that holds 3D data in a surface description consists typically of triangles that form the surface of the object. The STL format is one of the oldest and therefore most common formats that uses triangles. It is used to feed information to stereolithography machines. A more detailed description of STL can be found at http ://www.mmsonline.com/articles.019704.html.

Treatment Monitoring

Interactive, computer-based treatment monitoring is a significant advantage provided the treatment planning and appliance design aspects of the system described herein. Typically, when the patient comes into to the office during treatment, they will be scanned and a new digital model of the dentition is acquired. From this new model, differences can be monitored between the current situation and the original malocclusion, and differences between the current situation and the target situation or pre-defined limits or treatment stages as defined earlier. These differences can be quantified with precision. For example, a point on the tooth in the current model is selected, and the model of the tooth at the original malocclusion is overlaid on the screen. The superposition of the two teeth allows the user to view the change in position that has occurred. The measurement marker features described earlier allow the user to quantify precisely the amount of movement.

Any deviations between the therapeutic result that is observed and the expected result can be captured precisely and at an early stage in treatment using the scanning and treatment planning features described herein, and corrected for. For example, the orthodontist may need to place additional bends in the archwire. Such additional bends can be performed by simulating the wire shape on the screen, displaying the wire only on the screen, and printing out the screen and using it as a template for bending the wire. The current situation could also be forwarded to the precision appliance centre for manufacture of a new appliance. Of course, these monitoring and treatment corrections are applicable to any type of appliance selected for the patient.

Part 5: Appliance Manufacturing

The appliance that is manufactured in accordance with the treatment planned for the patient can vary within the scope of the invention and will depend on the application, including the type of appliance desired by the orthodontist and the patient. Obviously, the treatment planning and simulation features described above can be used in wide variety of treatment regimes, including flat wires and brackets, finishing wires, retainers, Herbst devices, expansion devices, and removable, transparent aligning devices such as those furnished by Align Technologies. For example, the movement of the teeth from the current or observed stage to the target or treatment stage can be broken into a series of movement steps. For each step in the process, the position of the teeth in that stage is known by virtue of the manipulation of the individual tooth models in the treatment planning software. These tooth positions can be used to manufacture the aligning devices.

In a representative embodiment, the results of the treatment planning software are used to generate a customized orthodontic archwire and a bracket placement tray for precise placement of off-the-shelf brackets onto the patient's teeth. When the treatment planning features have been executed to the satisfaction of the orthodontist and the proposed target situation finalized, the treatment planning software will store the following information (in addition to the patient records):

1) the virtual model of the current stage or malocclusion;
2) the placement location of the brackets on the malocclusion, including the type and dimensions of the brackets;
3) the orthodontist's selection of a type of archwire (including material and size);
and
4) the target situation, including the location of the teeth and brackets in three dimensions at the target situation.

Note that it is not absolutely necessary for the appliance manufacturing step to calculate or even know the shape of the archwire. Archwire geometry is dictated by bracket positions in three-dimensional space when the teeth are in the target situation. This bracket position information is included in the target situation, no. 4) above.

With reference again to Figure 1, the above information from the treatment planning software is sent over a suitable communications medium 24 in digital form to the precision appliance service centre 26. The service centre manufactures a customized archwire and a bracket placement tray for placement of the brackets at the intended location on the teeth in the malocclusion.

Basically, the position of the bracket slots, and the shape of the brackets, when the teeth are in a target situation, is information that is ultimately developed and stored by the treatment planning software. This position of the bracket slots and the shape of the slot (e.g., the length) is of course known in three dimensions. From the slot shape, it is possible to derive a three-dimensional set of line segments that represent the shape of an archwire passing through the bracket slots in the target situation, and calculating the optimal shape of bends that connect the bracket slots together. The positions of the straight sections and the bends are fed as an input file to a wire bending robot. The wire bending robot need only know the wire size, the shape and size of the slots, and the positions of the slots in the target situation. From this information, robot commands are generated to bend the archwire into the desired shape.

The bracket placement tray is separately manufactured using stereolithography or other similar technique. The treatment planning software generates items 1) and 2) above, and superimposes the brackets on the teeth to generate a three-dimensional model comprising the three-dimensional tooth objects plus the virtual brackets at their intended locations in the observed stage or malocclusion. This three-dimensional model is supplied to a stereolithography (SLA) instrument. The SLA instrument manufactures a plastic model of the teeth with the brackets superimposed on the teeth. A thermoplastic foil is placed above the SLA model and the model and foil are placed within a pressure chamber. The chamber is pressurized so that the foil envelops the dentition and the brackets. After cooling, the foil is removed from the model. The foil, now in the shape of a transfer tray, has small indentations where the brackets are located. Real brackets are placed in these indentations. The orthodontist uses indirect bonding techniques to bond the brackets to the teeth. The transfer tray positions the brackets on the teeth at the desired location. After the bonding is complete, the orthodontist removes the transfer tray, resulting in the brackets bonded to the teeth at the desired location. A further scan of the dentition can be made at this step to verify the position of the brackets. Any substantial deviation in the bracket position can be accounted for by modification of the archwire, again using the treatment planning features described above.

There will always be some small gap between the bracket base (the part bonded to the tooth) and the tooth, as an off-the-shelf bracket will never precisely match the individual tooth of any given patient. One option is to fill the gap using a surplus of bonding adhesive during bonding. Another option is to equip the base of the bracket with a customized pad made from an adhesive.

Customized bracket pads can be manufactured using a variety of techniques. One possibility is to bond a blob of adhesive to a bracket base, and mill the blob using a milling machine to match the tooth surface. Since the tooth surface is known precisely from the scanning, and the position of the bracket base relative to the surface is also known precisely, a very accurate bracket pad can be manufactured in this fashion. Another technique is to stamp out a bracket pad using stamping machine either in the desired shape or as a blank and milling the stamped pad to the desired shape. A third possibility is creating a negative of the customized pad, forming the pad in a mould, bonding the pad to the bracket and the orthodontist trimming the pad as necessary when the pad is bonded to the tooth.

Once the brackets have been bonded to the teeth, a scan of the bracket placement is made. The scan is compared to the digital template of the expected bracket position. If the bracket is placed incorrectly, the bracket can be re-bonded to the tooth. Alternatively, corrections to the wire may be made if necessary to account for displacement of the brackets from their expected position. Basically, this is done by simulating the position of the teeth with the actual bracket placement at the target situation and correcting the shape of the archwire as necessary, or obtaining a new archwire based on the actual placement of the brackets.

It is also possible to manufacture a transfer tray without the intermediate step of a positive SLA model representing the dentition and the brackets. These trays may be either similar to the plastic sheets and just be fabricated using rapid prototyping methods (SLA, laser sintering, milling, 3-D printing, etc.), or they have more sophisticated geometry including features to hold the brackets and features to provide proper positioning at the dentition. Additionally, bracket placement jigs can be milled from the three-dimensional models of the dentition and the brackets using techniques described in, for example, in the Andreiko patent cited earlier.

As another possibility, the customized bracket bonding pads based on the virtual tooth/bracket geometry can be fabricated. Such bonding pads may include additional material which conforms to the surface of the tooth to act as a placement jig for the tooth to allow the bracket bonding pad to be precisely located on the tooth. Then the bracket is bonded to the bracket bonding pad. The additional material is then removed.

With the above description in mind, it will be appreciated that the treatment planning functions can be performed with the importation of any 3D object from any source. While the preferred embodiment uses the scanned three-dimensional model from the handheld scanner, this is not necessary or required. The software preferably has import filter for common types of files of 3D objects including STL, DXF, VRML etc.

Additionally, another key aspect of the treatment planning software is that it permits the placing of brackets onto tooth models in an arbitrary manner and virtually "bonding" those brackets to the teeth so that they move together. While the present embodiment has described a landmarking feature by which the user places landmarks on the teeth and the brackets are placed on the landmarks, this may be performed in other ways, including automatically with no user involvement based on parameters such as crown height, bracket type, tooth size, etc.

As indicated above, the software also provides for aligning of the brackets along virtual wire of any shape. Thus, as the user changes the shape of the archwire, the brackets follow this shape, thereby indicating tooth position correction in the target situation. This feature allows the user to drive the treatment planning based on wire shape. Conversely, the wire shape can be dictated by the placement of the brackets on the teeth. Thus, the treatment plan can be one driven by bracket placement. Obviously, wide variety is possible in the shape and design of the wire.

Furthermore, by providing for the simulation of teeth in both maxilla and mandible together, the software provides for a wide variety in defining the maxillary/mandible relationship, the occlusal plane and the mid-line of the teeth.

Another significant aspect of the software is that it provides for virtually unlimited 3D measuring features using the marking icon and measurement feature described earlier. This feature offers a powerful diagnostic tool, as well as a tool for monitoring the progress of treatment and quantifying results.

Because the teeth are represented as complete three-dimensional virtual objects, it is possible to detect the collision between teeth or between teeth and brackets in the simulation of movement of teeth from the current to the target situation. The point cloud representing tooth objects defines a surface, when surfaces come in contact during the tooth movement a collision is simulated. This collision is detected by suitable collision detection algorithms. When the collisions are detected, the user can be notified of the collision and resolve the conflict between teeth, for example by selecting one tooth as a primary tooth and moving that tooth first in an initial stage of treatment, and then moving the other tooth in a secondary stage of treatment. These features are described in further detail in US Patent application serial no. 09/451, 609 filed November 30, 1999, now allowed.

Another advantage of the instant treatment planning software is that it offers the user real time simulation with immediate feedback on the effect of user specified tooth movement. When a value is entered into a field in the display or the user uses the navigation tools, results are displayed immediately. Further the system offers arbitrary access to every object, including election, navigation and export.

A. Robot design

Figure 88 is a schematic representation of a presently preferred archwire manufacturing system 34 shown in Figure 1. The key aspects of the system 34 are shown in block diagram form in Figure 88A. The system 34 includes a control system for controlling the operation of a wire bending robot 604. The control system in the illustrated embodiment comprises a general purpose computer 600 running a bending program and a robot controller 602. The computer 600 receives an input file from the precision appliance service centre computer that contains information as to the location of bracket slots in three dimensions, when the teeth are in a target situation. The computer 600 supplies the robot controller 602 with wire position information corresponding to points along the wire where bends need to be made. This information is translated by the controller 602 into motion commands for the wire bending robot 604.

It will be appreciated that the system works in an analogous fashion when bending other types of medical devices. The computer 600 receives an input file from some source that provides information as to how the medical device in question needs to be bent. The computer 600 supplies the robot controller 602 with position information corresponding to points along the length of the medical device where bends need to be made, and the robot responsively bends a medical device in accordance with the input file.

The wire bending robot 604 consists of a moveable arm 606 having a gripping tool at the distal end thereof. The moveable arm has a proximal end mounted to a table or base 610. The robot also includes a first gripping tool 608. In the illustrated embodiment, the first gripping tool 608 is fixed with respect to the base 610 and mounted to the table. Hence, the first gripper tool 608 will be referred to herein occasionally as the "fixed gripper". It would be possible to place the first gripper tool 608 at the end of second robot arm, in which case the first gripper tool would not necessarily be fixed, but rather would be free to move in space relative to the source of archwires, or the other moveable arm. In such as system, a coordinate system would be defined having an origin at which the first tool is positioned at a known position. The bending commands for the robot would be with respect to this known point.

A wire or other workpiece to be bent is held by the first gripper 608 and the gripping tool at the end of the moveable arm 606, and the arm is moved to a new position in space to thereby bend the workpiece. The details of the construction of the arm 606 and fixed gripper 608 are described in further detail below.

The system 34 of Figure 88 is set up to manufacture customized archwires one after the other continuously, as would be case in a precision appliance service centre serving a plurality of clinics. As such, the robot 604 includes a source of archwire material. The source could be a spool of wire in which case a cutting tool is needed to cut lengths of wire for individual archwires. Alternatively, as shown in Figure 88 and 93 the source consists of a magazine 614 containing plurality of straight archwires 615 ready to be grasped by the gripping tool at the end of the moveable arm 606. In an embodiment in which the first gripping tool is mounted to the end of a moveable arm, the first gripping tool could retrieve the next workpiece while the moveable arm places the finished workpiece at an exit location.

After an archwire is bent in accordance with an input file supplied to the computer 600, the moveable gripping tool at the end of the robot arm 606 places the wire (or workpiece being bent) at an exit location indicated at 616. A conveyor system 618 including a plurality of trays 620 is provided for carrying the finished archwires wires 622 from the exit location 616 to a labelling and packaging station 624. The labelling and packaging station 624 includes a printer 626 that prints a label for the archwire and a magazine 628 containing a supply of packages such as boxes or bags for the wires. A worker at the station 624 takes a label from the printer and applies it to the archwire 622 or to the package for the wire. The conveyor system 618 is also based on a commercially available, off-the-shelf conveyor system, such as of the type available from the Montech division of Montrac.

The wire manufacturing system 34 includes a heating controller 612 responsive to commands and settings from the wire manufacturing computer 600. The controller 612 controls the supply of current to heating elements 607A and 607B in the gripping fingers in the gripping tools in the robot, to thereby heat the gripping fingers above ambient temperature. Temperature sensors 605A and 605B detect the temperature of the gripper fingers and are used for feedback control of the heating of the gripper fingers. A direct or indirect system for measuring the temperature of the workpiece may also be provided, such as infrared heat detector. The heating controller 612 also controls a wire heating power supply 611 that supplies a current to the gripping tools when they are bending a wire. The power supply 611 is used when the robot is bending shape memory materials or Titanium Molybdenum Alloys (TMA) materials, or possibly other materials. The current produces a resistive heating in the wire. The current is controlled via a wire heating current sensor 613 so as to produce a wire temperature at which a bend formed in the wire is set into the material. The heating of the gripping fingers avoids excessive heat loss when resistive heating of the wire is performed.

Figure 89 is a perspective view of a moveable robot arm 606 used in the manufacturing system in Figure 88. In Figure 89, the gripping tool at the distal end 634 of the arm is omitted. In a preferred embodiment, the moveable arm is based on an off-the-shelf six-axis robot arm and fixed tool. A suitable arm, fixed tool, and robot controller for the robot 604 is available from Stäubli Unimation of Germany. The Stäubli robot arm and fixed tool is customized with gripping fingers, heating controller and ancillary systems, software, current heating subsystem, force sensors, and other features as described herein for bending archwires or other suitable medical devices.

The arm 606 consists of a proximal end or base 630 which mounts to the table 610 of Figure 88 and a free distal end 632 consisting of a tool flange 634, where the second gripping tool 651 A of Figure 92 is mounted, as described below. The arm 606 is capable of motion along six different rotational axes, with the directions indicated by the arrows numbered 1-6 in Figure 89. Thus, the base is fixed with respect to the table 610 and the head portion 636 rotates relative to the base 630. A joint in head portion 636 rotates arm segment 638 about an axis indicated by arrow 2. Similarly, the arm segment 640 is rotated about an axis indicated by arrow 3 by a joint 639. A joint 642 rotates an arm segment 640 about the axis indicated by the arrow 4. A joint 644 attached to the end of arm segment 640 rotates the tool flange 634 about the axis indicated by arrow 5. A sixth joint (not shown) rotates the tool flange 634 about an axis indicated by arrow 6.

Figure 90 is perspective view of the robot arm of Figure 89, showing the movement of the arm joint 639 and the corresponding motion of the arm segment 640 and tool flange 634. The motion commands for the robot are supplied from the robot controller 602 along a cable 650 which plugs into the base 630 of the robot.

Figure 91 is a detailed perspective view of a preferred gripping tool 651 A that is mounted to the tool flange 634 at the distal end of the robot arm 606 of Figure 89 and Figure 90. The construction shown in Figure 91 also applies to the fixed gripper 608 of Figure 88 and Figure 92. The gripping tool 651A consists of a pair of opposed gripping fingers 652 and 654 which open and close to grip and release the workpiece, in this case an orthodontic archwire 615. A pneumatic cylinder actuates gripping finger 652 by moving it about an axis relative to finger 654, to thereby permit the fingers 652 and 654 to grip and release a workpiece such as a wire. A positioning sensor 655 (Fig. 88A) detects the position of the fingers.

In a representative embodiment, the archwires are made from a shape memory alloy such as Nitinol, a material based on Nickel and Titanium plus Copper or other alloy. These materials can be heated to retain the shape of a bend formed in the wire. Accordingly, the wire heating power supply 611 of Figure 88A supplies a current to the gripping fingers of the fixed gripping tool and the moveable gripping tool. The flow of current along the wire creates a resistive heating of the wire sufficient for the material to take a set according to the shape of the wire as it is bent. To avoid dissipation of heat from the wire into the gripping fingers, the gripping fingers 652 and 654 are preferably heated by electrical heating elements 607A and 607B. Conductors 660 and 662 supply current to the heating elements in the gripper fingers.

The gripping tool 651 A of Figure 91 further includes a force sensor 664 in the form of a strain gauge. The force sensor is designed to detect forces that the wire imparts to the gripping fingers after a bend has been placed in the wire or during the bending movement. The forces detected by the force sensor 664 are determined both in magnitude and in direction in three dimensions. The use of the output from the force sensors to overbend wire is explained in further detail below.

Figure 92 is a perspective view of the fixed gripper 608 having a gripper tool 651 B as shown in Figure 91 along with a moveable gripping tool 651 A located at the distal end 632 of the moveable arm 606. An orthodontic archwire 615 is shown gripped by the gripping tools 651A and 651 B. The fixed gripper 608 and gripping tool 651 B remain stationary relative to the base 610. The archwire is bent or twisted by the fixed gripping tool 651 grasping the archwire 615 and the moveable gripping tool 651 A also grasping the archwire 615, and then moveable gripping tooling 651 A bending the wire by moving to a new location in three-dimensional space relative to the fixed gripping tools. The location in space for the moveable arm to move to is determined by the input file fed to the robot computer 600. Basically, the input file consists of a series of point locations in a three dimensional coordinate system which correspond to bracket locations and orientation in a three-dimensional coordinate system for the arch, as described in more detail below. The manner of calculation of these points and generating movement commands (i.e., arm position and switch states for the gripper fingers) for the robot's moveable arm and commands for the fixed gripper to bend the wire will be described in further detail below.

Other possibilities exist for input files and calculation of the bending points. For example, in extraction cases, the wire is needed to close a gap between teeth and the wire serves as a guide or rail for the bracket to slide along to close the teeth. In this situation, a smooth curve is needed between the teeth to allow the brackets to slide the required amount. In this situation, the space between the teeth is divided into small sections, and wire coordinates are obtained for each section. A series of small bends are formed at each section to generate the required smooth curve. It may be helpful in this situation to round the edges of the gripping fingers to help provide the desired smooth shape. As another alternative, free-form curves can be formed by bending the wire between two points which would encompass a plurality of brackets.

While the preferred embodiment of a robot arm is shown in Figure 89, that is not the only possible arrangement of a robot arm. The robot of Figure 89 is optimized for complex bends and twists in archwires. However, some medical devices or archwires may need only simple bends, in which case a lesser number of joints may be required. For example, a one, two or three axis robot may be sufficient for some applications.

Figure 95 is a perspective view of an alternative arrangement of a six-axis moveable robot arm. In this embodiment, the robot arm comprises a base 700, a motor that moves arm segment 702 along direction 704, a second section that contains a motor that moves second arm segment 706 along direction 708, and a third section 710 that contains a motor moving the arm section 712 along direction 714. A motor is provided for rotating arm section 712 about axis $\sigma$. A section 716 is connected to the end of section 712 and includes a motor for rotation of section 716 about an axis indicated by $\beta$. A sixth section 718 is rotated about an axis indicated by $\gamma$. The gripping tool 651 A is mounted to the end of the section 718. Robots of this type are also known and suitable for forming the basis of an orthodontic archwire bending robot. The term "moveable arm" as used in the claims is intended to interpreted broadly to encompass the arm segments of the type shown in Figure 95 as well as the construction shown in Figure 90.

The gripping fingers of the gripping tools 651 A and 652 preferably optimized, in terms of their physical configuration, for the type and shape of the workpiece being bent. This shape may change depending on the nature of the workpiece, e.g., wire, fixation plate, spectacle frames, etc. In the case of wires, wires come in various cross-sections and sizes. It may be desirable to form a plurality of contours in the gripping fingers so as to enable the gripping fingers to grip several different types and sizes of wires without changing gripping tools. For example, one part of the gripper fingers has a series of rectangular contours to grip wires of rectangular cross-section of varying sizes, and perhaps one or more circular contours to grip round wires.

The force sensors on the gripping tools may also be used to provide feedback for an adjustable gripping force to be applied to the workpiece (e.g., wires). It may be desirable to allow the wire to slide through the gripper fingers if the forces acting from the workpiece to the gripper exceed a certain limit. When these forces are sensed, the fixed gripper loosens its grip on the workpiece and allows it to slide.

Figure 93 is a perspective view of a magazine 614 of Figure 88 that holds a plurality of straight archwires needing to be bent in an presently preferred embodiment. Figure 94 is a detailed perspective view of the moveable gripping tool grasping one of the archwires from the magazine of Figure 93.

The magazine 614 consists of a tray 670 having a set of parallel raised elements 672 that define a series of grooves 674 in the upper surface thereof. The archwires 615 are placed in the grooves 674. The archwires are maintained spaced apart from each other in the tray. This permits the robot's moveable gripping tool 651A to pick up a single archwire at a time from the magazine 614 as shown in Figure 94 and insert it into the fixed gripping tool to commence a bending operation. Also, the magazine 614 is positioned at a known location and the dimensions of the tray and slot features thereof are known precisely. This location, information is supplied to the robot control software and allows the gripping tool 651A to remove the archwires one at a time from the magazine automatically and without human assistance. When the magazine 614 is empty a full one is placed at the same location.

Figures 96A and 96B are perspective views of alternative magazine constructions to the magazine of Figure 7. In Figure 96A, the magazine 614 consists of a cylindrical holder with a plurality of apertures 680 spaced from each other, each containing a straight archwire 615. In Figure 96B, the archwires are in a rectangular holder with the apertures arranged in rows and columns. In either case, the moveable arm grips an individual one of the archwires and removes it from the magazine by virtue of the spacing of the wires from each other in the magazine and because the location of each wire in the magazine can be known.

Figure 97 is a schematic illustration of a conveyor system 722 including a conveyor belt 724 that carries archwires 615 to the robot. Here, the robot is enclosed within a safety cage 726. A source feeds archwires 615 into slots 728 in the conveyor belt. When a wire has been bent and the robot is ready for a new wire, the belt advances one position and the robot grips the next wire placed on the belt 724. As another alternative, a spool of archwire can be fed to the robot and a cutting tool (not shown) provided for cutting the wire from the spool into a desired length. The cutting tool could be incorporated into the end of a second robot arm, for example. Still further implementations are possible.

It also possible for the archwire manufacturing system to have other workstations or workplaces in which one or more of the following tasks may be performed: loop bending, surface refining, and marking of the wires. These stations could be positioned at locations around the conveyor system 722 or be in separate locations.

It is also possible to enclosed the robotic wire bending system within an enclosure and fill the enclosure with an inert gas such as nitrogen. The inert gas prevents oxidation of the workpiece during bending or oxidation or other chemical reaction affecting the gripping tools.

B. Archwire Manufacture

The production flow for manufacturing archwires (or other similar appliances) with a representative embodiment of the wire manufacturing system of Figure 88 is shown in Figure 98. The production flow includes the step 800 of loading a wire magazine 614, such as spool of wire in an alternative embodiment, feeding the wire to the robot at step 802 and cutting the wire to length at step 804. At step 806, a series of bends are placed in the archwire in accordance with the prescription for the archwire. After the bending is complete, the wires are labelled at the station 624 at step 808 and packaged in a box or other package at step 810.

The bending of the wire at step 806 is based on slot data for bracket slots at described below in conjunction with Figures 99-106, or based on some other suitable criteria as explained herein. The wire bending computer 600 receives this slot data from the precision appliance centre computer of Figure 1. The computer 600 executes a bending program that processes the slot data into a set of points in three dimensional space and calculates movements of the moveable arm necessary to achieve the appropriate bends in the wire. The computer 600 has a software interface 812 to the robot controller, which translates position or movement signals for the robot arm into low level instructions for the robot controller 602. The robot controller executes a robot control program (adapted from the control program that comes with the robot) which causes the robot arm 606 to move relative to the fixed gripper 608 to bend and/or twist the wire. Where the archwire is a shape memory alloy, the wire heating power supply 611 supplies current to the gripper fingers 652 and 652 on the moveable arm and the gripper fingers on the fixed gripper 608 to heat the wire while the wire is held in the bent condition, and/or during bending motion, to set the shape of the wire.

Robot Input File

The input file, which dictates the shape of an archwire after bending, will now be discussed in conjunction with Figures 99-106. The input file includes a set of matrices, one matrix for each bracket in the arch of the patient. Each matrix consists of a combination of a vector of location of a point on the bracket and a matrix of orientation, indicating the orientation of the bracket in three-dimensional space. Both the vector of location and the matrix of orientation are based on the position of the brackets on the teeth when the teeth are in a target situation. The target situation is developed by the orthodontist from the scan of the dentition and the execution of a treatment planning using the treatment planning software at the clinic.

Figure 99 illustrates the target situation for one arch 820 a patient. The target situation is a three dimensional virtual model of the teeth 822 in which virtual brackets 824 are placed, for example, on the labial surface of the teeth. A coordinate system is defined for the arch 820 having an origin 826. The coordinate system is in three dimensions, with the X and Y dimensions lying in the plane of the arch and the Z direction pointing out of the page. The location of the origin 826 is not particularly important. In the illustrated embodiment, an average "mass" is assigned to each virtual tooth in the arch, and a centre of "mass" is calculated for the arch 820 and the original 826 is located at that centre.

As shown in Figures 100 and 101, a vector of location 828 is defined for each bracket. The vector 828 extends from the origin 826 to the centre of the slot 830 in the bracket along the wall 832 of the bracket slot, i.e., to point 834. The vector of location consists of the X, Y and Z coordinates of the point 834 in the defined arch coordinate system.

The orientation matrix consists of a 3x3 matrix of unit vectors of the form:

1)

$$\begin{matrix} X_1 & Y_1 & Z_1 \\ X_2 & Y_2 & Z_2 \\ X_3 & Y_3 & Z_3 \end{matrix}$$

where $X_1$ $X_2$ and $X_3$ are the X Y and Z components of the X unit vector shown in Figure 101, $Y_1$ $Y_2$ and $Y_3$ are the X, Y and Z components of the Y unit vector shown in Figure 101, and $Z_1$ $Z_2$ and $Z_3$ are the X, Y and Z components of the Z unit vector shown in Figure 101. As noted above, the matrix for each bracket thus consists of the combination of the 3x3 orientation matrix and the position matrix, and is thus as follows :

2)

$$\begin{matrix} X_1 & Y_1 & Z_1 & X \\ X_2 & Y_2 & Z_2 & Y \\ X_3 & Y_3 & Z_3 & Z \\ 0 & 0 & 0 & 1 \end{matrix}$$

where X, Y and Z in the right hand column of entries is the position vector.

The robot input file also includes an antitangential value and a tangential value for each bracket. The antitangential value consists of the distance from the centre of the bracket slot (point 834) to a point defining the terminus of the previous bend in the wire. The tangential value consists of the distance from the centre of the bracket slot to the point defining the terminus of the next bend in the wire. The input file also consists of the thickness of the wire, as measured in the direction of the Y unit vector in Figure 101.

With reference to Figure 102, an example of the 4x4 matrix 2) for a rearmost molar of a patient will be described. Figure shows the origin 826, the position vector 828, and the X and Y unit vectors which indicate the orientation of the bracket slot. Figure 102 also shows the scale (in units of millimetres) which gives absolute location and orientation information for the bracket slot. Here, we assume in the example that there is no Z component to the tangential vector X or the normal vector Y. Figure 103 shows the tangential distance $T_D$ and the antitangential distance $AT_D$ as measured along the centreline of the archwire. The resulting matrix is shown in Figure 104.

From a set of the matrices as shown in Figure 104 comprising all the brackets in the arch, the robot bending program extracts a series of line segments in three dimensional space, which are defined by the terminus of the antitangential and tangential distances for each bracket slot. The set of line segments 840 is shown in Figure 105. The line segments are defined by a set of points P1, P2, P3... Pn having known three dimensional coordinates due to the known location of the bracket slots and the known tangential and antitangential distances. The line segments can also be defined as a set of vectors having a location for the head of the vector and a magnitude and orientation in three directions. The following discussion will use the set of points P1, P2, P3... PN. In Figure 105, the slashes 842 indicate the end points of the bracket slot 830 of Figure 101.

The bends need to be placed in the wire before point P1, between points P2 and P3, between points P4 and P5, etc., that is, between the bracket slots. The slot-to-slot bends of the complete archwire are bent section by section. To form one slot-to-slot bend, the wire is fed so that the fixed gripper tool 651 B and the robot arm gripper tool 651 A can grip the wire in its initial shape. The wire length between fixed gripper and robot arm gripper is equal to the curved length of the wire along the bend. The straight wire sections 840 between the bends have to fit to the bracket slots. To bend the wire into the demanded shape, the main control computer 600 sends signals to the robot controller 602. The robot controller 602 generates signals to move the robot arm 606 with the robot gripper tool 651 A into a new position. The movement path is defined by a bending trajectory. The bend is indicated at 844 in Figure 106.

To form one slot-to-slot bend (e. g., bend 844 between P2 and P3), there might be several of these bending movements necessary. One slot-to-slot bend is considered finished if two consecutive straight wire sections (e.g., between P1 and P2 and between P3 and P4), have the desired relative positions between one another.

To achieve this position, there are different approaches dependent on the wire material properties possible: a) bending material with elastic/plastic properties, such as stainless steel, b) bending material with shape memory properties, and c) bending TMA alloys.

Material with elastic/plastic properties must be overbent to compensate for the elastic part of the deformation. The overbend process, which is described in further detail below, can be defined as a closed loop control. Within the first bending step, the robot arm 606 moves to a new position. Preferably the new position is equal to the planned position or to the planned position plus an amount of overbending. At the end of the move the forces and moments acting on the grippers are measured. They indicate the remaining elastic deformation in the wire. To determine the gripper position which correspond to the released wire shape, the robot arm 606 starts a new move in direction opposite to the acting forces and moments. The forces correspond to a translational move, the moments to a rotational move. By adjusting continuously the movement direction to the measured forces and moments, the robot achieves a position, where the forces and moments are in the order of the measurement resolution (zero-force-position). By choosing an appropriate measurement resolution, the remaining elastic deformation can be neglected and the relative position of the two grippers corresponds to the relative position of the straight wire sections in the released situation. This zero-force-position is compared to the planned position. If the differences are bigger than the tolerance limits, an additional bending step follows to decrease the difference. From the zero-force-position the robot moves now in direction to the planned position and overrides the planned position about the value of the difference between zero-force and planned position. The endpoint of this move is called overbend position. From the overbend position starts again the force and moment controlled move to find the new zero-force-position. If the new zero-force-position is within tolerance limits to the planned position, then the bending process for one slot-to-slot bend is completed and the wire is fed to bend the next slot-to-slot section. If the amount of overbend was too much, the new overbend position is calculated as described above. If the amount of overbend was not sufficient, then the new overbend position is calculated as the former overbend position plus the difference between new zero-force-position and planned position. The described process is repeated within a loop up to the situation, that the difference between zero-force-position and planned position is smaller than the tolerance limit.

Materials with shape memory properties and TMA will be bent to the planned position. To transfer this position into the memory of the alloy, the wire section between the two grippers is heated to a certain temperature for a certain time. The heating is possible e.g. by conductive resistance heating, laser, convection, radiation, or applying warm air or liquid to the material. Heating current and time must be appropriately adjusted to the respective alloy, the wire section length and the wire shape. To warm-up the wire, the wire heating can start already during the bending movement to the planned position. To avoid a heat sink effect at the gripper fingers and to ensure that the complete inter-bracket section of the wire obtains the necessary heating, the gripper fingers 652, 654 (Figure 91) or at least the contact areas of gripper and wire are heated too. The grippers may be heated continuously during the production process of the whole archwire. To compensate for an incomplete transition of the bending position to the alloy memory, there can be defined a certain amount of overbending.

In bending TMA materials, the material can be heated to a high temperature where there is no springback, however when the material cools, it retains its springback properties. The procedure for bending such materials is as follows: 1) heat the gripper fingers; 2) bend the wire to the desired configuration; 3) heat the wire up to the temperature where the springback tendency no longer exists; 4) turn off the heat source and allow the wire to cool, and 5) advance the wire to the next position for bending; and then repeat steps 1) - 5).

The bending of the wire from one section to the next requires that the location and alignment of one straight wire section (i), for example P3 to P4, is defined in reference to the previous straight wire section (i-1) in the counting order defined in the bending system. The origin of the bending system is defined at the end of the straight wire section (i-1), which aims towards the following straight section (i). The x-axis is equal to the direction of the straight wire section (i-1) directed to section (i). For wires with rectangular cross-section the y-axis is perpendicular to x and in direction to the wider dimension of the wire. For quadratic or circular cross-section the y-axis must be perpendicular to x and can be chosen according to practical reasons. The x, y, z-axis follow the right hand rule.

The bracket slot data as described above needs to be transformed to bending data for use by the robot controller 602. This is done by calculation, from the position of the bracket centre point 834 (Figure 100) to the position of the straight wire section centre point (located along the middle of the wire, point 840 in Figure 105).

Next, there needs to be a calculation of the bent wire shape and length. In Figure 30, this is shown as the shape of the wire between points P2 and P3. For each bending step, the robot gripper grips the wire in a certain distance from the fixed gripper corresponding to the length of the wire between points P2 and P3. The wire section between the two grippers will be bent. To minimize the bending forces and moments and to ensure that the bending forces and moments don't exceed limits, which may cause damage to the equipment or to the wire, the gripped wire length should be approximately the "natural" length of the wire in its bent shape. If the length is too short, the wire will be torn and there will be high tensional forces, if it's too long, the wire will tend to kink.

The robot computer 600 therefore calculates the approximate shape of the bent wire using an appropriate algorithm. One way is deriving a second or third order curve representing the shape of the wire using numerical techniques. Another would be using a regular spline algorithm. Ideally, there should be no sharp bends in the wire. In the illustrated embodiment, a Bezier spline algorithm is used. The algorithm gives an analytical description of a smooth curve and generates a set of points along the length of the curve. The length of the curve is obtained by summing up the distance (in three dimensions) along each segment of the curve. The separation distance between each point in the segments can be set arbitrarily and in the illustrated embodiment is 0.05 mm. The algorithm is as follows:

Input:

- Centerpoint location and alignment of two neighbored straight wire sections (given as 4x4matrice with local vector $\overline{l}$, tangential vector $\overline{t}$ normal vector $\overline{n}$ and vertical vector v) These matrices correspond to the 4 x 4 matrix described earlier.
- tangential and antitangential distances $s_t$ and $s_{at}$
- Bezier-distance $b_d$ (empirical value)

The Bezier formula, as known by literature, is described by four points as shown in Figure 20A. The points of the spline curve are given by:

$$\vec{P} = (1-\nu)^3 \cdot \vec{P_1} + (1-\nu)^2 \cdot \nu \cdot \vec{P_2} + (1-\nu) \cdot \nu^2 \cdot \vec{P_3} + \nu^3 \cdot \vec{P_4} \qquad \nu \in \{0,\ldots,1\}$$

Here it will be noted that the Bezier points $P_1$ to $P_4$ in Figure 106A are not necessarily the points P1 - P4 of the wire segments shown in Figure 106, but rather are the points used to calculate the Bezier spline length and shape.

To describe the curved wire shape between the straight wire sections from slot (*i* -1) to slot *i*, the Bezier points $\vec{P_1}$, $\vec{P_2}$, $\vec{P_3}$, $\vec{P_4}$ are calculated by:

$$\overrightarrow{P_1} = \overrightarrow{l_{i-1}} + s_{t,i-1} \cdot \overrightarrow{t_{i-1}}$$

$$\overrightarrow{P_2} = \overrightarrow{l_{i-1}} + (s_{t,i-1} + b_d) \cdot \overrightarrow{t_{i-1}}$$

$$\overrightarrow{P_3} = \overrightarrow{l_i} - (s_{t,i} + b_d) \cdot \overrightarrow{t_i}$$

$$\overrightarrow{P_4} = \overrightarrow{l_i} - s_{at,i} \cdot \overrightarrow{t_i}$$

The wire length L and the N intermediate spline points can be calculated by the algorithm shown in Figure 106B.

The empirical value Bezier-distance $b_d$ must be set to make the calculated and actual bent wire shape tally. For orthodontic wires, a good assumption is $b_d$ = 1x... 2x the larger wire cross-section dimension.

The bending trajectory needs to be calculated for each bend. The bending trajectory is a number of positions of the moveable arm's gripper 651A in relation to the fixed gripper 651 B, which connect the start position and the destination position of a bending movement. In general there are translational and rotational movement components from each bending trajectory position to the next.

For each bending trajectory position the calculated length of the curved wire must be equal to the calculated length in the planned position. To avoid kinking condition for the wire the movement can be divided into two parts:

1. Initial movement, to steer the wire into a distinctly deformed shape. The initial movement is defined as a rotational transformation around an axis through the endpoint of the fixed gripper perpendicular to the connecting vector of the start position and the end position. This is indicated in Figure 115A by the rotational motion of the moveable gripping tool 651 B and movements a, b, c, and d.
2. Finish movement to destination position. The finish movement is a gradual approach from the start position (or if there is an initial movement from the end position of the initial movement) to the destination position. The translational and rotational parts of the whole bending movement are split up steadily to the individual bending trajectory positions. Between two bending trajectory positions, the movement path of the robot gripper is defined by a linear translational movement along the straight line connection of the two positions
and by steadily divided rotational movement. The distance between two bending trajectory positions must be small enough to avoid kinking of the wire and inadmissible forces and moments. These movements are indicated at positions d, e, f, and g in Figure 115B.

In bending wire as described herein, the robot system 604 of Figure 88 has a coordinate system in which an origin 860 is defined as shown in Figures 109 and 110. The wire passes through the fixed gripping tool 651 B through a small gap formed when the fingers are in an open position. The origin 860 is defined as the along the centre of the axis of the wire at the planar edge of the fixed gripping tool 651 B. The robot controller software knows the location of the moveable gripping tool relative to the fixed gripping tool in this coordinate system at all times. Furthermore, the wire is gripped by the moveable gripping tool at a precise point on the moveable gripping tool. Therefore, when the wire is held by the fixed gripping tool and the moveable gripping tool, the distance between the two is known exactly. This allows the bending shown in Figure 115A-115C to be executed without stretching or contracting the wire. In particular, the distance as measured along the wire between the fixed and moveable gripping tools at the point of attachment to the wire is constantly maintained a distance equal to the calculated Bezier distance for the wire as bent between the points P2 and P3 of Figure 105 and 106, and of course for subsequent bends.

To advance the wire between bends or to place the wire in condition for the first bend, there are at least two possibilities. One is that the moveable gripper tool grips the wire and pulls it through the fixed gripping tool (with the fixed gripping tool opened to allow the sliding of the wire with respect to the gripping tool). As an alternative, the wire could be on a spool or coil, and the spool rotated by a motor to advance the wire through the fixed gripping tool. In the later embodiment, a cutting tool will need to be provided to cut the wire after the bending is completed. Archwire manufacturers sell wires in bulk already cut to length and the present description is made in the embodiment in which the wire segment is advanced by the moveable gripping tool advancing the wire through the fixed gripping tool.

Having the bent wire between the two grippers in a tensed state, the robot gripper is moved to a new position, where no forces and moments are acting on the gripper. The force sensors 640 on the fixed and moveable gripping tools are used to determine the position. This position is called the zero force position and corresponds to the released state of the wire. Forces, moments and the movements components are calculated in the main robot coordinate system of Figures 109 and 110.

Depending on the nature of the material, some overbending of the wire may be needed. This would be indicated for example if the zero force position is not the same as the calculated position for the robot's moveable arm 606. To better understand the overbending principles, attention is directed to Figures 107 and 108.

Figure 107 illustrates the relationship between force and deflection of wire. The solid line 866 indicates how much force is needed to give a certain deflection of the wire. Where the force is less than F1, when the force is released the wire returns to its initial state and experiences no deflection due to elastic properties of the wire. At force level F1, some permanent deformation, i.e., plastic deformation, of the wire starts to take place. With a force level of F2, the wire is deflected an amount D2, but when the force is release from the wire the wire bends back to a deflection amount D1, with the relaxation curve 868 essentially parallel to the curve 866 up to force level F1, as shown. Thus, some level of force indicated at F3 is required to be applied to the wire such that when the force is removed the required deflection Ds is achieved in the wire. The fact that F3 is greater than F2 and that the deflection D3 is greater than D2 illustrates that some overbending is generally needed to yield the proper shape of the wire after the bending forces are removed.

Figure 108 illustrates the stresses involved with wire material when it is bent. The wire has one portion experiencing elongation stress, as indicated at 856, while compression stresses are experienced at locations 858, with the length of the arrows indicating the relative magnitude of the stress. With small bends, the forces at the centre of the wire are small enough such that only elastic deformation occurs, while at the outside portion of the wire some plastic deformation may occur. The result is that bending wire is a mixture of elastic and plastic deformation, the character of which is determined by the amount of the bend, the wire material, and the cross-sectional shape.

To determine the amount of required overbending, there are several possibilities. One is a purely analytical solution like finite element analysis of wire. Alternatively, a piece of wire can be tested to determine its response to known forces, and the result stored as a calibration table of bends. Basically, the curves in Figure 107 are obtained experimentally. A more preferred approach uses force sensors 640 (Figure 88A) on the fixed and moveable gripping tools to sense the zero force position of the wire and compare the location of the moveable gripper in this position with the intended position. A geometrical or deformation approach is another alternative. In this approach, the wire is bent some amount and then released, the relaxation position noted, the wire bent some more, the wire released, etc. the process continuing until the wire is bent to the desired position.

With a force based system, perhaps augmented by an adaptive, self-learning artificial intelligence type learning program or calibration table based on previous bends of similar wire, the resulting configuration of the wire can usually be achieved more quickly. Basically, for every bend performed in the wire, information is stored as to the movement necessary to result in a specific bend. For example, to achieve a 13 degree bend in the wire of type T and cross-sectional shape W, the wire had to be bent 15.5 degrees, and this information is stored. With enough data, a mathematical relationship can be derived that that represents curves 866 and 868 for the wire of type T (at least in the portion of the curve of interest), and this mathematical relationship can be used, in conjunction with force sensors, to quickly and accurately place the required bends in the wire.

In either situation, an optical system such as a camera could be used for detecting the position of the wire in the relaxed position is used to determine the actual shape of the wire after any given bend.

Figure 111 is a flow chart of a deformation controlled overbending process that can be used with stainless steel or other wires, including shape memory wires where some overbending is still required.

At step 870, a calculation is made of overbending values in both a translation and rotational aspect. This calculation could be performed for example using finite elements methods, using a calibration table, using a derived mathematical relationship between force and bending, using stored values for overbending from previous bends, or some combination of the above.

At step 872, the bending curve is calculated up to the planned position and including the overbending values. This involves the Bezier spline algorithm set forth previously.

At step 874, the moveable gripping tool is moved to the position indicated by the sum of the planned position plus the overbending position. This forms a bend in the wire. Again, this position is determined in reference to the robot coordinate system and in reference to the spatial relationship between the points where a bend needs to be placed in the wire (P3 and P2 in Figure 105, for example).

At step 876, the force sensors are used to measure the residual forces imparted by the wire onto the gripping tools, and if the forces are greater than some threshold, the moveable gripping tool 651 A is moved to the position where the force measurement is zero or substantially zero.

At step 878 the actual position of the moveable gripping tool is measured using the position sensors in the moveable robot arm.

At step 880, a check is made to see if the difference between the actual position and the planned position is less than a limit. If not, new overbending values are calculated (step 882), and a new bending curve is calculated to overbend the wire an additional amount, in the desired direction, to bring the wire closer to the desired shape (step 883).

Steps 874-883 are repeated until the difference between the actual position of the moveable gripping tool and the planned position is less than a limit.

At step 884, the error in the actual position relative to the planned position is noted and compensated for by correcting the next slot position. In particular, the next slot position represented by the next pair of points in the set of points in Figure 105 is translated in three dimensions by the amount of the error. This correction of the next slot position is needed so as to avoid propagation of an error in any given bend to all subsequent bends.

At step 886, the overbending results from steps 874-882 are saved in memory and used in an adaptive technique for estimating future overbending requirements as explained above.

Figure 112 is a schematic representation of the overbending performed by the method of Figure 111. The line 900 represents the slot between the fingers of the fixed gripper and the line 902 represents the wire in various stages of bending. The dashed line 904 indicates the movement of step 874, with line 906 representing the slot of the moveable gripping tool between the gripping fingers where the wire is gripped in the planned position. The zero force position where the moveable gripper is moved to is indicated at 908 (step 876 in Figure 111). There is both a rotational (dw) and translational (dx) aspect to the difference between the zero force position and the planned position. The last overbend position is shown as position 910 (the overbend calculated at step 870). The required correction is shown by the position 912. This new overbend position, calculated at step 880 in Figure 111 is equal to the last overbend position 910 plus the planned position 906 minus the zero position 908. This new overbend position includes both translational and rotational aspects as indicated by -dx and -dw.

One possible example of actual robot gripper movements to feed the wire through the grippers and execute a representative bend will be explained conjunction with Figure 113A-113E. As shown in Figure 113A, the wire is threaded through the fixed gripper tool 651 B or placed there by the moveable gripping tool such that some length of wire is extending beyond the end of the fixed gripping tool 651 B. The points P1 and P2 along the wire segment are indicated. The moveable gripper tool 651 A is positioned above and slightly to the right of the fixed gripping tool, here 0.2 mm away. The moveable gripping fingers open and the moveable gripper moves down to clamp the wire. The 0.2 mm distance is merely chosen so that the fingers do not collide and can vary from the illustrated embodiment. The fingers cooperate with each other by translation movements of the moveable gripping tool and releasing and closing the fixed and moveable grippers such that the wire is advanced through the fixed gripping tool. This is also indicated by Figure 113B, showing the fixed gripping tool 651 B open (OP) to allow the wire to be slid through a slot formed between the fingers of the tool 651 B. The moveable gripping tool moves to the right to draw the wire through the fixed gripping tool until the point P2 is to the right of the fixed gripping tool (as shown in Figure 113C), where it can be grasped by the moveable gripping tool. As shown in Figure 113C and 113D, the moveable gripping tool opens and releases its grip on the wire (indicated by OP) and moves to the position where it closes (CL) and grasps the wire at location P2. Then moveable gripping tool 651 A draws the wire through the fixed gripping tool while gripping the wire at point P2, such that point P3 is located at the origin of the robot coordinate system, as shown in Figure 113D. Since the planned location of both P2 and P3 after a bend in the wire is made is known in the robot coordinate system, the moveable gripping tool 651A moves to the position shown in figure 113B to place a bend in the wire. At this point, if further overbending is called for, the process of, e.g., Figure 111 and 112 is performed to place the required overbend in the wire. The movements of Figure 113B-113D could be combined to one movement if the distance is small enough, and depending on the thickness of the wire.

Figure 118 illustrates before and after positions of the wire when the bending of Figure 113E occurs. The figure illustrates that the movement of Figure 113E is not a straight line movement which might cause excessive elongation or kinking of the wire. Rather, the movement of the gripper is illustrated as steps a, b, c, d, e, f such that the distance, as measured along the length of the wire, is maintained constant. The movement may performed in two stages (such as shown in Figures 115A and 115B). The result is that two bends 912 are placed in the wire, as shown in Figures 115C. Of course, a twist could also be performed in the wire if required by the prescription.

After the bend has been placed in the wire, the steps shown in Figure 116A-116D are performed to advance the wire along to the position of the next bend. First, as indicated at Figure 116A, the moveable gripping tool 651 A is translated to the right an amount indicated by $\Delta X$. This moves point P3 to the right of the fixed gripping tool by an amount $\Delta X$, as shown in Figure 116B. Then, the moveable gripping tool releases the wire and regrips the wire to the right of the fixed gripping tool as shown in Figure 116C and again translates to the right an amount sufficient to move point P4 to the right of the fixed gripping tool. The moveable gripping tool releases the wire again and grips the wire at point P4. The wire is again translated to the right such that the situation shown in Figure 116D is obtained. The wire is now in position for a bend in the wire between P4 and P5. The process of Figure 111 and 112 occurs again to calculate new bending and overbending positions and the bend is formed in the wire. The process of Figure 116A-116D continues until all the bends have been formed in the archwire. When the final bend is complete, the wire is released from the moveable gripper at the exit location of the wire manufacturing system, and carried by conveyor to the labelling and packaging station described earlier.

Shape memory alloy materials require heating to take on the shape given by the bend produced in Figure 113E. Thus, for these wires, while the wire is held in the position shown by Figure 113E, heat is applied to the wire to raise the temperature of wire to the value needed to take the set. The temperature varies with the type of material. In the illustrated embodiment, a resistance heating system is used as described previously. The current is adjusted until the proper temperature is reached. The heat treating is deemed complete when the force sensors read zero (or less than some limit) when the wire is held by the grippers in the planned position. The amount of current and time applied to the wire is again stored information that can be used for future heating of the same type of wire.

For some softer shape memory materials, e. g., NiTi, the force sensor 640 (Figure 88A) provided in the gripping tools must be very sensitive to detect the small forces involved. While shape memory materials may not require force sensors at all, they can be used to give information as to the effectiveness of the heating process.

In a preferred embodiment, two force sensors are used. A coarser force sensor, used for measuring larger forces during bending, is fitted to the moveable gripping tool. A finer force sensor, with a higher resolution, low noise and higher sensitivity, e.g., with a sensitivity of less than 0.0005N, is fitted to the fixed gripping tool, in order to detect the zero force position. The force sensors are both based on strain gauge technology and can be readily adapted from commercially available strain gauges or off the shelf units. For example, the finer force sensor may have different amplifiers or other modifications to the circuitry to have greater sensitivity, signal to noise ratio and force resolution. Other types of force sensors, such as those based on piezo technology, would be suitable. Suitable off-the-shelf strain gauge force sensors are available from JR3 Inc. of Woodland California, model nos. 45E15A-U760 (fixed gripping tool) and 67M25A-140 (moveable gripping tool).

Other types of heating systems could be adopted for archwires and other types of workpieces to be bent, such as laser, flame, infrared, conductive or radiant heating. Some springback may still be observed in shape memory materials even when heating is performed unless the wire is heated close to the maximum permitted temperature of the wire. Therefore, with some shape memory materials it may be desirable to perform some overbending in order to lower the temperature needed to set the new shape into the wire. Again, the required amount of overbending at a given wire temperature can be stored in memory and used to derive a relationship between temperature, overbending and resulting position for the material, which can be used for subsequent bends in the wire.

Due to the complexities of wire deformation and twisting in wire that can occur when wire of a rectangular cross section is bent, and the difficulty in controlling the resulting shape of the wire (particularly when complex bends and twists are formed in the wire), the usage of force measuring devices, and position sensors to detect the shape of the wire when the wire is in a zero force condition, gives accurate information as to the shape of the wire after a bend. Thus, a force based approach to overbending is a preferred embodiment. The actual position of the wire in the zero force condition can be obtained by position sensors on the robot arm (which makes no contribution to the measurement of forces), or better yet, by releasing the wire from the moveable arm and detecting the position of the wire with a camera or other optical system. Basically, the camera would image the wire immediately in front of the fixed gripping tool. Pattern recognition and image processing algorithms are used to determine the edge of the wire, and thereby calculate its shape. More than one camera could be used if necessary to image the wire sufficiently to calculate twist in the wire. The effects of gravity would have to be compensated for in any position measuring system in which the wire is not held by the moveable gripping tool.

Thus, in one possible embodiment the robot further comprises an optical sensor system such as a CCD camera detecting the shape of the orthodontic appliance after the bend in said orthodontic appliance has been made, such as by releasing the appliance from the moveable gripping tool and allowing the appliance to take its natural position, and then using the optical system to detect the shape of the appliance.

It is also possible to use both the force measuring systems and the optical system as a final check on the shape. The force sensor system (e.g., coupled to the fixed and/or moveable gripping tools) detects forces generated by the orthodontic appliance after the orthodontic appliance has been bent. The moveable arm is operated to move the orthodontic appliance to a zero-force position in which the forces detected by the force system are below a predetermined threshold. The optical sensor system detects the shape of the orthodontic appliance in the zero-force position. The position detected by the optical sensor system can be used as a feedback mechanism for further wire bending if the zero force position is not the intended or desired configuration of the appliance. An optional type of sensor system would be calipers that contact the workpiece and responsively provide position information as to the location (i.e., bend) formed in the workpiece.

For stainless steel wires, there is generally no need for heat treatment of the wire. It is simply bent into the desired position, with overbending performed as required. The shorter the distance between endpoints of a bend, the greater the deformation in the wire, therefore the greater the predictability in the deformation. With orthodontic archwires, the situation can occur where there is a relatively long distance between bracket slots (particularly in the region of the molars) and it can be difficult to obtain a stable bending result. A preferred solution here is to make this distance shorter by adding on some length to the tangential distance of one slot position and the antitangential distance of the next slot position, as shown in Figure 117. Here, point P2 is extended in space to point P2', and point P3 is brought closer to point P2 by moving it to point P3'. The required bending of the wire is now calculated relative to points P3' and P2'. The bend placed between P3' and P2' is now sufficiently short that it can be formed with enough control.

In practice, it known that after an archwire has been fitted to the patient's brackets, the archwire imparts forces to move the teeth to the desired position. However, after a certain amount of time, some small amount of bend remains in the wire but it is insufficient to cause any further tooth movement. Consequently, the teeth are not moved to their desired position. This can be compensated for by adding an additional amount of bend to the wire so that when the wire is installed, it will continue to exert forces until the teeth have been moved all the way to their desired position. As shown in Figure 118, this small additional bend is shown as 920. Figure 33 shows the wire of Figure 118 installed in the brackets of a patient. The bend 920 of Figure 32 is in addition to other bends that may be placed between the brackets 824. Figure 119 illustrates that enough residual force exists by virtue of bend 920 to move the teeth 822 to their desired position.

In certain orthodontic situations, loops may need to be bent in the wires. Figure 120 illustrates a loop 922 formed in a wire 615. The loop may be formed by the robot of Figure 90. Alternatively, only the peripheral corners 924 of the loop 920 are formed by the bending robot, as shown in Figure 121A, with the remainder of the loop formed by placing the wire over a die 926 having a shape 928 matching the shape of the bottom portion 930 of the loop 920. A forming tool 928 is moved against the wire and die 926 to form the bottom portion of the loop as indicated in Figure 121B.

The robot may also include a separate arm or tooling by which stops, or other features are bonded to the wire by suitable techniques such as welding. For example, the robot can be fitted with other tools for forming a Herbst appliance or expansion devices. Alternatively, different types of wires could be joined together by welding.

The robot may also be used to bend clear, transparent wires made from polymeric or plastic materials, such as thermoplastics, duroplastics, polyacrylic plastics, epoxy plastics, thermoplastics, fibre reinforced composites, glass fibre containing materials or other similar materials suitable for an orthodontic archwire. These plastics archwires may require heating during bending, but current sources may not be suitable heating devices. Recommended techniques for heating the plastic wire include blowing hot air over the wires during bending, using heated pliers, placing a heat conductive material onto the wire, using a laser to heat the wire, or spraying a hot vapour or liquid onto the wire.

As noted above, additional possibilities are presented for bending fixation plates, orthotic devices, prosthetic devices, endodontic devices, surgical guidewires, surgical archbars, implants or surgical tools with the robot manufacturing system. The gripper fingers and associated structures may be optimized depending on the workpiece or appliance in question. However, the principles of operation are basically the same.

For example, the robot of the present invention is particularly useful for bending fixation plates, rods, compression plates and the like, for example facial, cranial, spinal, hand, and long bone and other osteosynthesis plates, such as, for example, the titanium appliances provided by Leibinger GmbH of Germany. These fixation plates may consists of, for example, an elongate skeletal frame having a plurality of apertures for receiving screws, arranged in straight lengths, C, Y, J H, T or other shape configurations, or a long cylindrical rod. At the present, these appliances are manually bent by the surgeon to the shape of the bone in the operating room using special manual bending tools. It is possible to automate this process and bend the plates in advance using the principles of the present invention. In particular, the shape of the bone or bone fragments is obtained a CAT scan, from a scan of the exposed bone using a hand-held scanner (such as described above. Once a three-dimensional virtual model of the bone is obtained, e.g., from CAT scan data, the virtual model is manipulated using a computer to fuse the bones together in the desired position. The surgeon then overlays the three-dimensional virtual implant in the desired location on the virtual model, and bends the virtual implant using the user interface of a general purpose computer storing the virtual model of the bone and implant. The required shape of the implant to fit the bone in the desired location is derived.

Alternatively, a physical model of the bone in the desired configuration can be manufactured from the virtual model using stereolithography (SLA), three-dimensional lithography, or other known technology, and the shape of the implant derived from the physical model.

As another alternative, a SLA physical model of the bones (e.g., skull) is made from a CT scan or other source, and the surgeon performs a simulated surgery on the physical model to place the bones in the desired condition. The model is then scanned with an optical scanner and a virtual model of the bones in the desired condition is obtained, as described in the patent application of Rudger Rubbert et al., cited above. The virtual fixation device is then compared or fitted to the virtual model of the bones to arrive at a desired shape of the fixation device.

In either situation, the shape of the implant is then translated to the robot controller as a series of straight sections and bends of known geometry (and specifically position commands for the moveable gripping tool relative to the fixed gripping tool). The moveable and fixed gripping tools of the bending device grip the implant or fixation device at one end and then either bend the appliance or advance the position of the implant to the location of the next bend, and continue along the length of the device to form the device in the desired configuration. Obviously, some modification to the gripping tools may be needed from the disclosed embodiment depending on the physical characteristics of the device being bent, and such modifications are within the ability of persons skilled in the art.

The bending apparatus described above is also adaptable to generic workpieces, such as tubes, cylinders, wires or other types of structures.

The bending apparatus may use resistive heating, force sensors, overbending, and the other features described at length in the context of orthodontic archwires, depending on the application for other workpieces.

Bonding Pads

Customized bracket pads can be manufactured using a variety of techniques. One possibility is to bond a blob of adhesive to a bracket base, and mill the blob using a milling machine to match the tooth surface. Since the tooth surface is known precisely from the scanning, and the position of the bracket base relative to the surface is also known precisely, a very accurate bracket pad can be manufactured in this fashion. Another technique is to stamp out a bracket pad using stamping machine either in the desired shape or as a blank and milling the stamped pad to the desired shape. A third possibility is creating a negative of the customized pad, forming the pad in a mould, bonding the pad to the bracket and the orthodontist trimming the pad as necessary when the pad is bonded to the tooth.

Since the treatment planning software gives precise information as to the location of the bracket relative to the tooth surface in three dimension, the shape of the customized bonding pad can be determined with high precision. This shape is transferred to a suitable milling machine using known techniques and the pad is milled in accordance with the desired shape. The pad is then affixed to the bracket (either at the precision appliance centre or at the orthodontic clinic and the bracket + pad bonded to the tooth.

Other inventive features disclosed herein

Orthodontic Appliance Optimization

Generally, the present system also provides for a method and apparatus for arch wire receptacle (e.g., brackets, bands, headgear tubes, etc.) optimization. Such a method and apparatus includes processing that begins by obtaining a digital model of an orthodontic structure of an orthodontic patient. This may be done in accordance with the teachings described above. The processing then continues by retrieving a digital model of an initial arch wire receptacle (e.g., a bracket, a band, a headgear tube, etc.) that was selected from a plurality of digital models of arch wire receptacles. Generation of a digital model of an arch wire receptacle may be further performed as described above. The processing then continues by digitally placing the digital model of the initial arch wire receptacle on a given tooth of the digital model of the orthodontic structure to provide a digital arch wire receptacle placement. The process then proceeds by retrieving a digital model of an arch wire. For the given tooth, the processing continues by digitally modelling a force system on the tooth based on the digital model of the initial arch wire receptacle, the digital arch wire receptacle placement, and the digital model of the arch wire. Having done this, when the force system is not optimal, the force system is modified to achieve an optimal force system. The force system includes the effects of bonding pad thickness, bonding agent properties, the arch wire, ligation forces, and biological characteristics (e.g., growth, tooth interference, tooth anatomy). Each of these factors may be adjusted individually or in combination to optimize the force system. With such a method and apparatus, optimal force systems given orthodontic parameter constraints may be obtained without the limitations of prior art orthodontic practices.

Determining and Monitoring Orthodontic Treatment

Generally, the present system also provides for a method and apparatus for treating an orthodontic patient. Such a method and apparatus include processing that begins by generating digital information regarding the orthodontic patient by a site orthodontic system. The digital information may include a three-dimensional scan image of the patient's orthodontic structure (e.g., teeth, jaw bone, gums, and other facial features), x-rays, clinical examination and measurements, dental history, medical history, photographs, etc. The site orthodontic system then transmits the digital information to an orthodontic server. The orthodontic server and/or the site orthodontic system creates an electronic patient record from the digital information. The electronic patient record includes, but is not limited to, clinical examination interpretations, radiology examination measurements, automatic and manual cephalometric analysis, a created electronic cephalometric tracings, an electronic composite including an integration of the three-dimensional images, radiographic data (e.g., x-rays, CAT scans, MRI scans), photographs, a generated electronic articulation module, measurements and analysis of electronic models, data quality assurance checks, and/or supplemental data.

The orthodontic server then generates an initial treatment from the electronic patient record, wherein the initial treatment plan includes precise steps to obtain a desired orthodontic structure. In other words, the initial treatment plan includes a series of steps, wherein, for each step, a corresponding orthodontic apparatus will be designed to reshape the patient's orthodontic structure into a desired structure for the precise step. The orthodontic server then transmits a digital version of the initial treatment plan to the site orthodontic system. Upon confirmation from the site orthodontic system, the orthodontic server designs an orthodontic apparatus (e.g., brackets, bands, wires, head gear, rubber band placement, and/or a retainer) for one of the precise steps based on the treatment plan. The orthodontic apparatus is then fabricated/assembled and provided to the site orthodontic system. In addition, the orthodontic server may provide practitioner and/or patient instructions for use, maintenance, and/or installation of the orthodontic apparatus.

Upon receipt of the orthodontic apparatus, the orthodontist verifies receipt of the orthodontic apparatus and subsequently installs it. At a predetermined point in time after the installation in accordance with the treatment plan, the patient's mouth is electronically scanned to obtain updated digital information. The site orthodontic system provides the updated digital information to the orthodontic server, which uses the updated digital information to update the electronic patient record. From the updated electronic patient record, the orthodontic server determines whether the actual movement of the patient's teeth is as predicted. Such a determination may be supplemented with input from a practitioner (e.g., a technician, dental assistant, orthodontist, specialist, consultant, etc.) at the site or at a remote site with respect to the patient. If so, the next step of the initial treatment plan is executed (e.g., the wire for the next step is fabricated, provided to the orthodontist, and installed). If, however, the actual movement is not as predicted, the orthodontic server adjusts the treatment plan to obtain the desired results. After the treatment plan has been adjusted, the next step of the revised treatment plan is executed. This monitoring of a patient's progress and revising the treatment plan, when necessary, continues throughout the treatment. Thus, with such a method and apparatus, a scientific approach is provided to orthodontic treatment throughout the treatment, maintains the treatment costs at reasonable levels, and provides a more consistent and reduced treatment time. In addition, the present method and apparatus allow orthodontic practitioners to have just-in-time inventory, allows for treatment adjustments that would be required due to changes in tooth position and/or jaw development, and further allows for changes in the orthodontic structure if the force system becomes sub-optimal.

Treating an Orthodontic Patient

Generally, the present system also provides for a method and apparatus for treating an orthodontic patient. Such a method and apparatus include processing that begins by generating digital information regarding the orthodontic patient by a site orthodontic system. The digital information may include a three-dimensional scan image of the patient's orthodontic structure (e.g., teeth, jaw bone, gums, and other facial features), x-rays, clinical examination and measurements, dental history, medical history, photographs, etc. The site orthodontic system then transmits the digital information to an orthodontic server. The orthodontic server and/or the site orthodontic system creates an electronic patient record from the digital information. The electronic patient record includes, but is not limited to, clinical examination interpretations, radiology examination measurements, automatic and manual cephalometric analysis, a created electronic cephalometric tracings, an electronic composite including an integration of the three-dimensional images, radiographic data (e.g., x-rays, CAT scans, MRI scans), photographs, a generated electronic articulation module, measurements and analysis of electronic models, data quality assurance checks, and/or supplemental data.

The orthodontic server then generates an initial treatment from the electronic patient record, wherein the initial treatment plan includes precise steps to obtain a desired orthodontic structure. In other words, the initial treatment plan includes a series of steps, wherein, for each step, a corresponding orthodontic apparatus will be designed to reshape the patient's orthodontic structure into a desired structure for the precise step. The orthodontic server then transmits a digital version of the initial treatment plan to the site orthodontic system. Upon confirmation from the site orthodontic system, the orthodontic server designs an orthodontic apparatus (e.g., brackets, bands, wires, head gear, rubber band placement, and/or a retainer) for one of the precise steps based on the treatment plan. The orthodontic apparatus is then fabricated/assembled and provided to the site orthodontic system. In addition, the orthodontic server may provide practitioner and/or patient instructions for use, maintenance, and/or installation of the orthodontic apparatus.

Upon receipt of the orthodontic apparatus, the orthodontist verifies receipt of the orthodontic apparatus and subsequently installs it. At a predetermined point in time after the installation in accordance with the treatment plan, the patient's mouth is electronically scanned to obtain updated digital information. The site orthodontic system provides the updated digital information to the orthodontic server, which uses the updated digital information to update the electronic patient record. From the updated electronic patient record, the orthodontic server determines whether the actual movement of the patient's teeth is as predicted. Such a determination may be supplemented with input from a practitioner (e.g., a technician, dental assistant, orthodontist, specialist, consultant, etc.) at the site or at a remote site with respect to the patient. If so, the next step of the initial treatment, plan is executed (e.g., the wire for the next step is fabricated, provided to the orthodontist, and installed). If, however, the actual movement is not as predicted, the orthodontic server adjusts the treatment plan to obtain the desired results. After the treatment plan has been adjusted, the next step of the revised treatment plan is executed. This monitoring of a patient's progress and revising the treatment plan, when necessary, continues throughout the treatment. Thus, with such a method and apparatus, a scientific approach is provided to orthodontic treatment throughout the treatment, maintains the treatment costs at reasonable levels, and provides a more consistent and reduced treatment time. In addition, the present method and apparatus allow orthodontic practitioners to have just-in-time inventory, allows for treatment adjustments that would be required due to changes in tooth position and/or jaw development, and further allows for changes in the orthodontic structure if the force system becomes sub-optimal.

Producing a Three-Dimensional Digital Model of an Orthodontic Patient

Generally, the present system also provides for a method and apparatus for producing a three-dimensional digital model of an orthodontic patient. Such a method and apparatus include processing that begins by obtaining data of an orthodontic structure of the orthodontic patient. The processing then continues by obtaining at least one scaling reference point of the orthodontic structure. For example, the scaling reference point may be a marking placed on a tooth prior to obtaining the data. The processing continues by scaling the data of the orthodontic structure based on the at least one scaling reference point to produce scaled data. Note that the data may be video data, x-rays, CAT scans, ultrasound, and/or magnetic resonance images. The process then continues by obtaining at least two orientation reference points relating to the orthodontic structure (i.e., fixed physical attributes of the orthodontic structure that will not change, or will change with negligible effects, during the course of treatment). The processing then continues by mapping the scaled data to a coordinate system based on at least two orientation reference points to produce an enhanced three-dimensional digital model of the orthodontic patient. With such a method and apparatus, the automated treatment of an orthodontic patient occurs in a scientific and controlled manner. By obtaining an accurate three-dimensional digital model that includes a dentition structure of the orthodontic patient, patient treatment can be simulated by a computer in three-dimensional space, thereby providing a scientific approach to orthodontic treatment.

Producing a Desired Three-Dimensional Digital Model of an Orthodontic Structure

Generally, the present system also provides for a method and apparatus for generating a three-dimensional digital model of a desired orthodontic structure. Such a method and apparatus include processing that begins by obtaining a three-dimensional model of an actual orthodontic structure, wherein the three-dimensional digital model is defined in x, y, z space. The processing then continues by generating an interim three-dimensional model of the desired orthodontic structure less teeth. The interim three-dimensional model is designed in x, y, z space and includes the desired placement of an occlusal plane, an upper-arch form, a lower-arch form, an upper-arch midline, and a lower-arch midline. The processing then continues by positioning the upper and lower teeth with respect to the interim digital model and the defined x, y, z space to obtain a first pass three dimensional digital model of the desired orthodontic structure. The processing continues by determining whether achieving the first pass three-dimensional model is feasible. In essence, the determination is based on treatment constraints and whether the desired orthodontic structure can be achieved from the actual orthodontic structure given such constraints. When achieving the first pass three-dimensional image is feasible, the processing continues by utilizing the first pass three-dimensional model as the desired three-dimensional digital model. With such a method and apparatus, automation of orthodontic treatment is realized thereby providing a scientific approach to practice of orthodontics.

Designing an Orthodontic Apparatus to Provide Tooth Movement

Generally, the present system also provides for a method and apparatus for designing an orthodontic structure to provide tooth movement. Such a method and apparatus includes processing that begins by obtaining a three-dimensional digital model of a desired orthodontic structure. The orthodontic structure includes a plurality of teeth that have been positioned into a desired location. The processing continues by generating a zero force arch wire based on the three-dimensional digital model of the desired orthodontic structure. The processing continues by placing brackets on at least some of the plurality of teeth in a zero force position with respect to the zero force arch wire. Having placed the brackets on the desired orthodontic structure, the placement is transferred to a three-dimensional digital model of an actual orthodontic structure. Having transferred the bracket placement, a jig may be formed from the digital model of the actual orthodontic structure with the brackets installed. The jig holds the physical embodiments of the brackets such that the brackets may be placed in the patient's mouth in the position determined by the automated process. With such a method and apparatus, the practice of orthodontics is transformed from an art to a science throughout the treatment process, reduces costs, and provides for more consistent treatment times.

Automated Generation of a Patient Treatment Plan

Generally, the present system also provides for a method and apparatus for generating a patient treatment plan. Such a method and apparatus includes processing that begins by providing a list of health care services to a patient and/or care provider. The processing continues by prompting for input of digital information regarding the patient when a health care service has been selected. The processing continues by determining whether a sufficient amount of digital information has been received. If so, the processing continues by simulating treatment of a patient based on the digital information, a treatment objective, and normalized patient data. The processing then continues by generating the patient treatment plan in accordance with the simulating of the treatment when the simulated treatment results have been acknowledged. With such a method and apparatus, the generation of a patient treatment plan may be automated for particular types of health care services, including orthodontic care, dental care, and medical care.

While presently preferred embodiments of the invention have been described for purposes of illustration of the best mode contemplated by the inventors for practicing the invention, wide variation from the details described herein is foreseen without departure from the scope of the invention. This scope is to be determined by reference to the appended claims. The term "bend", as used in the claims, is interpreted to mean either a simple translation movement of the workpiece in one direction or a twist (rotation) of the workpiece, unless the context clearly indicates otherwise.

## Claims

1. A manufactured orthodontic archwire, comprising:

   a wire,
   wherein the wire, in a relaxed state, has a generally arcuate configuration corresponding to an archform for a particular patient to receive the wire;
   wherein the wire further comprises a series of straight slot segments having precision lengths, each of the segments for placement into a slot of a bracket bonded to a tooth of the particular patient and, as needed, to allow for sliding of the bracket along such straight segments, the straight slot segments connected to each other by curved segments, comprising bends and/or twists, each forming a precision geometrical relationship between adjacent straight segments of the wire; and
   wherein at least one pair of adjacent straight slot segments are connected to each other by a curved segment comprising a combination of a bend and a twist forming a precision geometrical relationship between the adjacent straight segments in the wire.

2. The manufactured orthodontic archwire of claim 1, wherein the wire comprises a material having a rectangular cross-section.

3. The manufactured orthodontic archwire of claim 1, wherein the wire comprises a material having a round cross-section.

4. The manufactured orthodontic archwire of any preceding claim, wherein the wire is made from a shape memory alloy.

5. The manufactured orthodontic archwire of any of claims 1 to 3, where the wire is made from stainless steel.

6. The manufactured orthodontic archwire of any of claims 1 to 3, wherein the wire is made from a titanium molybdenum alloy.

7. The manufactured orthodontic archwire of any of claims 1 to 3, wherein the wire is made from a polymeric or plastic material.

8. The manufactured orthodontic archwire of claim 7, wherein the polymeric or plastic material is selected from the group consisting of thermoplastics, duroplastics, polyacrylic plastics, epoxy plastics and fibre reinforced composites.

9. The manufactured orthodontic archwire of any of claims 1 to 3, wherein the wire is made from glass fibre materials.

10. The manufactured orthodontic archwire of any preceding claim, wherein the wire further comprises a loop in the wire.

11. The manufactured orthodontic archwire of any preceding claim, wherein a stop is welded to the wire.

12. The manufactured orthodontic archwire of any preceding claim, further comprising a marking in the wire.

13. The manufactured orthodontic archwire of any preceding claim, wherein a plurality of adjacent straight slot segments are connected to each other by a single curved segment comprising a combination of a bend and a twist forming a precision geometrical relationship between the adjacent straight segments in the wire.

**Patentansprüche**

1. Ein hergestellter kieferorthopädischer Bogendraht, umfassend:

   einen Draht,
   wobei der Draht in einem entspannten Zustand eine im Allgemeinen bogenförmige Anordnung aufweist, die einer Bogenform für einen bestimmten Patienten, der den Draht erhält, entspricht;
   wobei der Draht ferner eine Reihe an geraden Slotsegmenten umfasst, die genaue Längen aufweisen, zum Platzieren jedes der Segmente in einen Slot eines Brackets, das an einem Zahn des bestimmten Patienten befestigt ist und, falls benötigt, das Verschieben des Brackets entlang der geraden Segmente ermöglicht, die geraden Slotsegmente miteinander durch gebogene Segmente verbunden sind, umfassend Biegungen und/oder Verdrehungen, die jeweils ein präzises geometrisches Verhältnis zwischen benachbarten geraden Segmenten des Drahts bilden;
   und
   wobei mindestens ein Paar an benachbarten geraden Slotsegmenten miteinander durch ein gebogenes Segment verbunden sind, das eine Kombination einer Biegung und einer Verdrehung, die ein präzises geometrisches Verhältnis zwischen den benachbarten geraden Segmenten in dem Draht bilden,
   umfasst.

2. Der hergestellte kieferorthopädische Bogendraht gemäß Anspruch 1, wobei der Draht ein Material umfasst, das einen rechteckigen Querschnitt aufweist.

3. Der hergestellte kieferorthopädische Bogendraht gemäß Anspruch 1, wobei der Draht ein Material umfasst, das einen runden Querschnitt aufweist.

4. Der hergestellte kieferorthopädische Bogendraht gemäß einem vorherigen Anspruch, wobei der Draht aus einer Form-Gedächtnis-Legierung hergestellt ist.

5. Der hergestellte kieferorthopädische Bogendraht gemäß einem der Ansprüche 1 bis 3, wobei der Draht aus Edelstahl hergestellt ist.

6. Der hergestellte kieferorthopädische Bogendraht gemäß einem der Ansprüche 1 bis 3, wobei der Draht aus einer Titan-MolybdänLegierung hergestellt ist.

7. Der hergestellte kieferorthopädische Bogendraht gemäß einem der Ansprüche 1 bis 3, wobei der Draht aus einem polymeren Material oder Kunststoffmaterial hergestellt ist.

8. Der hergestellte kieferorthopädische Bogendraht gemäß Anspruch 7, wobei das polymere oder Kunststoffmaterial ausgewählt ist aus der Gruppe bestehend aus Thermoplasten, Duroplasten, Kunststoffen auf Polyacryl-Basis, Kunststoffen auf Epoxid-Basis und faserverstärkten Kompositen.

9. Der hergestellte kieferorthopädische Bogendraht gemäß einem der Ansprüche 1 bis 3, wobei der Draht aus Glasfasermaterialien hergestellt ist.

10. Der hergestellte kieferorthopädische Bogendraht gemäß einem vorherigen Anspruch, wobei der Draht ferner eine Schlaufe in dem Draht umfasst.

11. Der hergestellte kieferorthopädische Bogendraht gemäß einem vorherigen Anspruch, wobei eine Arretierung auf den Draht geschweißt ist.

12. Der hergestellte kieferorthopädische Bogendraht gemäß einem vorherigen Anspruch ferner umfassend eine Markierung in dem Draht.

13. Der hergestellte kieferorthopädische Bogendraht gemäß einem vorherigen Anspruch, wobei mehrere der benachbarten geraden Slotsegmente miteinander durch ein einzelnes gebogenes Segment verbunden sind, das eine Kombination einer Biegung und eine Verdrehung umfasst, die ein präzises geometrisches Verhältnis zwischen den benachbarten geraden Segmenten im Draht bilden.

## Revendications

1. Fil arqué orthodontique fabriqué, comprenant :

   un fil,
   dans lequel le fil, à l'état relaxé, a une configuration généralement arquée correspondant à une forme arquée pour un patient particulier qui recevra le fil ;
   dans lequel le fil comprend en outre une série de segments de fente rectilignes ayant des longueurs précises, chacun des segments étant destiné à être placé dans une fente d'un crochet relié à une dent du patient particulier et, selon les besoins, à permettre le glissement du crochet le long de ces segments de fente rectilignes, les segments de fente rectilignes étant raccordés l'un à l'autre par des segments incurvés, comprenant des coudes et/ou des torsions, chacun formant une relation géométrique de précision entre des segments rectilignes adjacents du fil ; et
   dans lequel au moins deux segments de fente rectilignes adjacents sont raccordés l'un à l'autre par un segment incurvé comprenant une combinaison d'un coude et d'une torsion formant une relation géométrique de précision entre les segments rectilignes adjacents dans le fil.

2. Fil arqué orthodontique fabriqué selon la revendication 1, dans lequel le fil comprend un matériau ayant une section transversale rectangulaire.

3. Fil arqué orthodontique fabriqué selon la revendication 1, dans lequel le fil comprend un matériau ayant une section transversale ronde.

4. Fil arqué orthodontique fabriqué selon l'une quelconque des revendications précédentes, dans lequel le fil est formé d'un alliage à mémoire de forme.

5. Fil arqué orthodontique fabriqué selon l'une quelconque des revendications 1 à 3, dans lequel le fil est formé d'acier inoxydable.

6. Fil arqué orthodontique fabriqué selon l'une quelconque des revendications 1 à 3, dans lequel le fil est formé d'un alliage de titane et de molybdène.

7. Fil arqué orthodontique fabriqué selon l'une quelconque des revendications 1 à 3, dans lequel le fil est formé d'un matériau polymère ou plastique.

8. Fil arqué orthodontique fabriqué selon la revendication 7, dans lequel le matériau polymère ou plastique est choisi dans le groupe constitué des thermoplastiques, des thermodurcissables, des plastiques polyacryliques, des plastiques époxy et des composites renforcés par des fibres.

9. Fil arqué orthodontique fabriqué selon l'une quelconque des revendications 1 à 3, dans lequel le fil est formé de matériaux en fibres de verre.

10. Fil arqué orthodontique fabriqué selon l'une quelconque des revendications précédentes, dans lequel le fil comprend en outre une boucle ménagée dans le fil.

11. Fil arqué orthodontique fabriqué selon l'une quelconque des revendications précédentes, dans lequel un arrêt est soudé sur le fil.

12. Fil arqué orthodontique fabriqué selon l'une quelconque des revendications précédentes, comprenant en outre un marquage dans le fil.

13. Fil arqué orthodontique fabriqué selon l'une quelconque des revendications précédentes, dans lequel une pluralité de segments de fente rectilignes adjacents sont raccordés l'un à l'autre par un seul segment incurvé comprenant une combinaison d'un coude et d'une torsion formant une relation géométrique de précision entre les segments rectilignes adjacents dans le fil.

FIG. 1

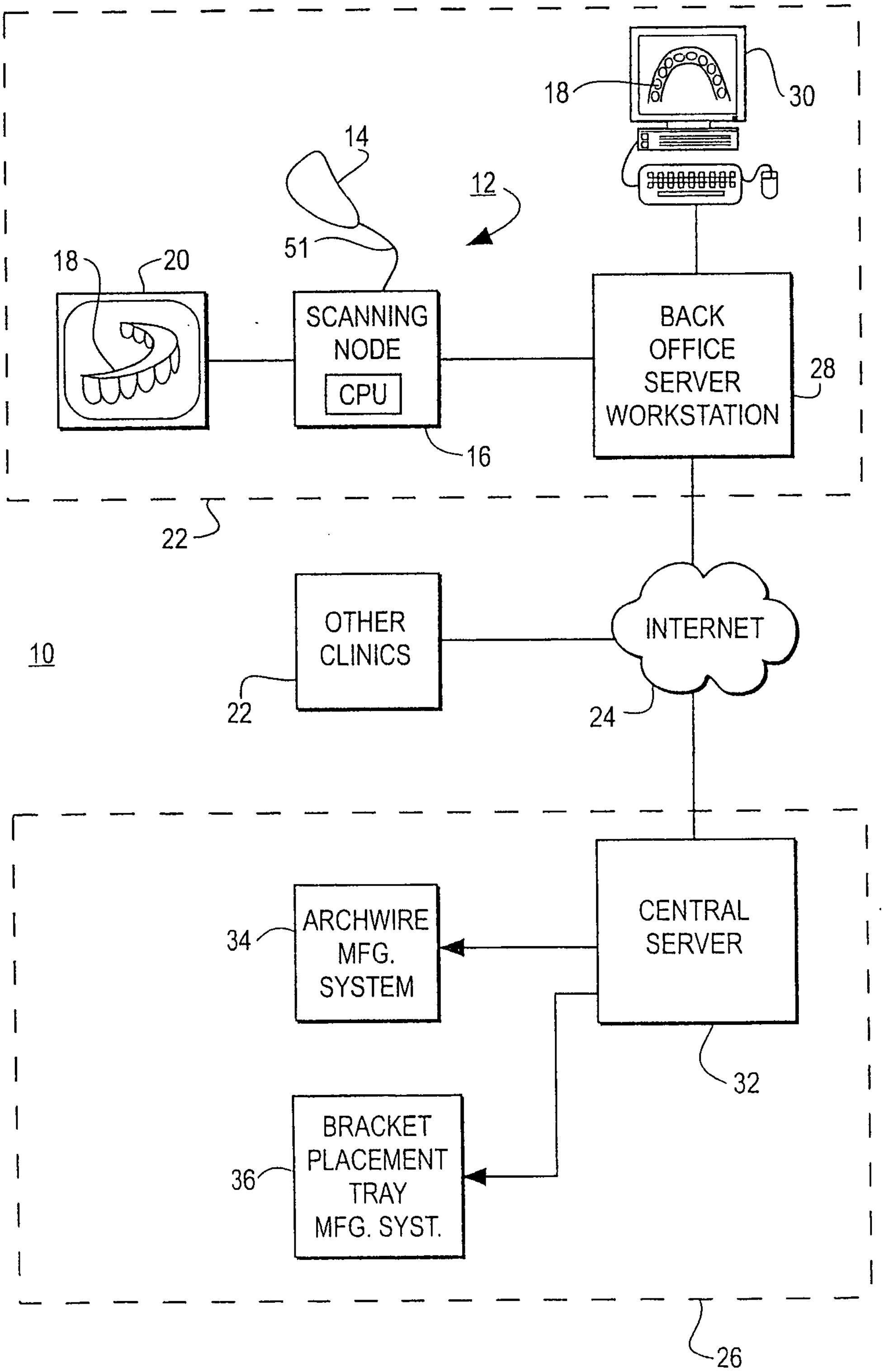
14
12
18
30
51
18
20
SCANNING NODE
CPU
BACK OFFICE SERVER WORKSTATION
28
16
22
OTHER CLINICS
INTERNET
10
22
24
CENTRAL SERVER
ARCHWIRE MFG. SYSTEM
34
32
BRACKET PLACEMENT TRAY MFG. SYST.
36
26

FIG. 2

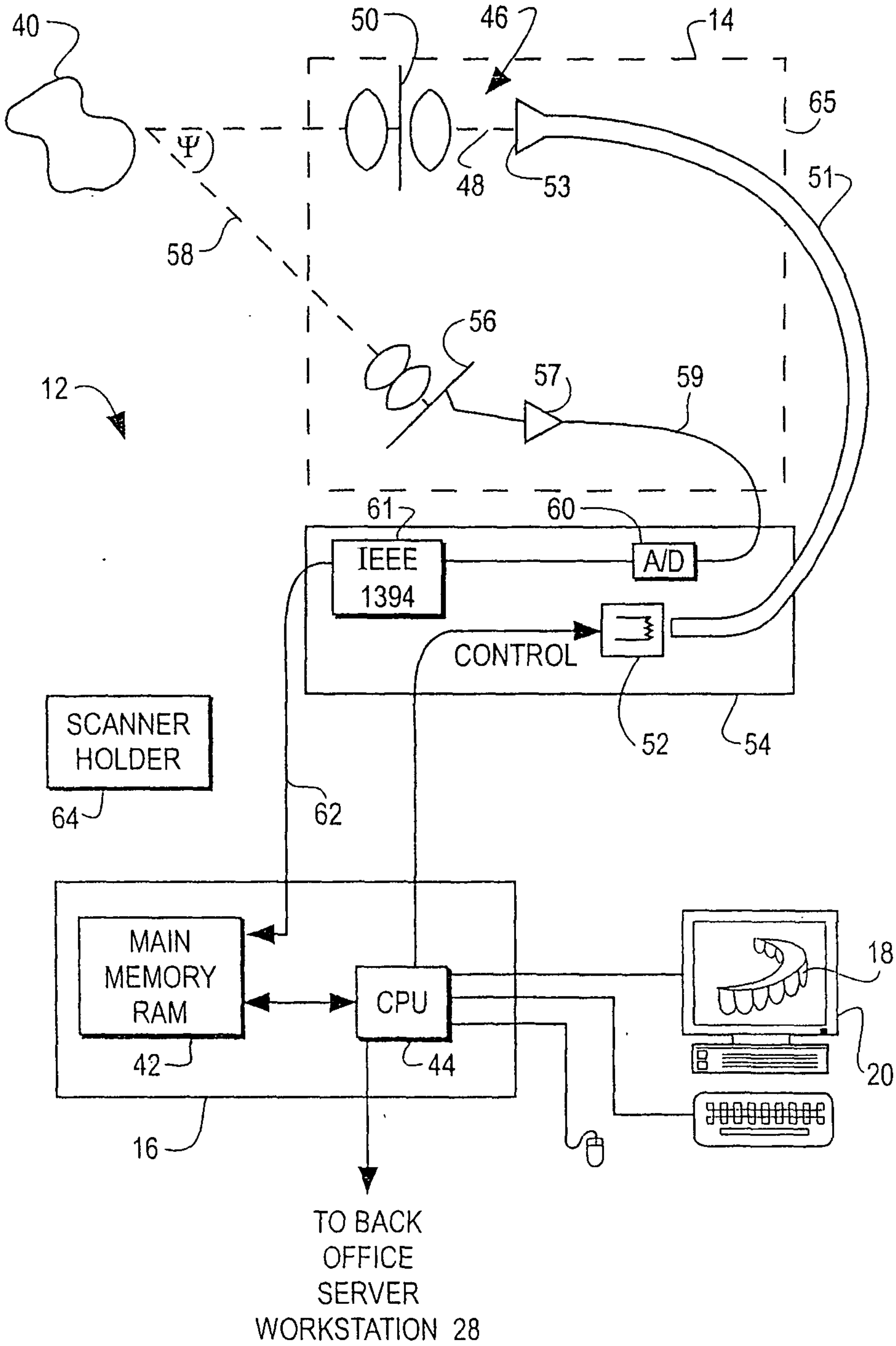
40
50
46
14
65
Ψ
48
53
51
58
56
57
59
12
61
60
IEEE
1394
A/D
CONTROL
52
54
SCANNER
HOLDER
64
62
MAIN
MEMORY
RAM
CPU
42
44
18
20
16
TO BACK
OFFICE
SERVER
WORKSTATION 28

FIG. 3

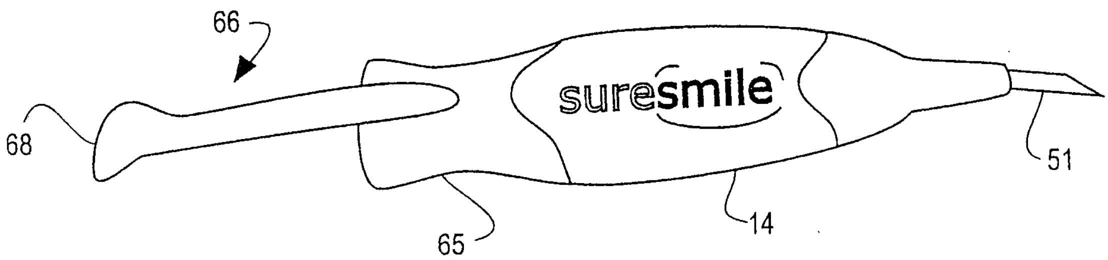


FIG. 4

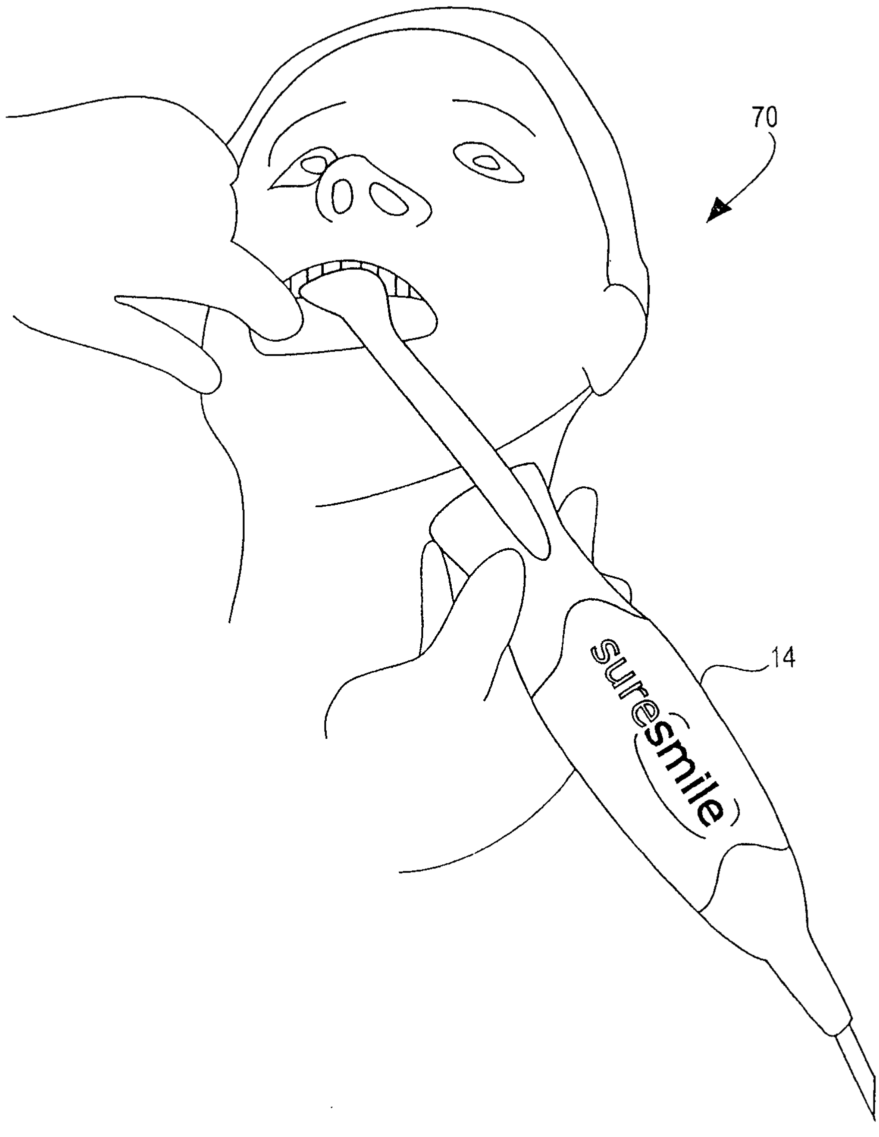

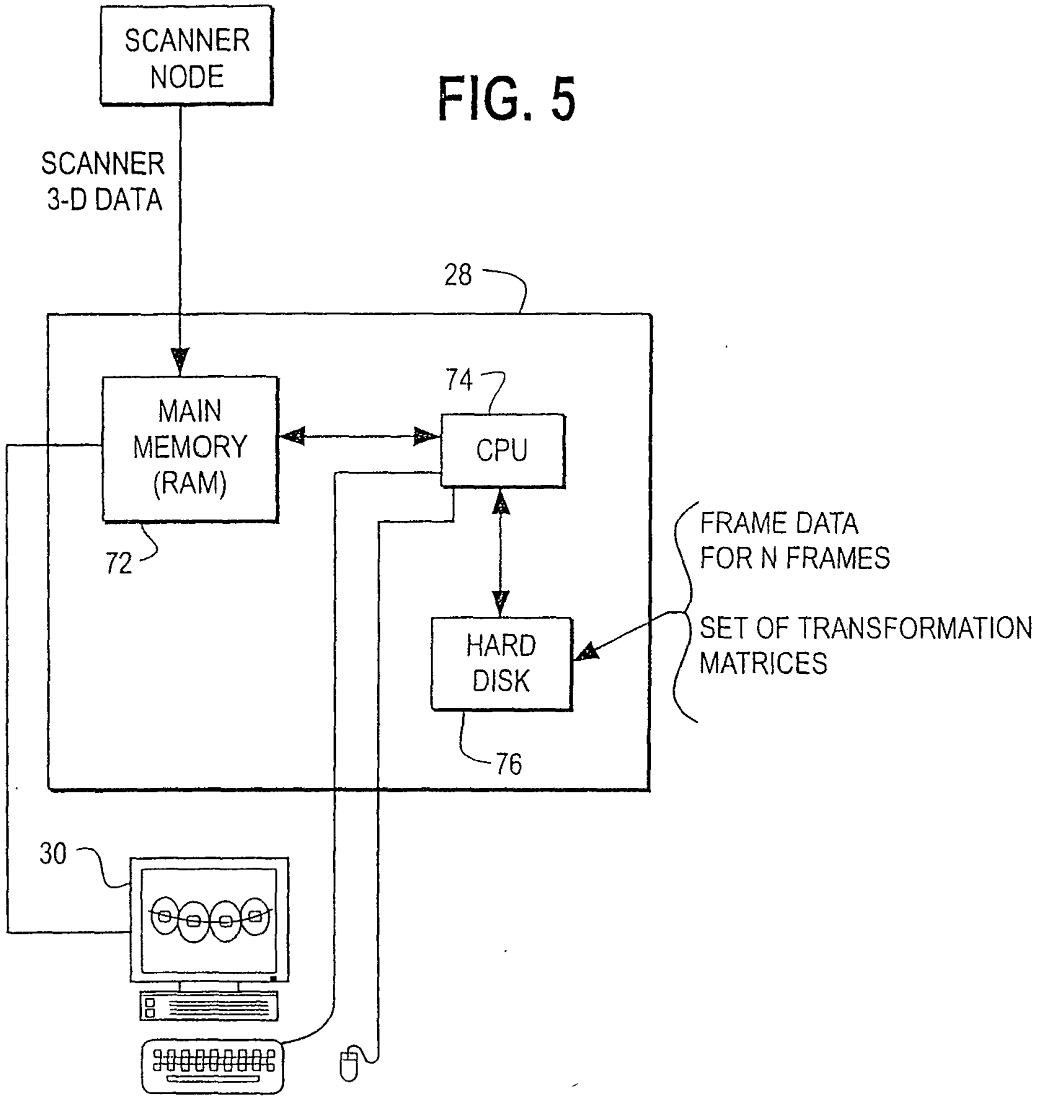
SCANNER
NODE
SCANNER
3-D DATA
28
MAIN
MEMORY
(RAM)
72
74
CPU
HARD
DISK
76
FRAME DATA
FOR N FRAMES
SET OF TRANSFORMATION
MATRICES
30

FIG. 5

FIG. 60

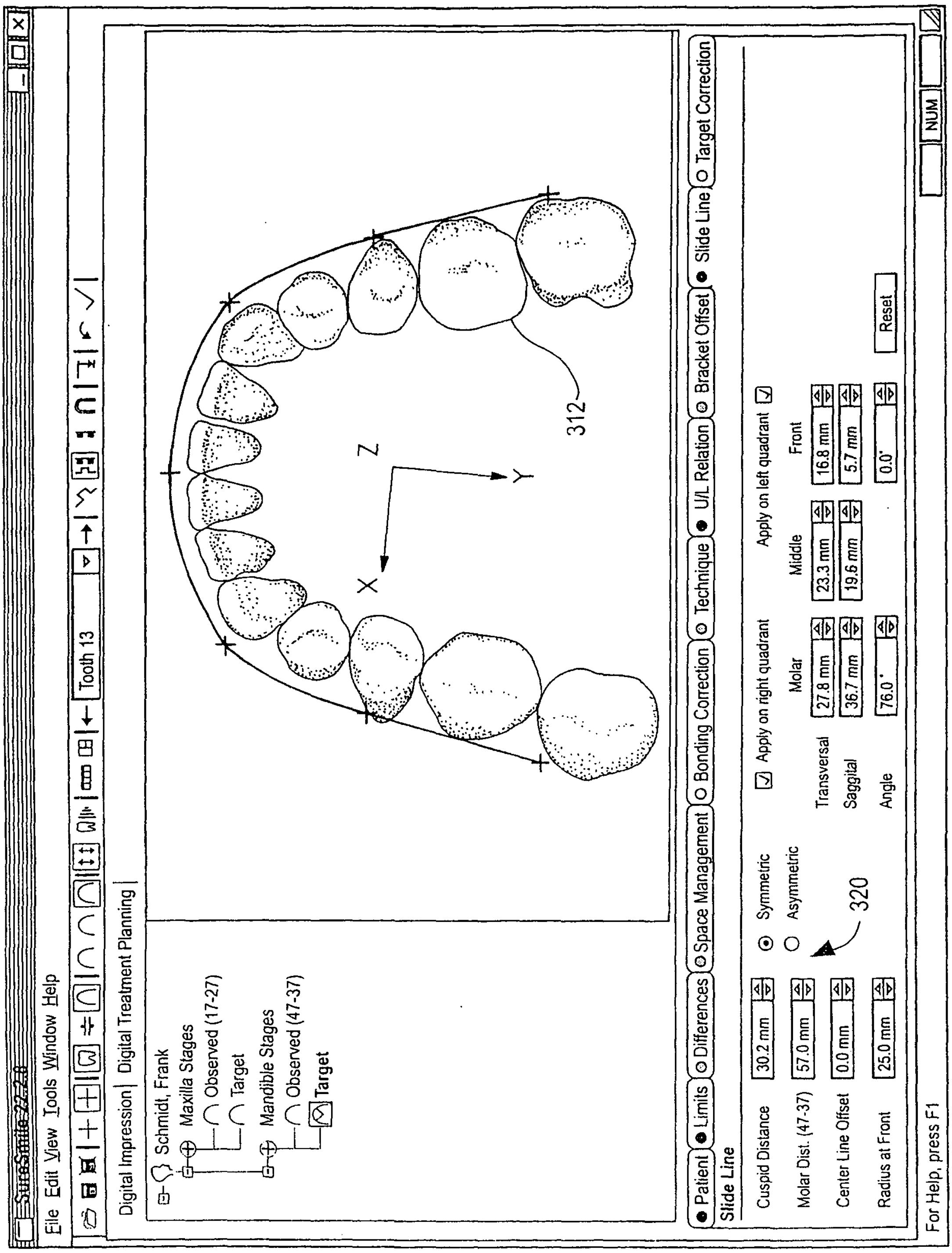
SureSmile-22.2.8
File Edit View Tools Window Help
Tooth 13
Digital Impression
Digital Treatment Planning
Schmidt, Frank
Maxilla Stages
Observed (17-27)
Target
Mandible Stages
Observed (47-37)
Target
Z
X
Y
312
Patient
Limits
Differences
Space Management
Bonding Correction
Technique
U/L Relation
Bracket Offset
Slide Line
Target Correction
Slide Line
Cuspid Distance
30.2 mm
Molar Dist. (47-37)
57.0 mm
Center Line Offset
0.0 mm
Radius at Front
25.0 mm
Symmetric
Asymmetric
320
Apply on right quadrant
Apply on left quadrant
Molar
Middle
Front
Transversal
27.8 mm
23.3 mm
16.8 mm
Saggital
36.7 mm
19.6 mm
5.7 mm
Angle
76.0°
0.0°
Reset
For Help, press F1
NUM

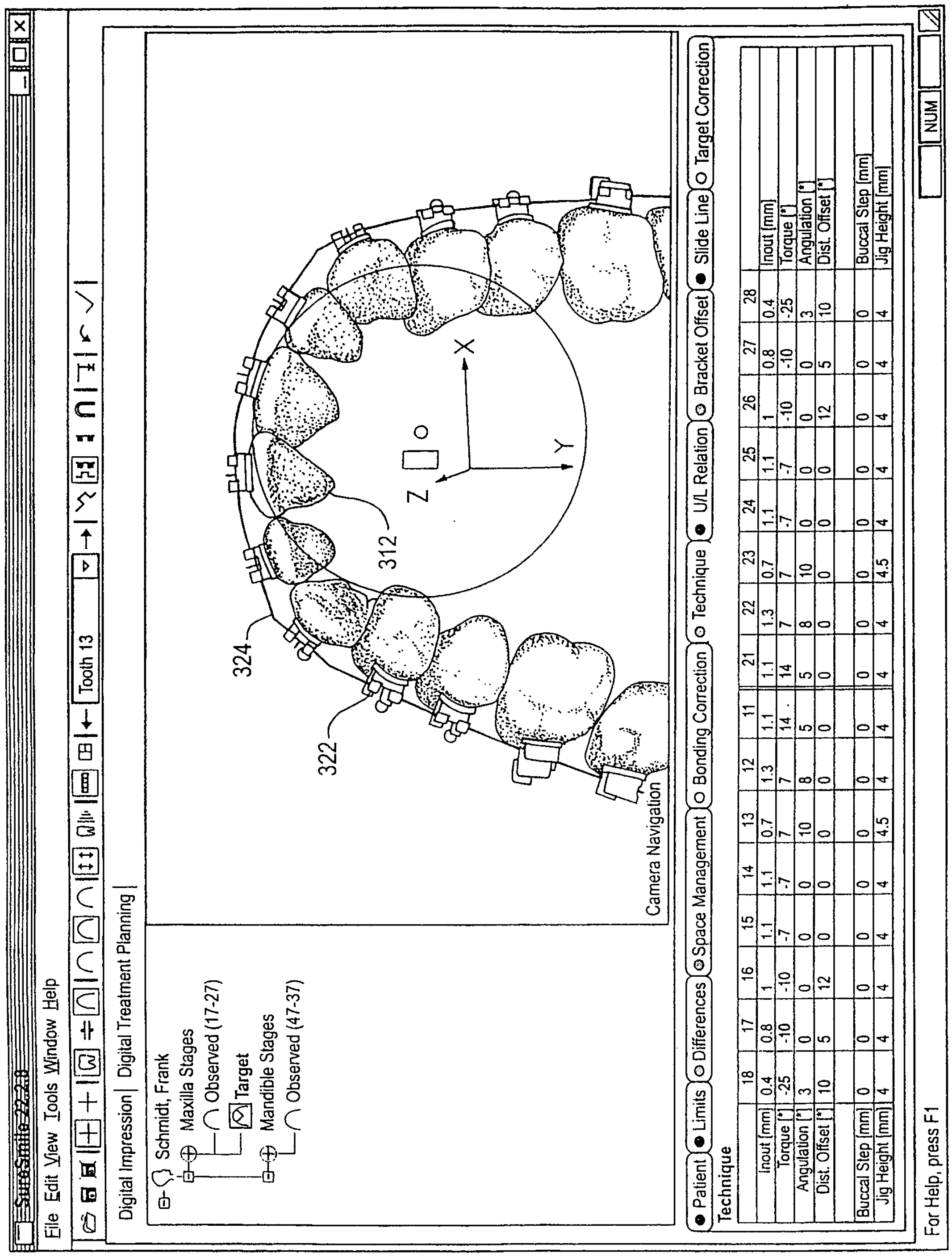
File Edit View Tools Window Help
Tooth 13
Digital Impression
Digital Treatment Planning
Schmidt, Frank
Maxilla Stages
Observed (17-27)
Target
Mandible Stages
Observed (47-37)
Camera Navigation
322
324
312
Z
Y
X
Patient
Limits
Differences
Space Management
Bonding Correction
Technique
U/L Relation
Bracket Offset
Slide Line
Target Correction
Technique
Inout [mm]
Torque [°]
Angulation [°]
Dist. Offset [°]
Buccal Step [mm]
Jig Height [mm]
For Help, press F1
NUM

FIG. 61

FIG. 62

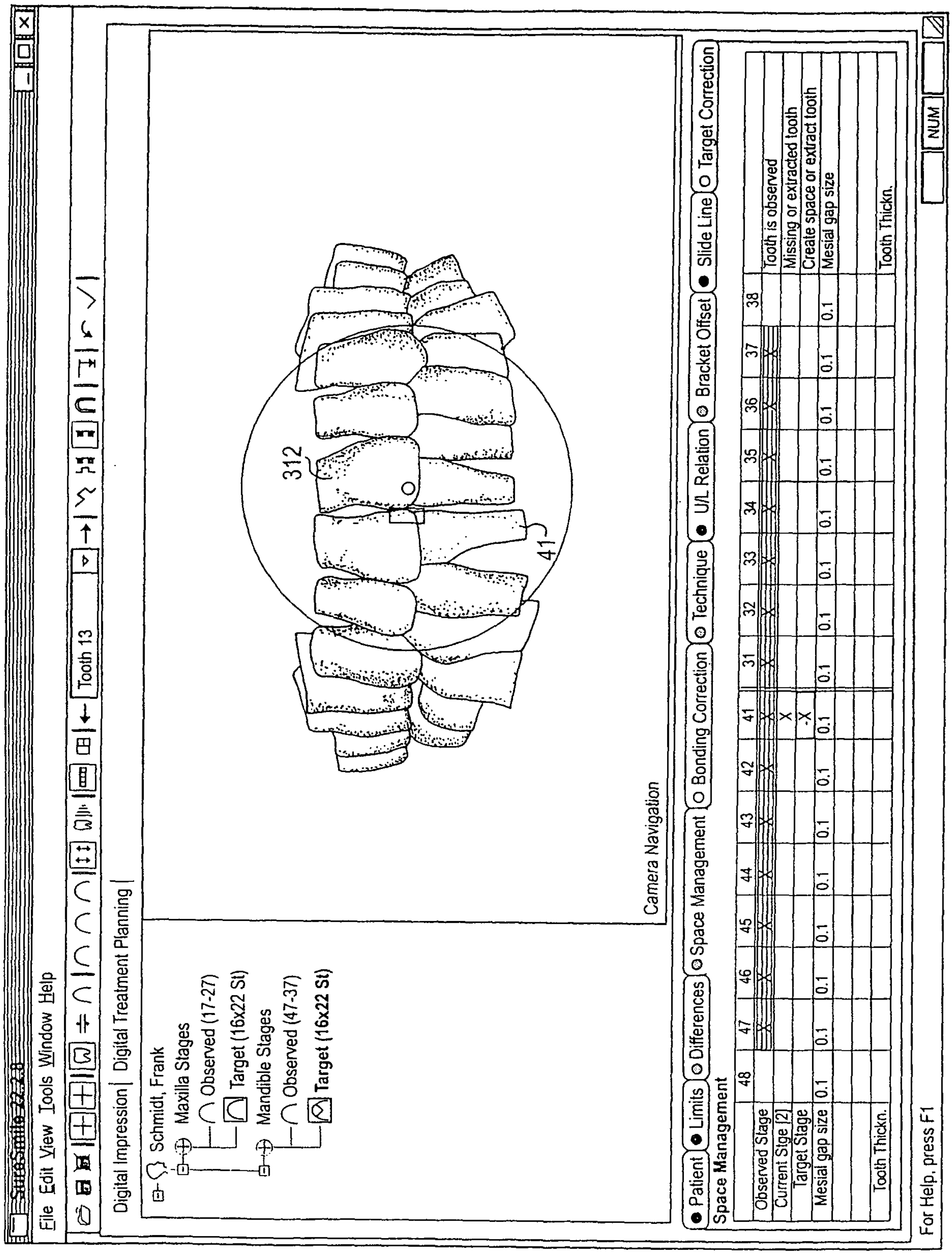
File Edit View Tools Window Help
Tooth 13
Digital Impression
Digital Treatment Planning
Schmidt, Frank
Maxilla Stages
Observed (17-27)
Target (16x22 St)
Mandible Stages
Observed (47-37)
Target (16x22 St)
312
41
Camera Navigation
Patient
Limits
Differences
Space Management
Bonding Correction
Technique
U/L Relation
Bracket Offset
Slide Line
Target Correction
Space Management
48 47 46 45 44 43 42 41 31 32 33 34 35 36 37 38
Observed Stage
Current Stge [2]
Target Stage
Mesial gap size
Tooth Thickn.
Tooth is observed
Missing or extracted tooth
Create space or extract tooth
Mesial gap size
Tooth Thickn.
For Help, press F1
NUM

FIG. 63

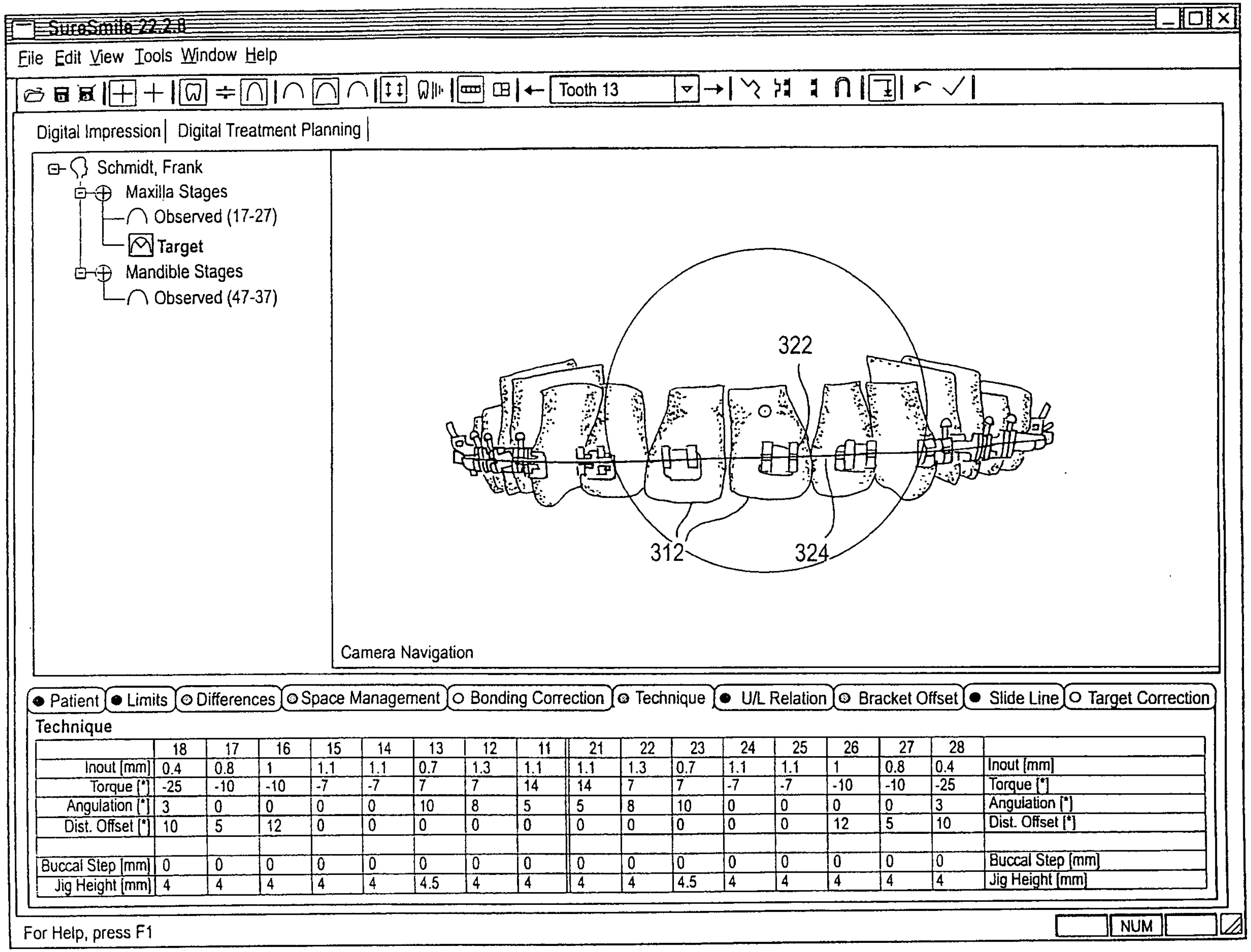
SureSmile 22.2.8
File Edit View Tools Window Help
Tooth 13
Digital Impression
Digital Treatment Planning
Schmidt, Frank
Maxilla Stages
Observed (17-27)
Target
Mandible Stages
Observed (47-37)
322
312
324
Camera Navigation
Patient
Limits
Differences
Space Management
Bonding Correction
Technique
U/L Relation
Bracket Offset
Slide Line
Target Correction
Technique
18 17 16 15 14 13 12 11 21 22 23 24 25 26 27 28
Inout [mm] 0.4 0.8 1 1.1 1.1 0.7 1.3 1.1 1.1 1.3 0.7 1.1 1.1 1 0.8 0.4 Inout [mm]
Torque [°] -25 -10 -10 -7 -7 7 7 14 14 7 7 -7 -7 -10 -10 -25 Torque [°]
Angulation [°] 3 0 0 0 0 10 8 5 5 8 10 0 0 0 0 3 Angulation [°]
Dist. Offset [°] 10 5 12 0 0 0 0 0 0 0 0 0 0 12 5 10 Dist. Offset [°]
Buccal Step [mm] 0 0 0 0 0 0 0 0 0 0 0 0 0 0 0 0 Buccal Step [mm]
Jig Height [mm] 4 4 4 4 4 4.5 4 4 4 4 4.5 4 4 4 4 4 Jig Height [mm]
For Help, press F1
NUM

FIG. 66

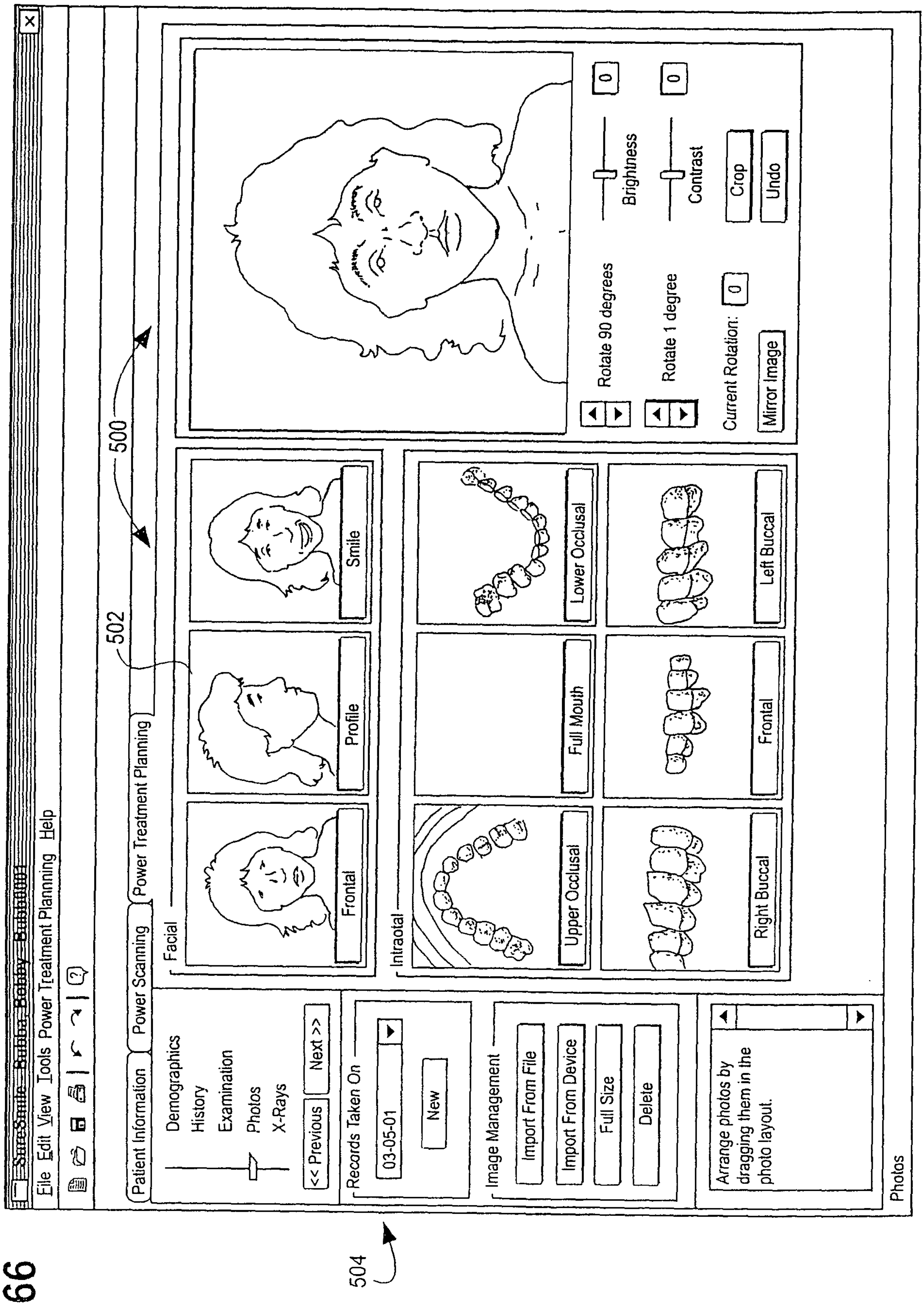

SureSmile Bubba, Bobby Bub0001
File Edit View Tools Power Treatment Plannning Help
Patient Information
Power Scanning
Power Treatment Planning
Demographics
History
Examination
Photos
X-Rays
<< Previous
Next >>
Records Taken On
03-05-01
New
Image Management
Import From File
Import From Device
Full Size
Delete
Arrange photos by dragging them in the photo layout.
Photos
Facial
Frontal
Profile
Smile
Intraotal
Upper Occlusal
Full Mouth
Lower Occlusal
Right Buccal
Frontal
Left Buccal
Rotate 90 degrees
Rotate 1 degree
Current Rotation:
0
Mirror Image
Brightness
Contrast
Crop
Undo
500
502
504

FIG. 67

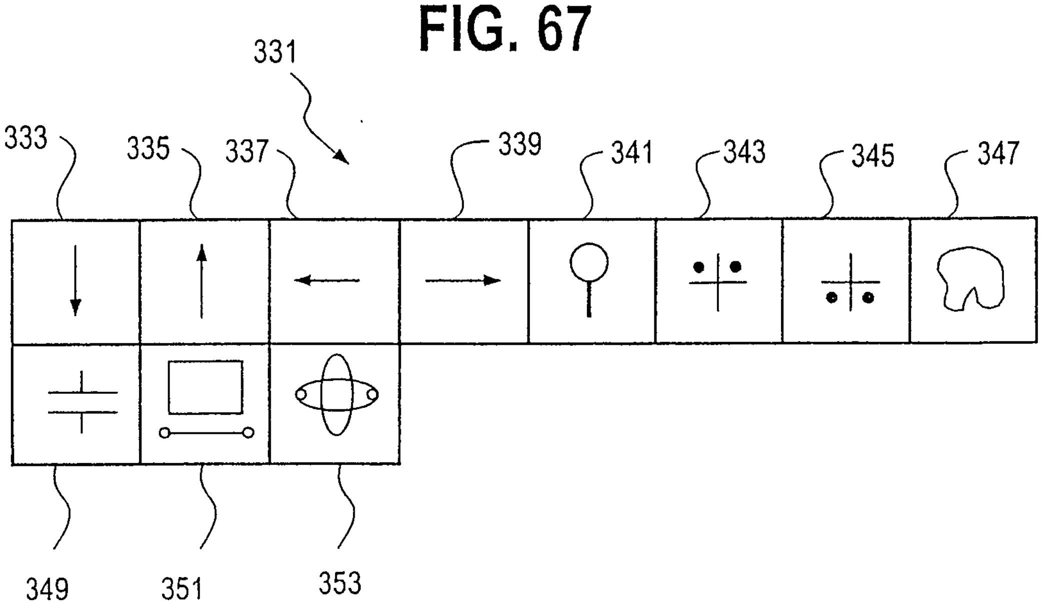
331
333
335
337
339
341
343
345
347
349
351
353

FIG. 68

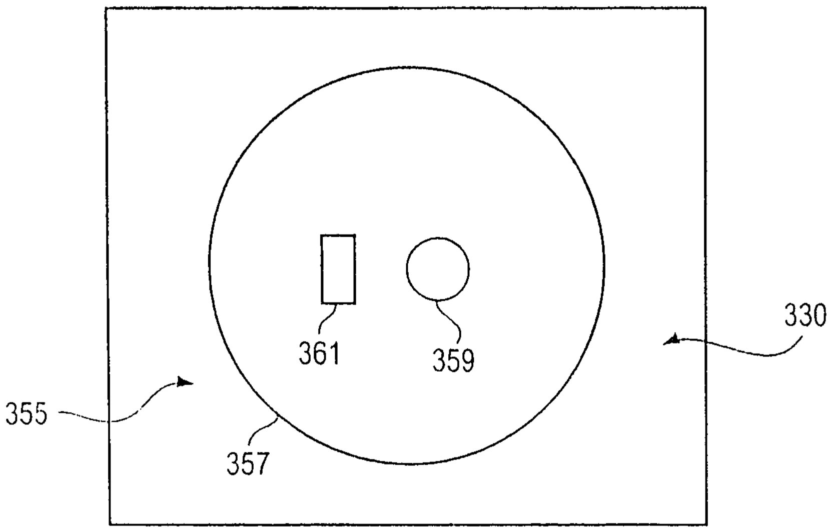
361
359
330
355
357

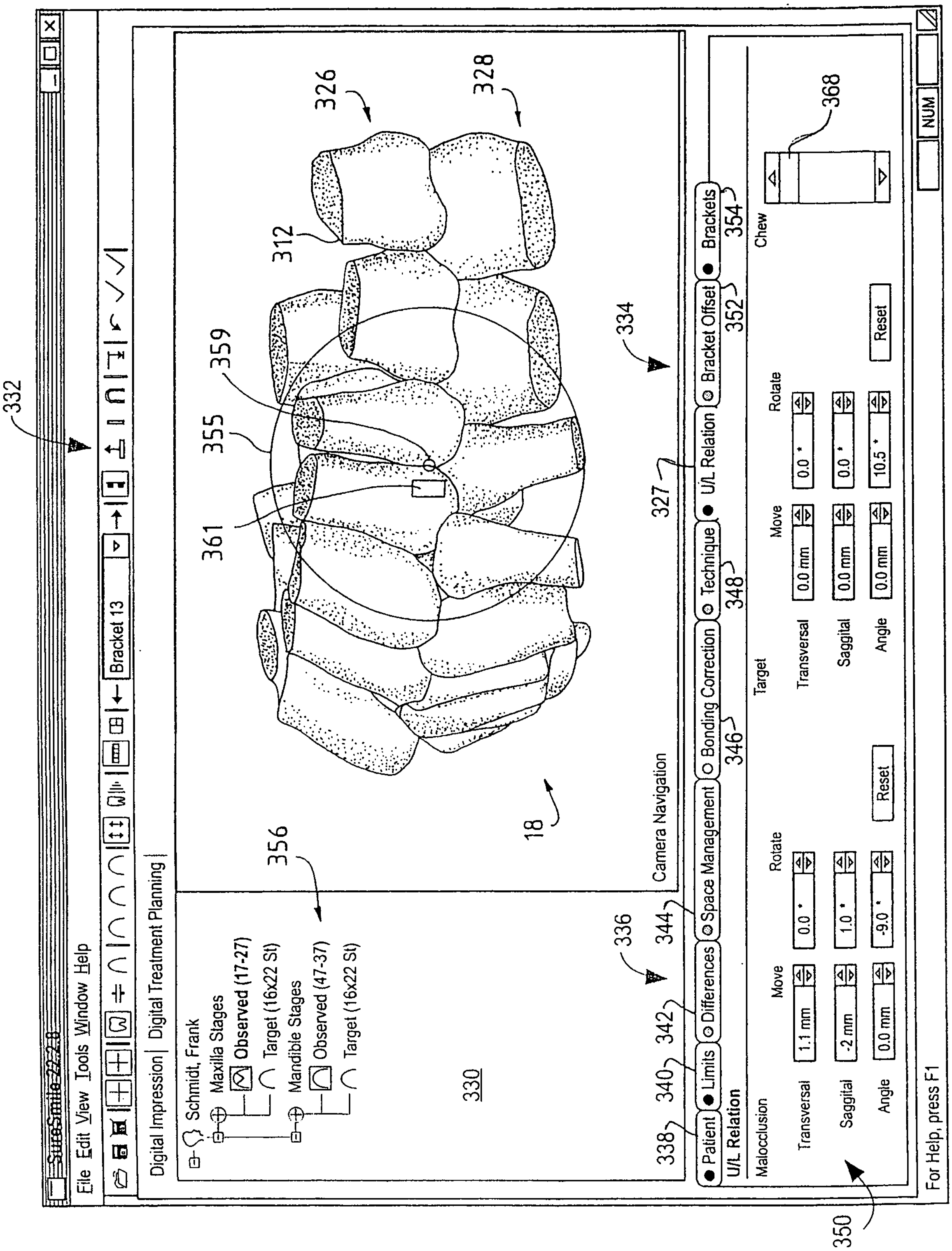
SureSmile 22.7.0
File Edit View Tools Window Help
Bracket 13
Digital Impression
Digital Treatment Planning
Schmidt, Frank
Maxilla Stages
Observed (17-27)
Target (16x22 St)
Mandible Stages
Observed (47-37)
Target (16x22 St)
330
Camera Navigation
Patient
Limits
Differences
Space Management
Bonding Correction
Technique
U/L Relation
Bracket Offset
Brackets
U/L Relation
Malocclusion
Move
Rotate
Transversal
Saggital
Angle
1.1 mm
-2 mm
0.0 mm
0.0 °
1.0 °
-9.0 °
Reset
Target
0.0 mm
0.0 °
10.5 °
Chew
NUM
For Help, press F1
332
326
328
312
359
355
361
356
18
334
336
327
338
340
342
344
346
348
352
354
368
350

FIG. 69

FIG. 70

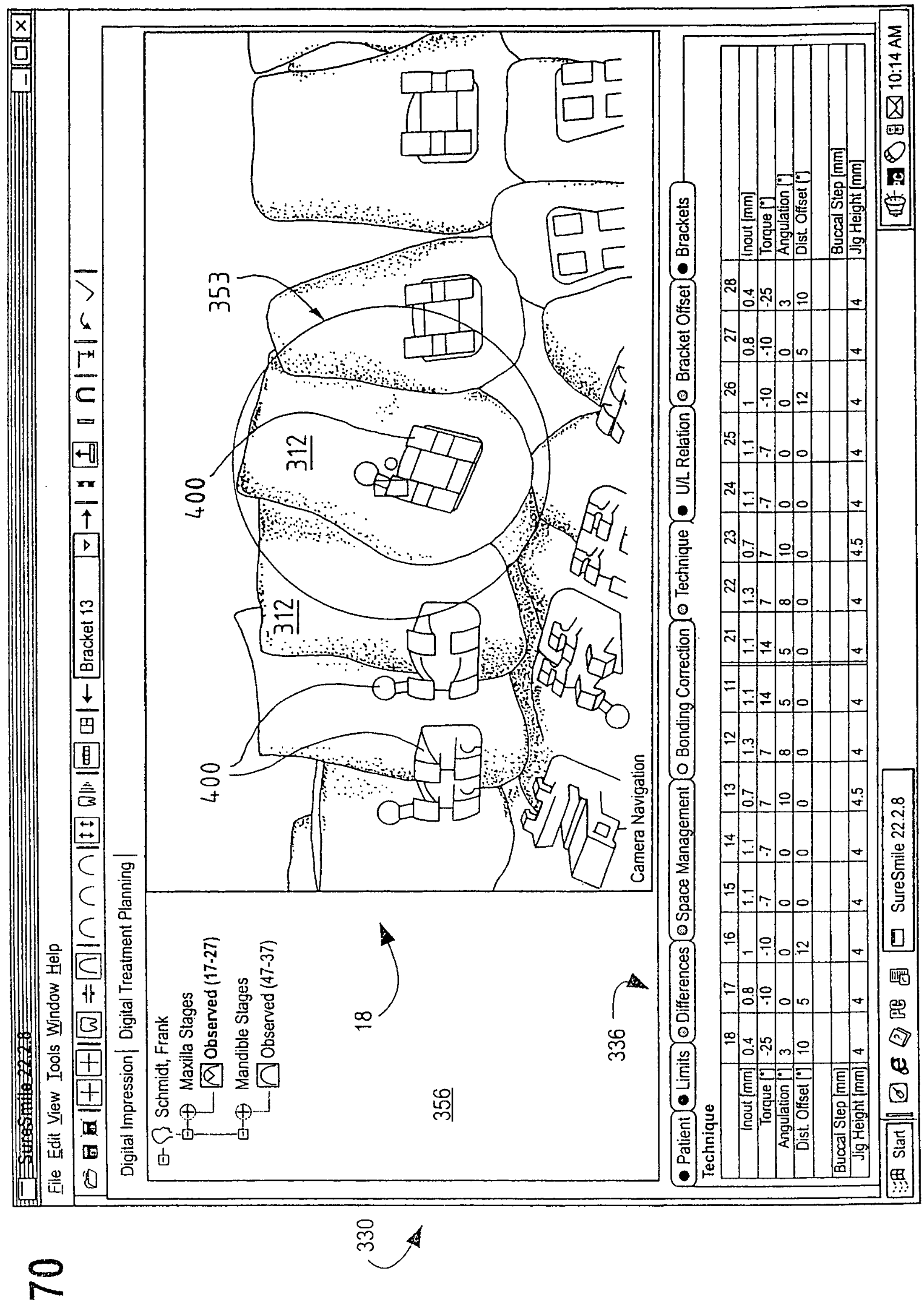
SureSmile 22.2.8
File Edit View Tools Window Help
Bracket 13
Digital Impression
Digital Treatment Planning
Schmidt, Frank
Maxilla Stages
Observed (17-27)
Mandible Stages
Observed (47-37)
356
18
336
Camera Navigation
400
312
312
400
353
Patient
Limits
Differences
Space Management
Bonding Correction
Technique
U/L Relation
Bracket Offset
Brackets
Technique
18 17 16 15 14 13 12 11 21 22 23 24 25 26 27 28
Inout [mm] 0.4 0.8 1 1.1 1.1 0.7 1.3 1.1 1.1 1.3 0.7 1.1 1.1 1 0.8 0.4
Torque [°] -25 -10 -10 -7 -7 7 7 14 14 7 7 -7 -7 -10 -10 -25
Angulation [°] 3 0 0 0 0 10 8 5 5 8 10 0 0 0 0 3
Dist. Offset [°] 10 5 12 0 0 0 0 0 0 0 0 0 0 12 5 10
Buccal Step [mm]
Jig Height [mm] 4 4 4 4 4 4.5 4 4 4 4 4.5 4 4 4 4 4
Start
SureSmile 22.2.8
10:14 AM
330

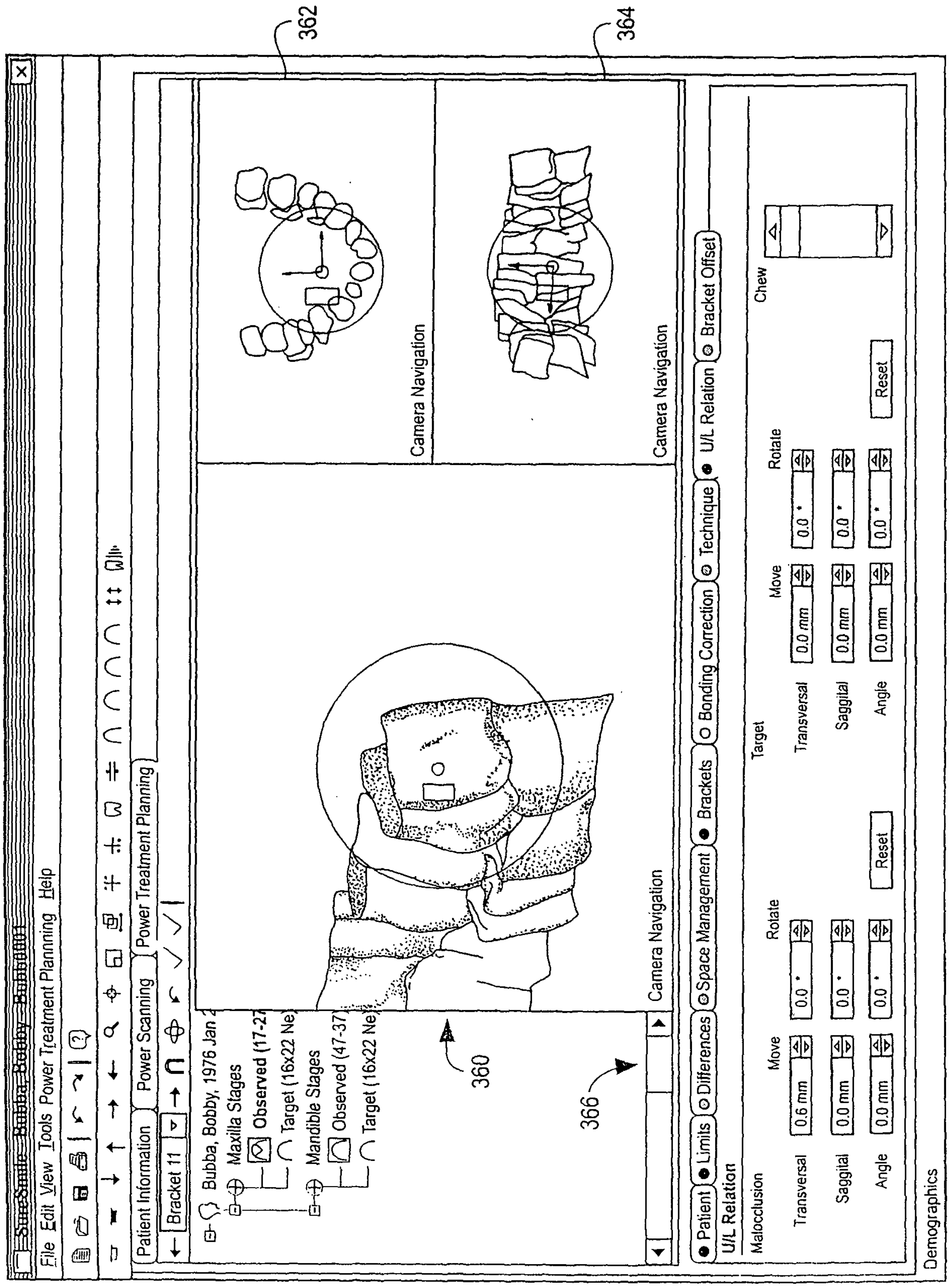
File Edit View Tools Power Treatment Plannning Help
Patient Information
Power Scanning
Power Treatment Planning
Bracket 11
Bubba, Bobby, 1976 Jan 2
Maxilla Stages
Observed (17-27
Target (16x22 Ne
Mandible Stages
Observed (47-37
Target (16x22 Ne
Camera Navigation
Camera Navigation
Camera Navigation
360
362
364
366
Patient
Limits
Differences
Space Management
Brackets
Bonding Correction
Technique
U/L Relation
Bracket Offset
U/L Relation
Malocclusion
Target
Move
Rotate
Transversal
Saggital
Angle
0.6 mm
0.0 mm
0.0 mm
0.0 °
0.0 °
0.0 °
Reset
Transversal
Saggital
Angle
0.0 mm
0.0 mm
0.0 mm
0.0 °
0.0 °
0.0 °
Reset
Chew
Demographics

FIG. 71

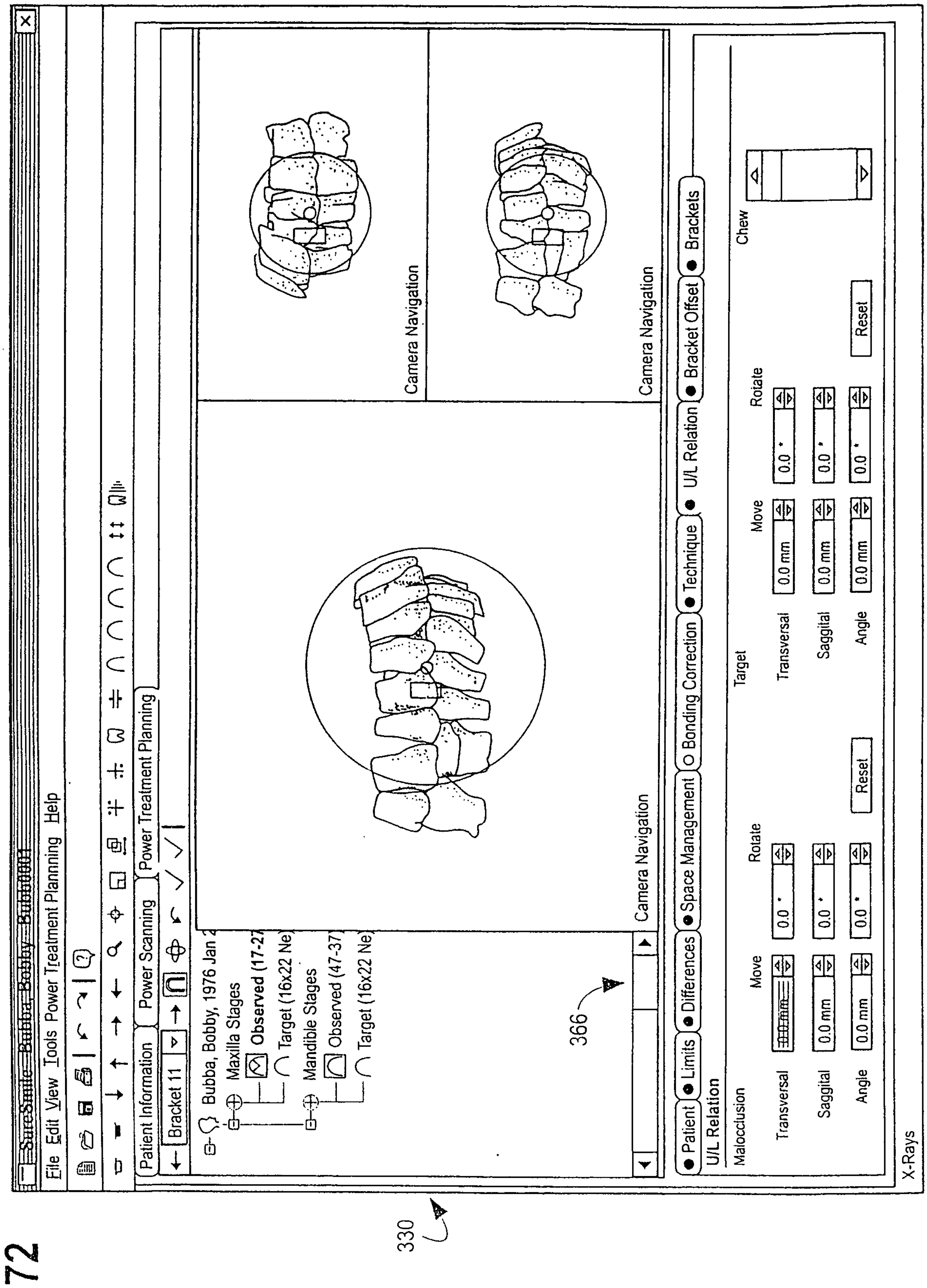
SureSmile - Bubba, Bobby - Bub00001
File Edit View Tools Power Treatment Plannning Help
Patient Information
Power Scanning
Power Treatment Planning
Bracket 11
Bubba, Bobby, 1976 Jan 2
Maxilla Stages
Observed (17-27
Target (16x22 Ne
Mandible Stages
Observed (47-37
Target (16x22 Ne
Camera Navigation
Camera Navigation
Camera Navigation
366
330
Patient
Limits
Differences
Space Management
Bonding Correction
Technique
U/L Relation
Bracket Offset
Brackets
U/L Relation
Malocclusion
Move
Rotate
Transversal
Saggital
Angle
0.0 mm
0.0 °
Reset
Target
Transversai
Chew
X-Rays

FIG. 72

FIG. 73

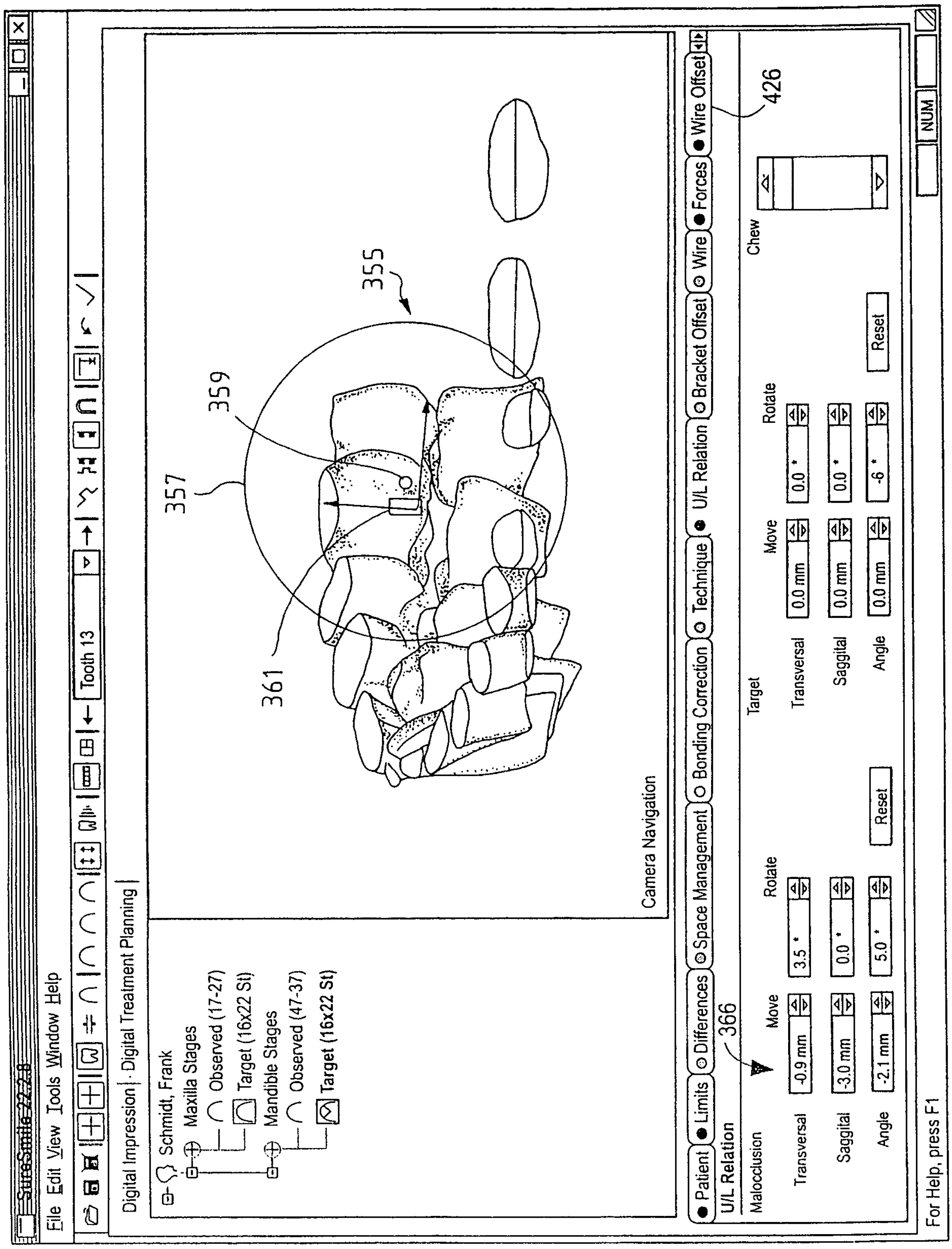

SureSmile 2.2.8
File Edit View Tools Window Help
Tooth 13
Digital Impression | Digital Treatment Planning |
Schmidt, Frank
Maxilla Stages
Observed (17-27)
Target (16x22 St)
Mandible Stages
Observed (47-37)
Target (16x22 St)
Camera Navigation
355
357
359
361
Patient
Limits
Differences
Space Management
Bonding Correction
Technique
U/L Relation
Bracket Offset
Wire
Forces
Wire Offset
U/L Relation
366
Malocclusion
Move
Rotate
Transversal
-0.9 mm
3.5 °
Saggital
-3.0 mm
0.0 °
Angle
-2.1 mm
5.0 °
Reset
Target
Move
Rotate
Transversal
0.0 mm
0.0 °
Saggital
0.0 mm
0.0 °
Angle
0.0 mm
-6 °
Reset
Chew
426
For Help, press F1
NUM

FIG. 74

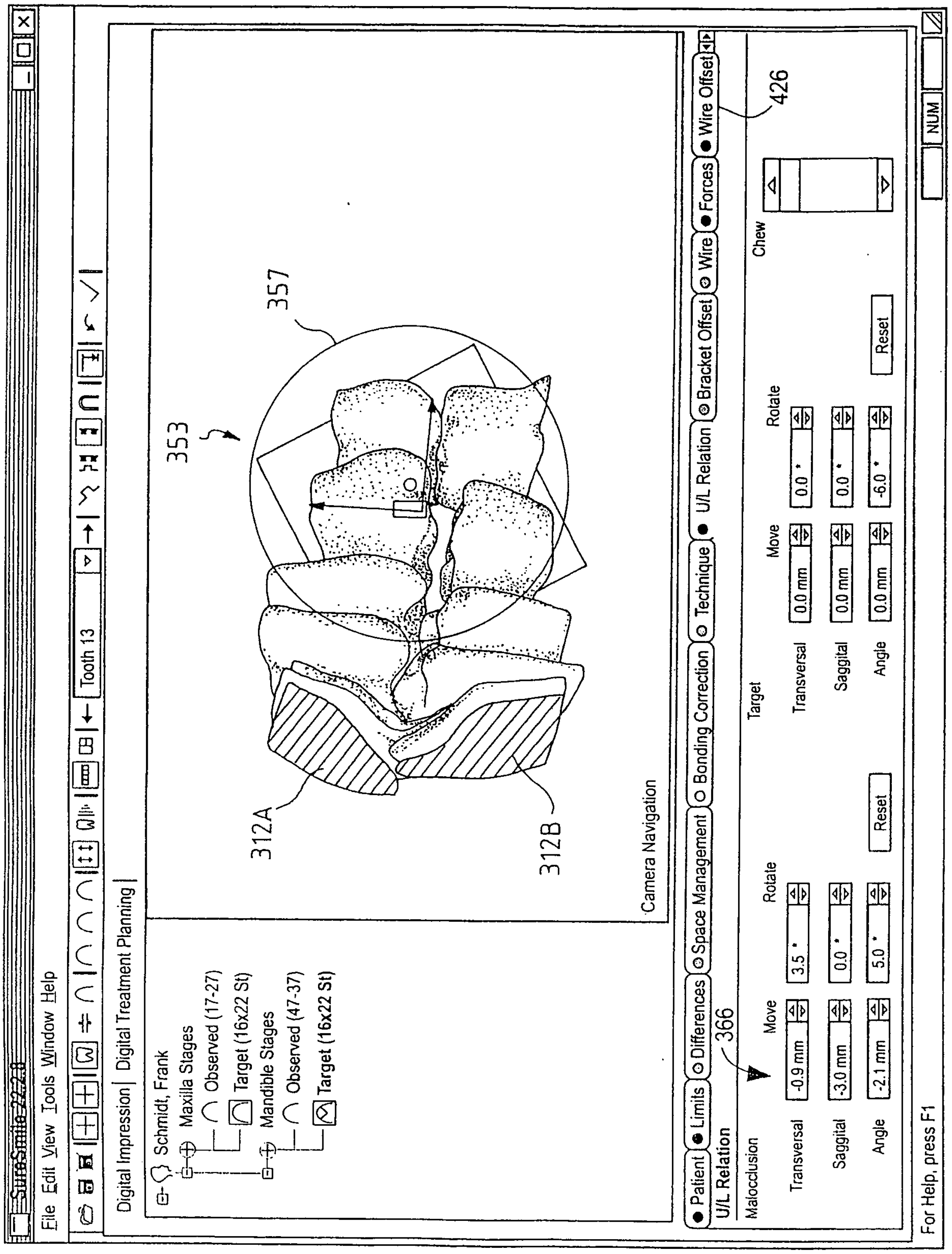
SureSmile 22.2.0
File Edit View Tools Window Help
Tooth 13
Digital Impression
Digital Treatment Planning
Schmidt, Frank
Maxilla Stages
Observed (17-27)
Target (16x22 St)
Mandible Stages
Observed (47-37)
Target (16x22 St)
353
357
312A
312B
Camera Navigation
Patient
Limits
Differences
Space Management
Bonding Correction
Technique
U/L Relation
Bracket Offset
Wire
Forces
Wire Offset
426
366
U/L Relation
Malocclusion
Move
Rotate
Transversal
-0.9 mm
3.5 °
Saggital
-3.0 mm
0.0 °
Angle
-2.1 mm
5.0 °
Reset
Target
Move
Rotate
Transversal
0.0 mm
0.0 °
Saggital
0.0 mm
0.0 °
Angle
0.0 mm
-6.0 °
Reset
Chew
For Help, press F1
NUM

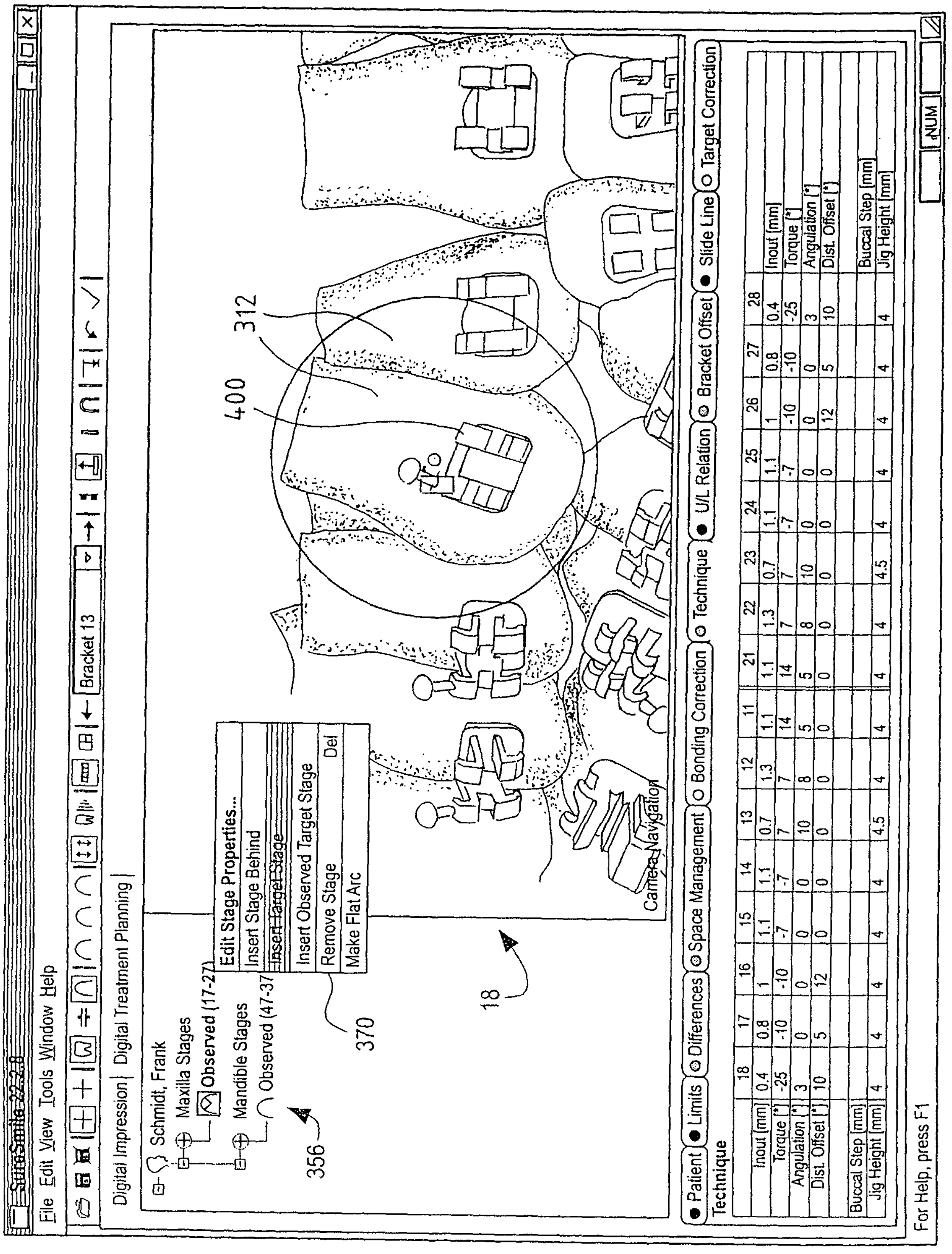
SureSmile 22.2.8
File Edit View Tools Window Help
Bracket 13
Digital Impression
Digital Treatment Planning
Schmidt, Frank
Maxilla Stages
Observed (17-27)
Mandible Stages
Observed (47-37)
Edit Stage Properties...
Insert Stage Behind
Insert Target Stage
Insert Observed Target Stage
Remove Stage Del
Make Flat Arc
Camera Navigation
356
370
18
400
312
Patient
Limits
Differences
Space Management
Bonding Correction
Technique
U/L Relation
Bracket Offset
Slide Line
Target Correction
Technique
Inout [mm]
Torque [°]
Angulation [°]
Dist. Offset [°]
Buccal Step [mm]
Jig Height [mm]
For Help, press F1
NUM

FIG. 75

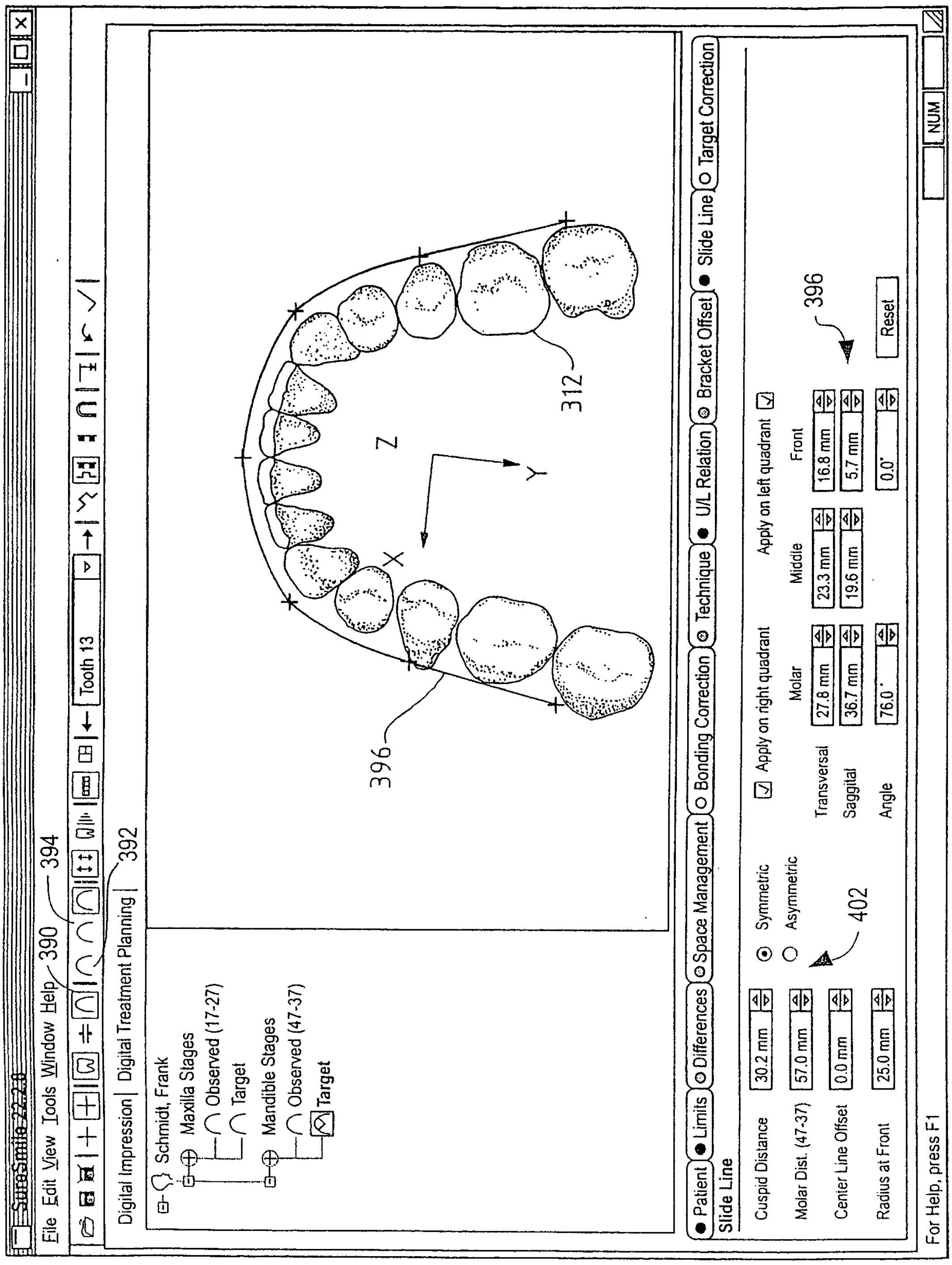
SureSmile 22.2.8
File Edit View Tools Window Help
Tooth 13
Digital Impression
Digital Treatment Planning
Schmidt, Frank
Maxilla Stages
Observed (17-27)
Target
Mandible Stages
Observed (47-37)
Target
390
394
392
396
312
402
Z
Y
X
Patient
Limits
Differences
Space Management
Bonding Correction
Technique
U/L Relation
Bracket Offset
Slide Line
Target Correction
Slide Line
Cuspid Distance
30.2 mm
Molar Dist. (47-37)
57.0 mm
Center Line Offset
0.0 mm
Radius at Front
25.0 mm
Symmetric
Asymmetric
Apply on right quadrant
Apply on left quadrant
Molar
Middle
Front
Transversal
27.8 mm
23.3 mm
16.8 mm
Saggital
36.7 mm
19.6 mm
5.7 mm
Angle
76.0˚
0.0˚
Reset
For Help, press F1
NUM

FIG. 76

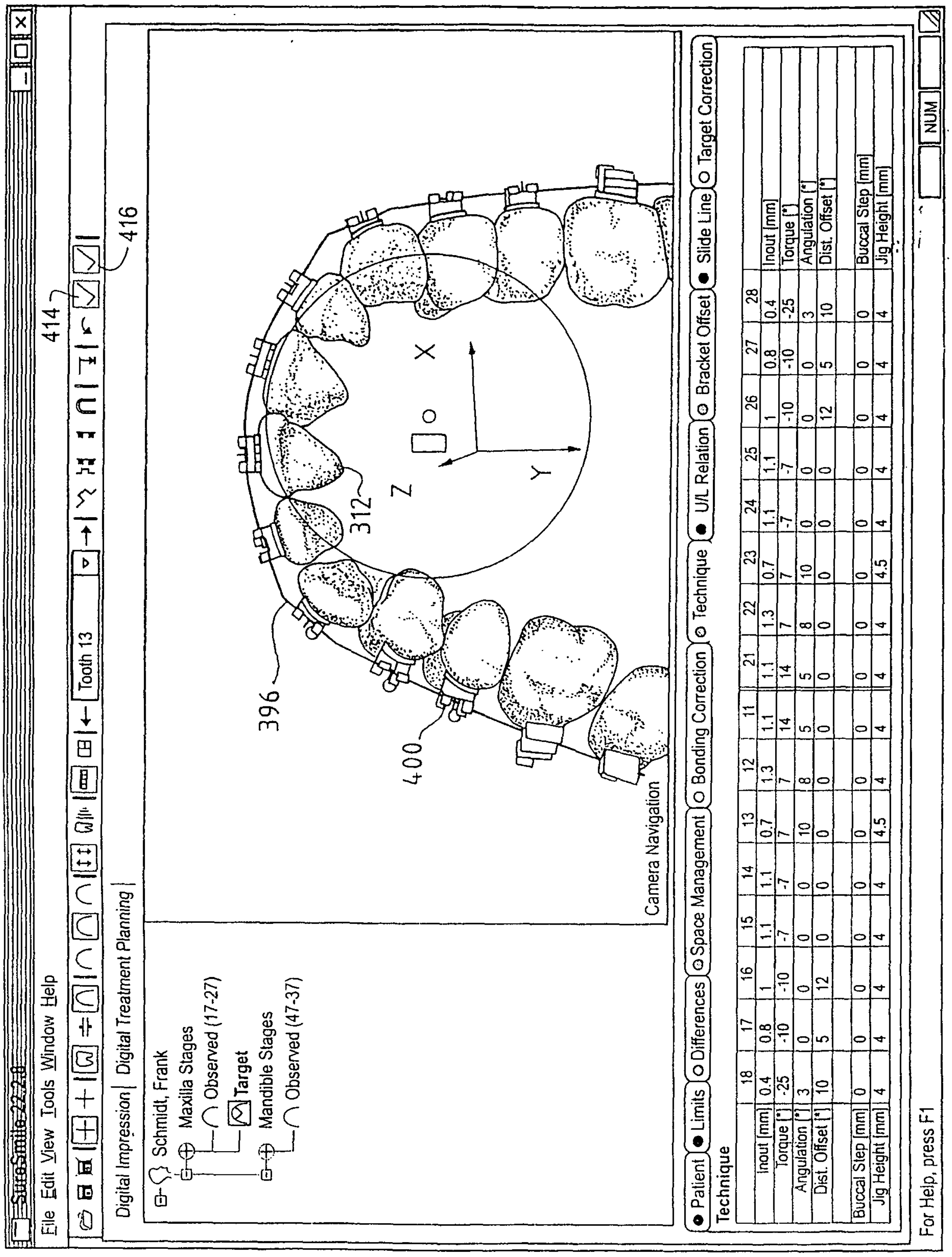
SureSmile 2.2.8
File Edit View Tools Window Help
Tooth 13
414
416
Digital Impression
Digital Treatment Planning
Schmidt, Frank
Maxilla Stages
Observed (17-27)
Target
Mandible Stages
Observed (47-37)
312
396
400
X
Y
Z
Camera Navigation
Patient
Limits
Differences
Space Management
Bonding Correction
Technique
U/L Relation
Bracket Offset
Slide Line
Target Correction
Technique
Inout [mm]
Torque [°]
Angulation [°]
Dist. Offset [°]
Buccal Step [mm]
Jig Height [mm]
For Help, press F1
NUM

FIG. 77

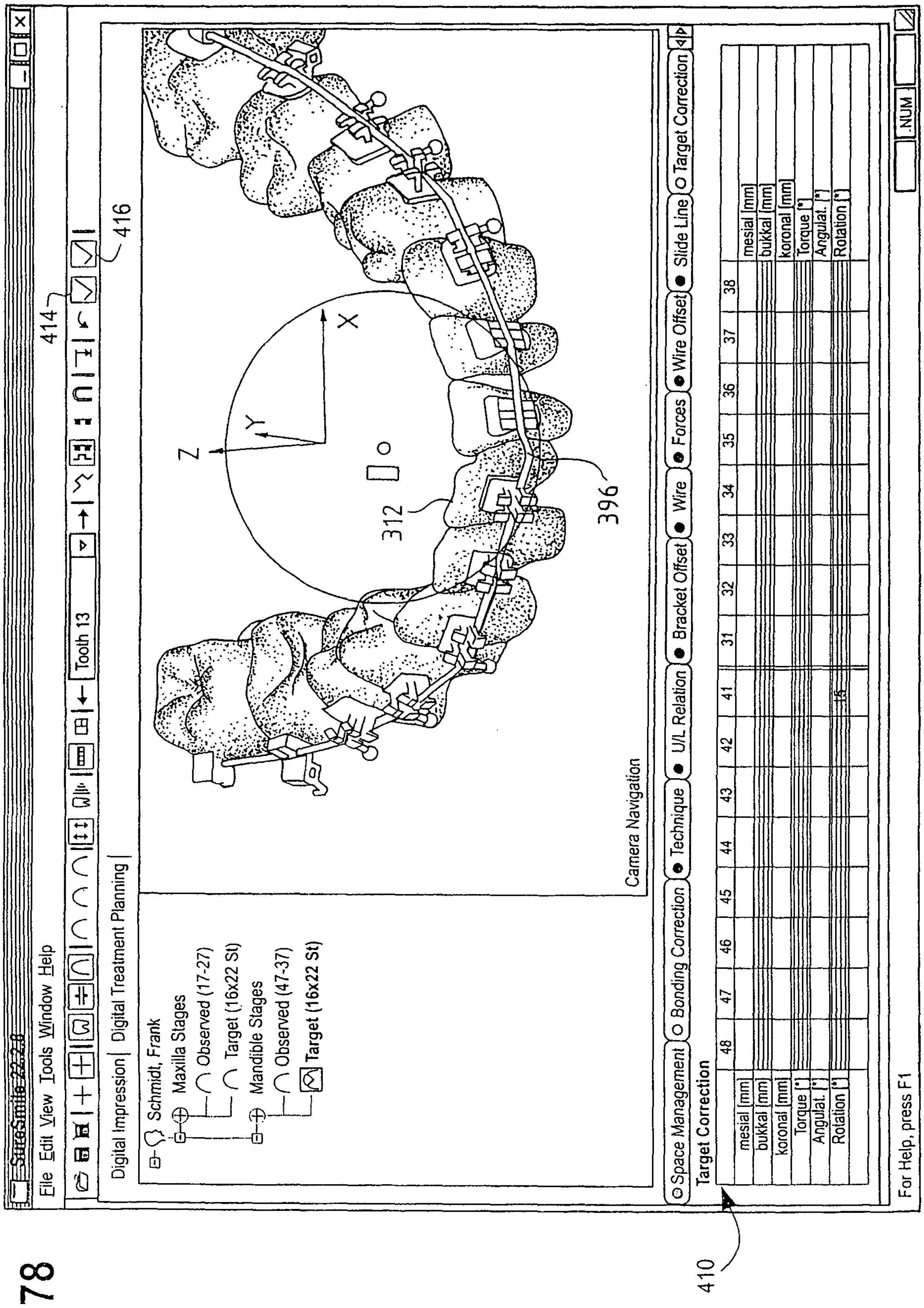
SureSmile 22.2.8
File Edit View Tools Window Help
Tooth 13
414
416
Digital Impression
Digital Treatment Planning
Schmidt, Frank
Maxilla Stages
Observed (17-27)
Target (16x22 St)
Mandible Stages
Observed (47-37)
Target (16x22 St)
Z
Y
X
312
396
Camera Navigation
Space Management
Bonding Correction
Technique
U/L Relation
Bracket Offset
Wire
Forces
Wire Offset
Slide Line
Target Correction
48
47
46
45
44
43
42
41
31
32
33
34
35
36
37
38
mesial [mm]
bukkal [mm]
koronal [mm]
Torque [°]
Angulat. [°]
Rotation [°]
For Help, press F1
NUM
410

FIG. 78

FIG. 79

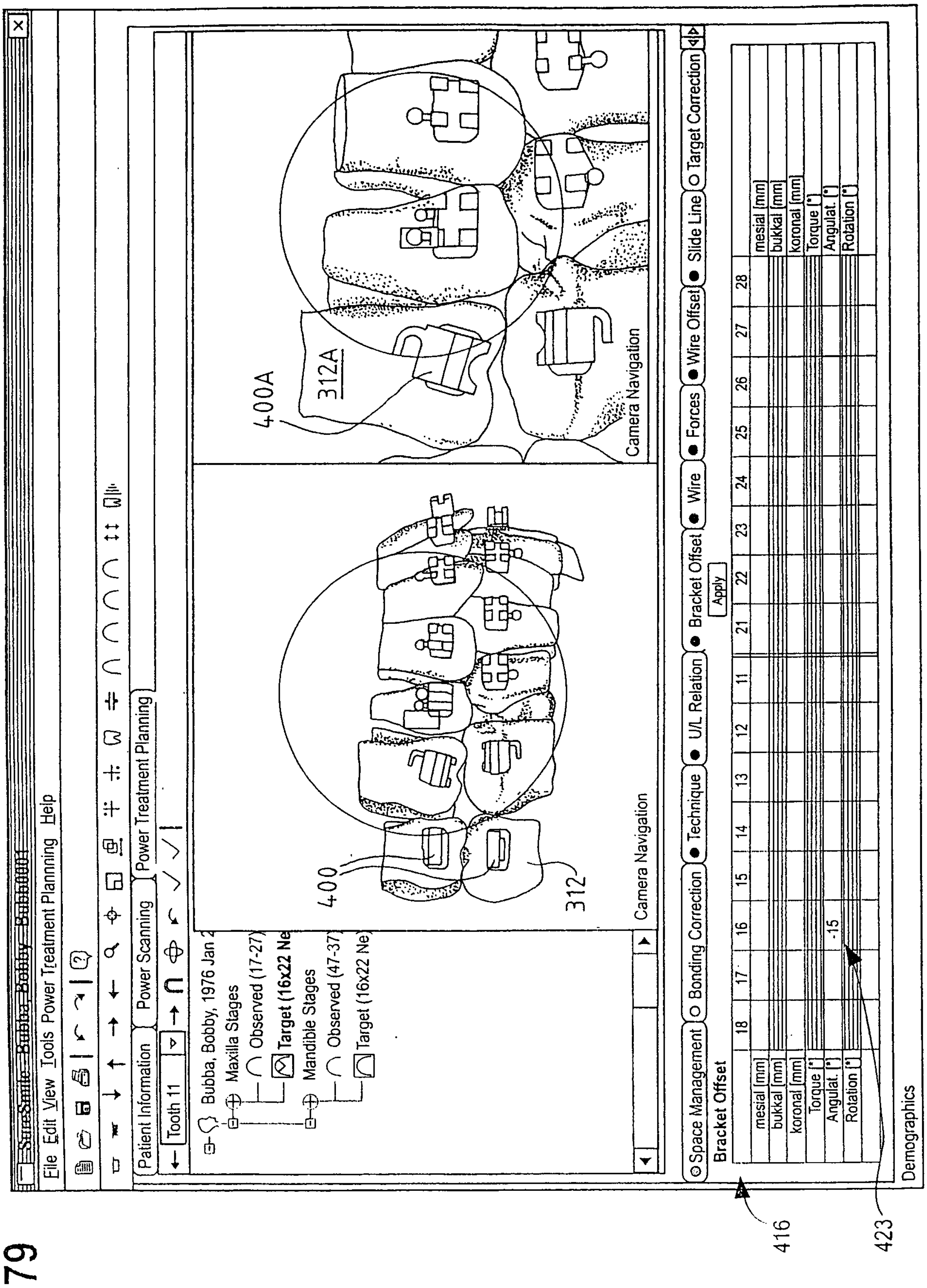
SureSmile - Bubba_Bobby - Bubb0001
File Edit View Tools Power Treatment Plannning Help
Patient Information
Power Scanning
Power Treatment Planning
Tooth 11
Bubba, Bobby, 1976 Jan 2
Maxilla Stages
Observed (17-27)
Target (16x22 Ne
Mandible Stages
Observed (47-37
Target (16x22 Ne
Camera Navigation
Camera Navigation
400
312
400A
312A
Space Management
Bonding Correction
Technique
U/L Relation
Bracket Offset
Wire
Forces
Wire Offset
Slide Line
Target Correction
Apply
Bracket Offset
18
17
16
15
14
13
12
11
21
22
23
24
25
26
27
28
mesial [mm]
bukkal [mm]
koronal [mm]
Torque [°]
Angulat. [°]
Rotation [°]
-15
Demographics
416
423

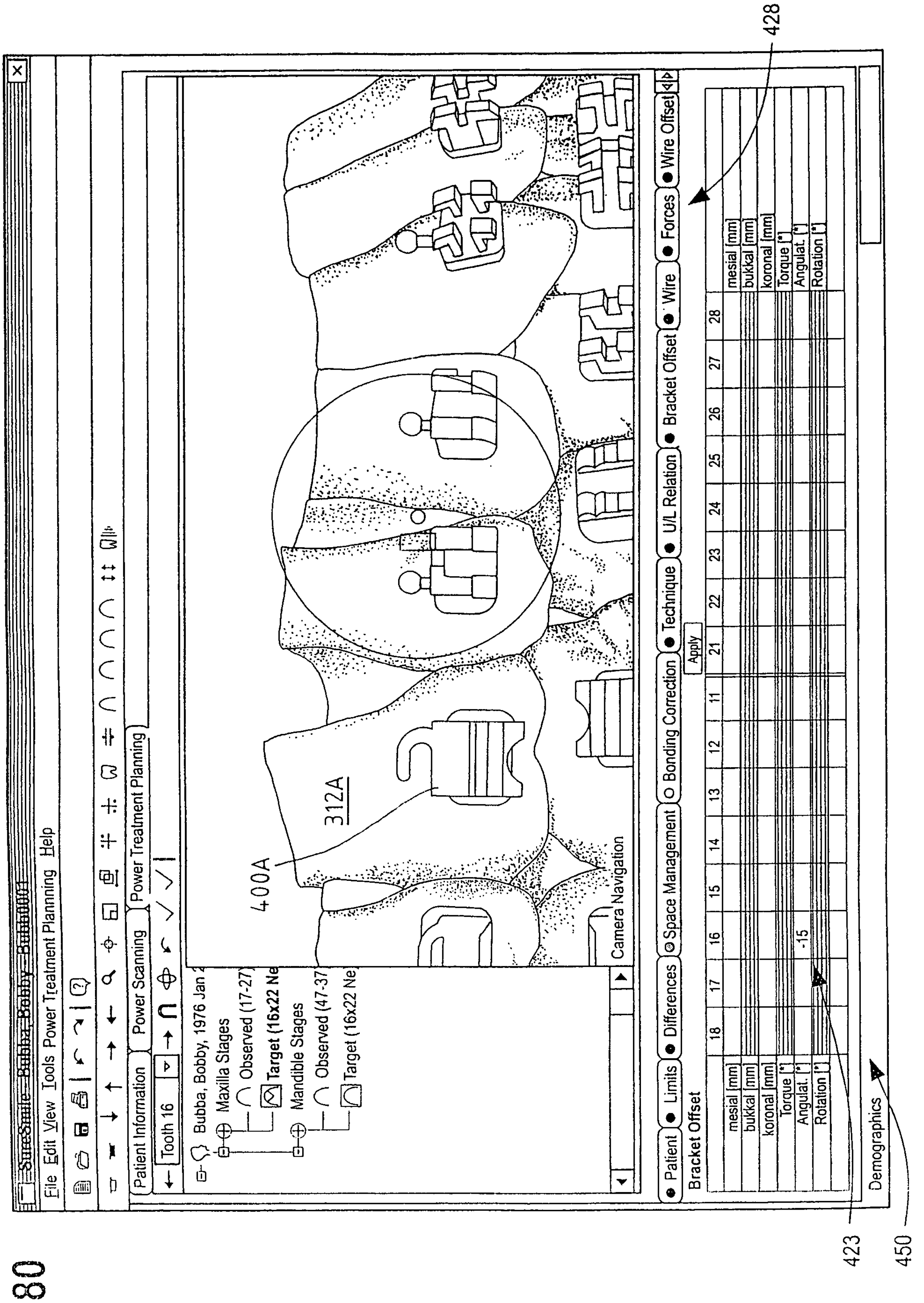
428
400A
312A
423
450
SureSmile - Bubba, Bobby - Bubb0001
File Edit View Tools Power Treatment Plannning Help
Patient Information
Power Scanning
Power Treatment Planning
Tooth 16
Bubba, Bobby, 1976 Jan 2
Maxilla Stages
Observed (17-27)
Target (16x22 Ne
Mandible Stages
Observed (47-37
Target (16x22 Ne
Camera Navigation
Patient
Limits
Differences
Space Management
Bonding Correction
Technique
U/L Relation
Bracket Offset
Wire
Forces
Wire Offset
Bracket Offset
Apply
mesial [mm]
bukkal [mm]
koronal [mm]
Torque [°]
Angulat. [°]
Rotation [°]
18
17
16
15
14
13
12
11
21
22
23
24
25
26
27
28
-15
Demographics

FIG. 80

FIG. 81

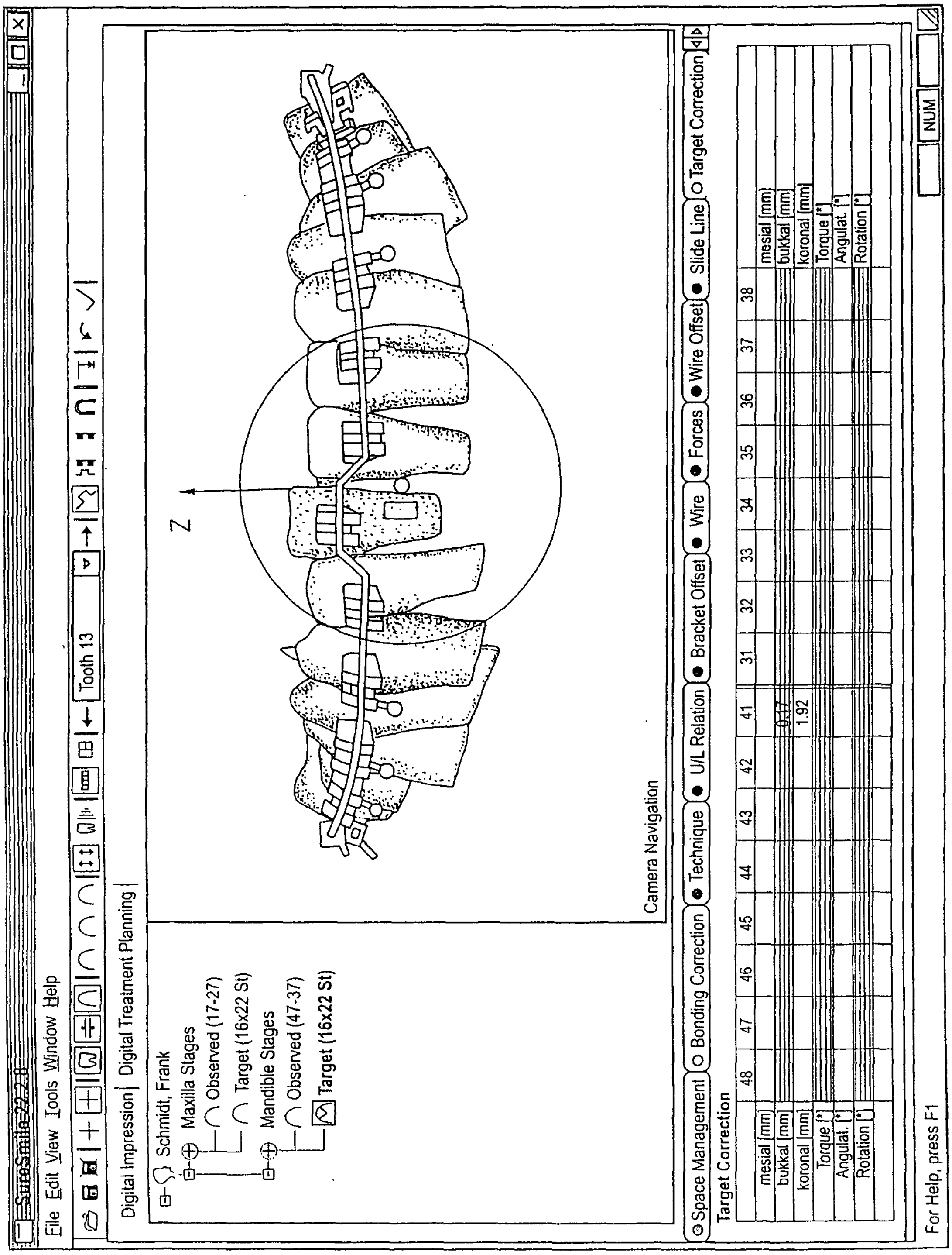
SureSmile 22.2.8
File Edit View Tools Window Help
Tooth 13
Digital Impression
Digital Treatment Planning
Schmidt, Frank
Maxilla Stages
Observed (17-27)
Target (16x22 St)
Mandible Stages
Observed (47-37)
Target (16x22 St)
Z
Camera Navigation
Space Management
Bonding Correction
Technique
U/L Relation
Bracket Offset
Wire
Forces
Wire Offset
Slide Line
Target Correction
48
47
46
45
44
43
42
41
31
32
33
34
35
36
37
38
mesial [mm]
bukkal [mm]
koronal [mm]
Torque [°]
Angulat. [°]
Rotation [°]
0.17
1.92
For Help, press F1
NUM

FIG. 82

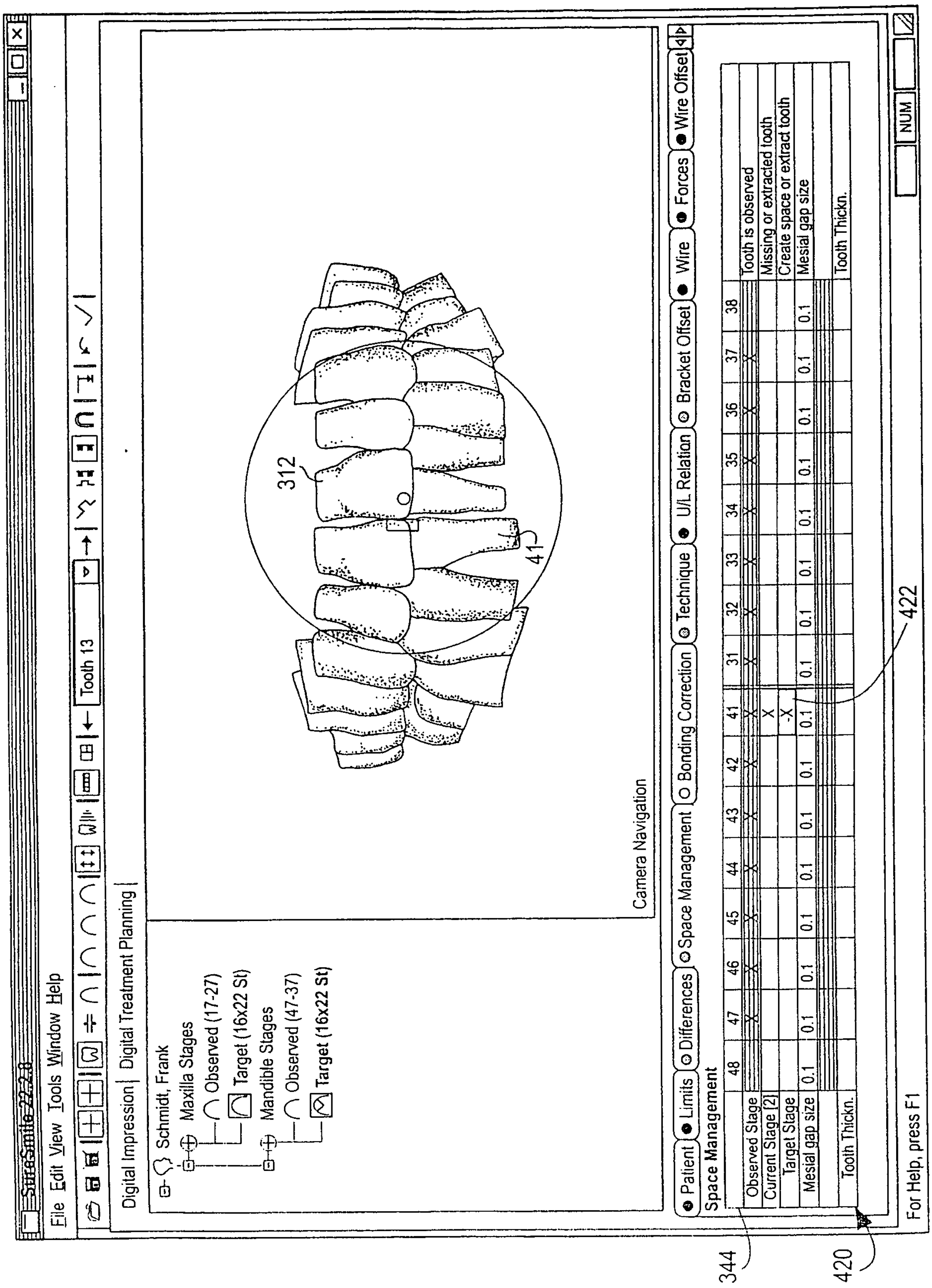
SureSmile 22.2.0
File Edit View Tools Window Help
Tooth 13
Digital Impression
Digital Treatment Planning
Schmidt, Frank
Maxilla Stages
Observed (17-27)
Target (16x22 St)
Mandible Stages
Observed (47-37)
Target (16x22 St)
Camera Navigation
312
41
Patient
Limits
Differences
Space Management
Bonding Correction
Technique
U/L Relation
Bracket Offset
Wire
Forces
Wire Offset
Space Management
Observed Stage
Current Stage [2]
Target Stage
Mesial gap size
Tooth Thickn.
48 47 46 45 44 43 42 41 31 32 33 34 35 36 37 38
Tooth is observed
Missing or extracted tooth
Create space or extract tooth
Mesial gap size
Tooth Thickn.
For Help, press F1
NUM
344
420
422

FIG. 83

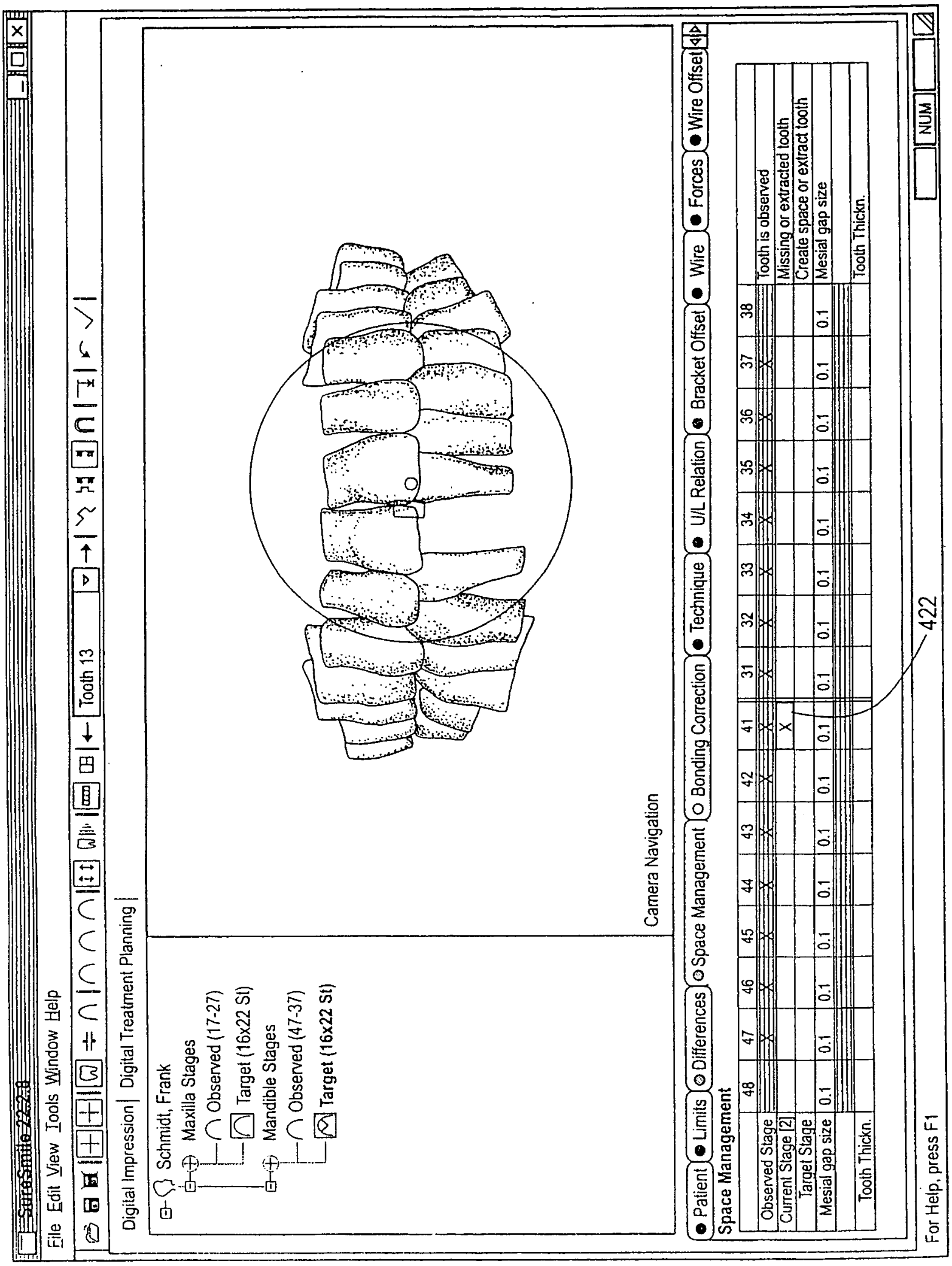

File Edit View Tools Window Help
Tooth 13
Digital Impression
Digital Treatment Planning
Schmidt, Frank
Maxilla Stages
Observed (17-27)
Target (16x22 St)
Mandible Stages
Observed (47-37)
Target (16x22 St)
Camera Navigation
Patient
Limits
Differences
Space Management
Bonding Correction
Technique
U/L Relation
Bracket Offset
Wire
Forces
Wire Offset
Space Management
Observed Stage
Current Stage [2]
Target Stage
Mesial gap size
Tooth Thickn.
Tooth is observed
Missing or extracted tooth
Create space or extract tooth
Mesial gap size
Tooth Thickn.
422
For Help, press F1
NUM

FIG. 84

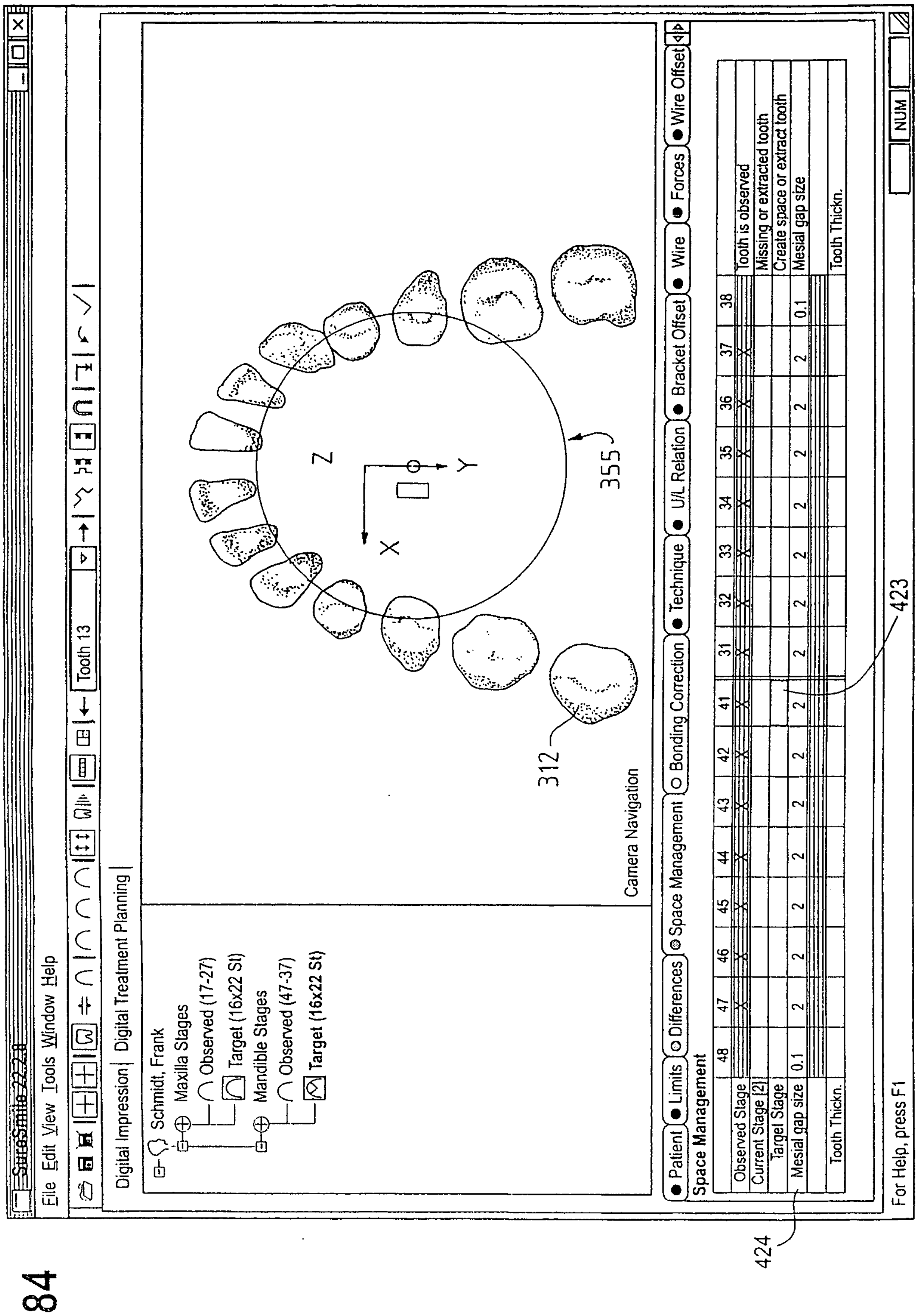
SureSmile 22.2.8
File Edit View Tools Window Help
Tooth 13
Digital Impression | Digital Treatment Planning |
Schmidt, Frank
Maxilla Stages
Observed (17-27)
Target (16x22 St)
Mandible Stages
Observed (47-37)
Target (16x22 St)
Camera Navigation
Z
X
Y
355
312
Patient
Limits
Differences
Space Management
Bonding Correction
Technique
U/L Relation
Bracket Offset
Wire
Forces
Wire Offset
Space Management
48 47 46 45 44 43 42 41 31 32 33 34 35 36 37 38
Observed Stage
Current Stage [2]
Target Stage
Mesial gap size
Tooth Thickn.
Tooth is observed
Missing or extracted tooth
Create space or extract tooth
Mesial gap size
Tooth Thickn.
For Help, press F1
NUM
423
424

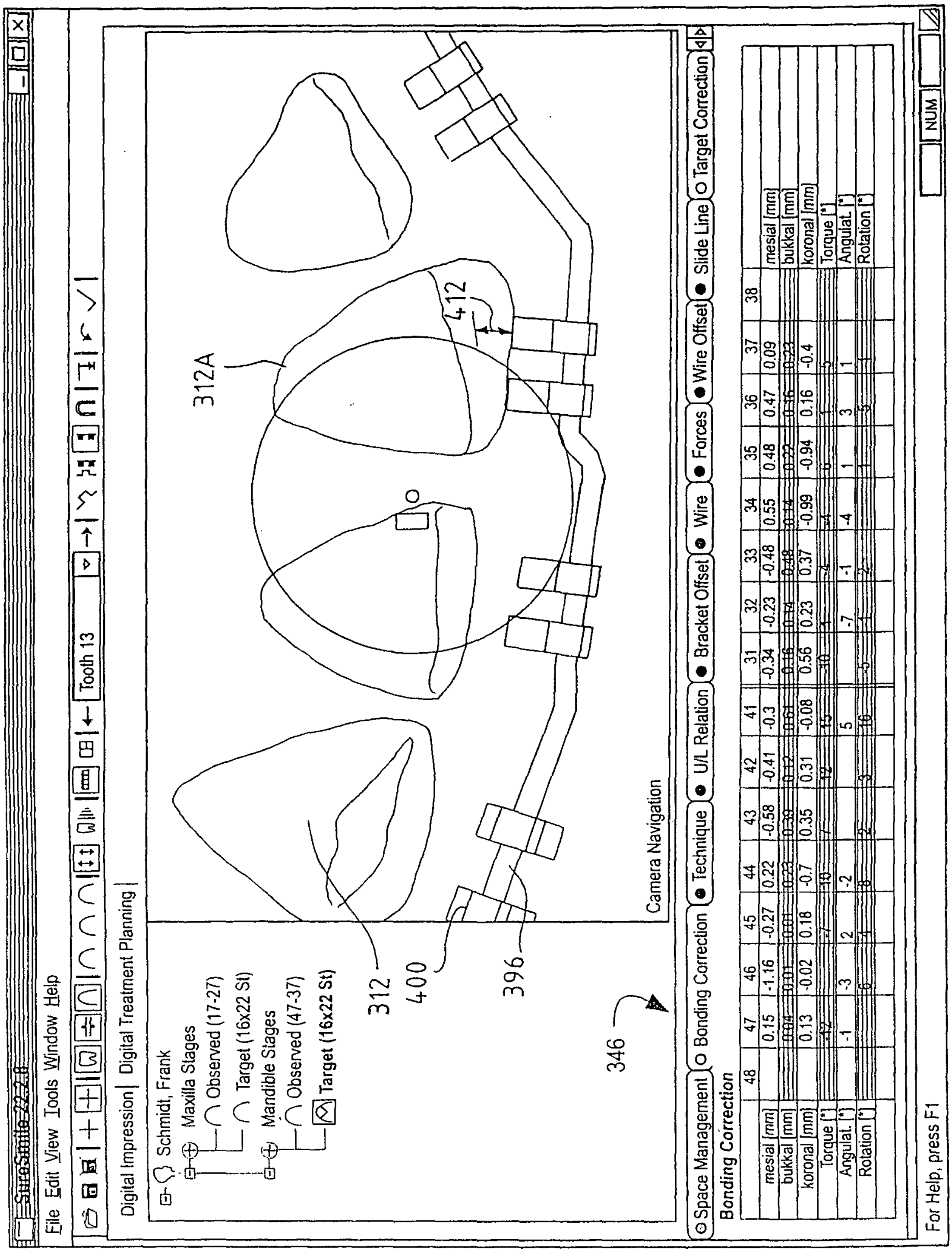
File Edit View Tools Window Help
Tooth 13
Digital Impression
Digital Treatment Planning
Schmidt, Frank
Maxilla Stages
Observed (17-27)
Target (16x22 St)
Mandible Stages
Observed (47-37)
Target (16x22 St)
Camera Navigation
312A
412
312
400
396
346
Space Management
Bonding Correction
Technique
U/L Relation
Bracket Offset
Wire
Forces
Wire Offset
Slide Line
Target Correction
Bonding Correction
48 47 46 45 44 43 42 41 31 32 33 34 35 36 37 38
mesial [mm]
bukkal [mm]
koronal [mm]
Torque [°]
Angulat. [°]
Rotation [°]
For Help, press F1
NUM

FIG. 85

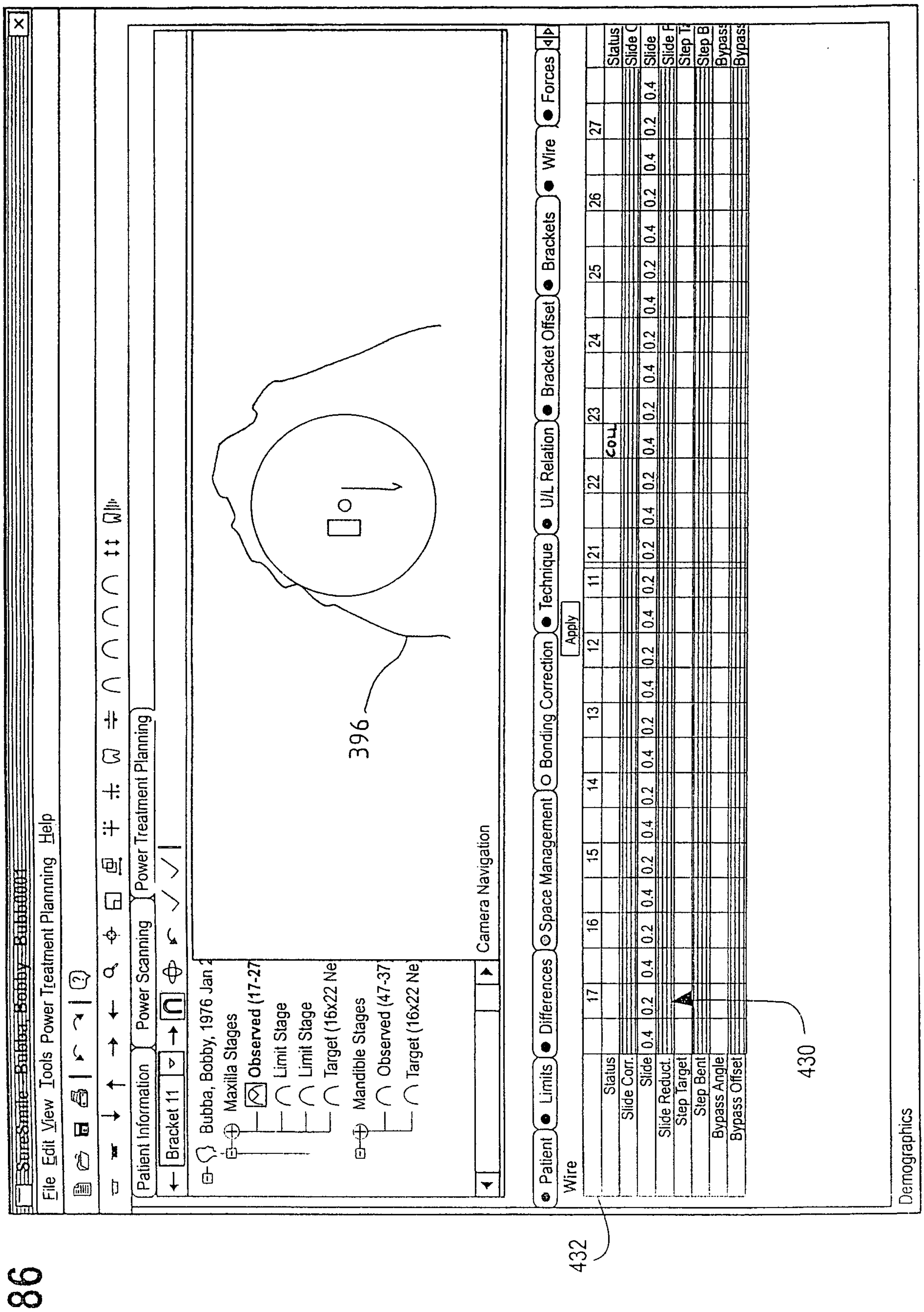
SureSmile - Bubba, Bobby - Bubb0001
File Edit View Tools Power Treatment Planning Help
Patient Information
Power Scanning
Power Treatment Planning
Bracket 11
Bubba, Bobby, 1976 Jan 2
Maxilla Stages
Observed (17-27
Limit Stage
Limit Stage
Target (16x22 Ne
Mandible Stages
Observed (47-37
Target (16x22 Ne
Camera Navigation
396
Patient
Limits
Differences
Space Management
Bonding Correction
Technique
U/L Relation
Bracket Offset
Brackets
Wire
Forces
Apply
Wire
Status
Slide Corr.
Slide
Slide Reduct.
Step Target
Step Bent
Bypass Angle
Bypass Offset
17 16 15 14 13 12 11 21 22 23 24 25 26 27
COLL
430
432
Demographics

FIG. 86

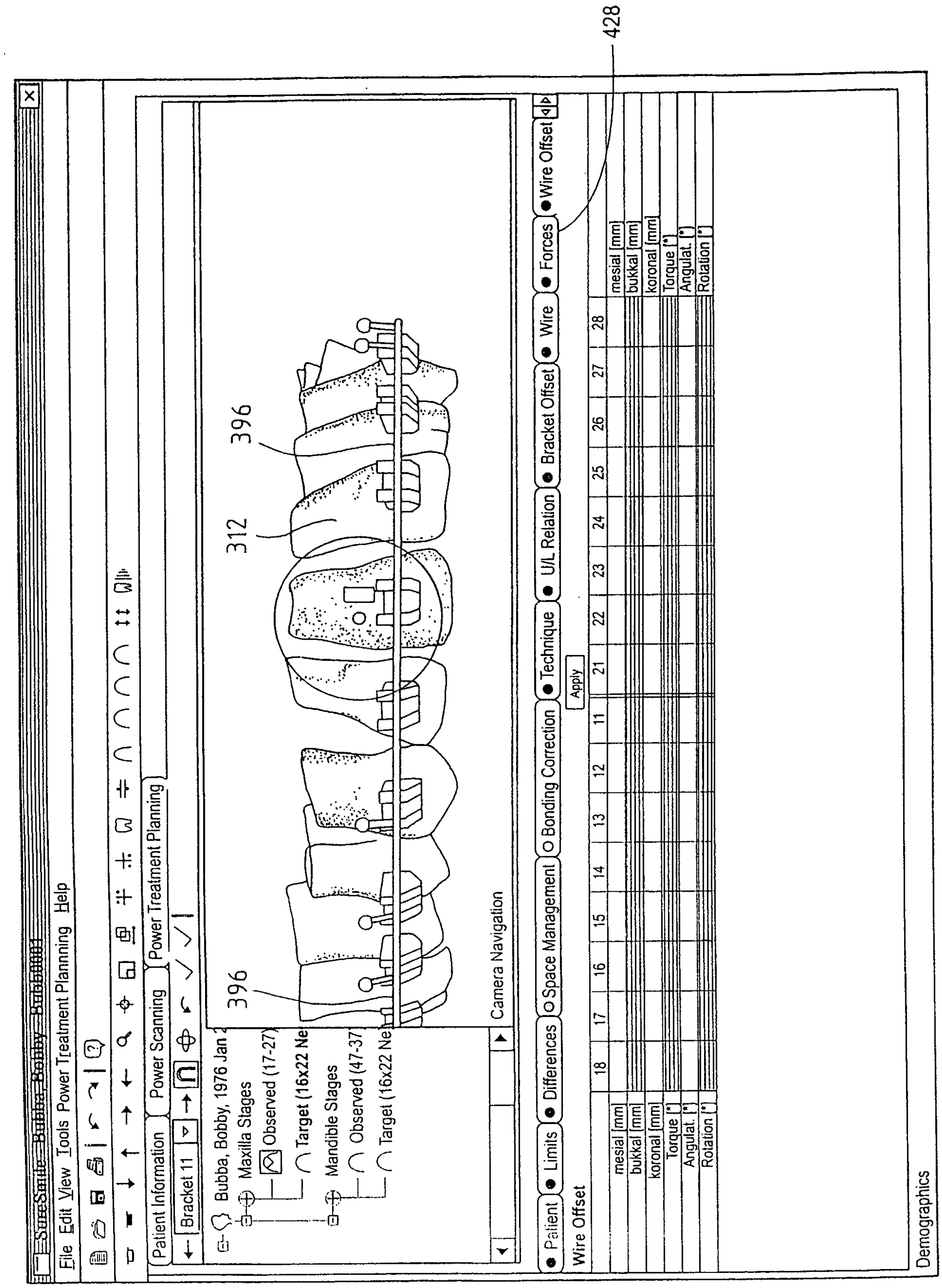
428
396
312
396
File Edit View Tools Power Treatment Plannning Help
Patient Information
Power Scanning
Power Treatment Planning
Bracket 11
Bubba, Bobby, 1976 Jan 2
Maxilla Stages
Observed (17-27)
Target (16x22 Ne
Mandible Stages
Observed (47-37
Target (16x22 Ne
Camera Navigation
Patient
Limits
Differences
Space Management
Bonding Correction
Technique
U/L Relation
Bracket Offset
Wire
Forces
Wire Offset
Apply
Wire Offset
18 17 16 15 14 13 12 11 21 22 23 24 25 26 27 28
mesial [mm]
bukkal [mm]
koronal [mm]
Torque [°]
Angulat. [°]
Rotation [°]
Demographics

FIG. 87

FIG. 88

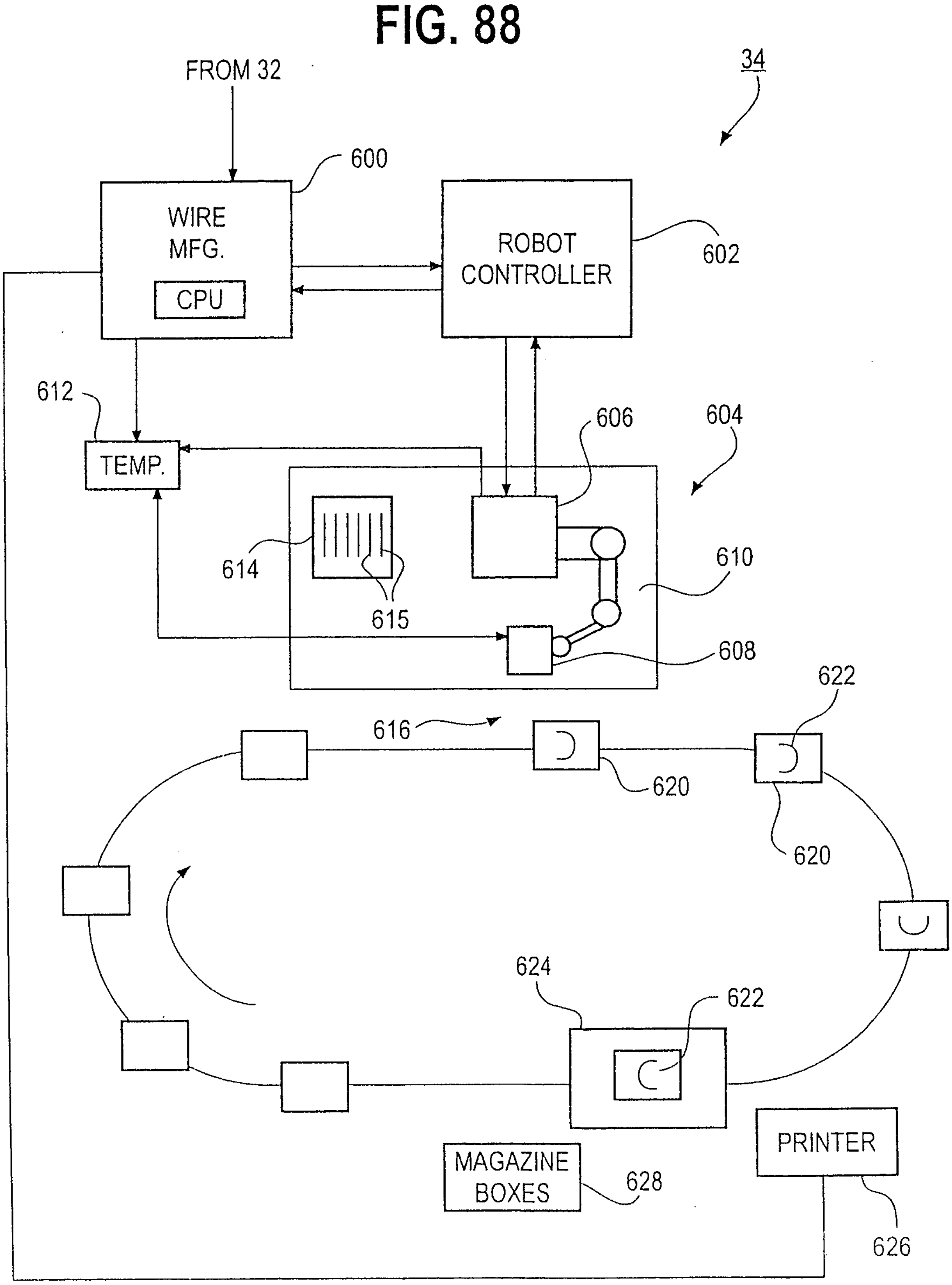
FROM 32
34
600
WIRE
MFG.
CPU
ROBOT
CONTROLLER
602
612
TEMP.
606
604
614
615
610
608
622
616
620
620
624
622
PRINTER
MAGAZINE
BOXES
628
626

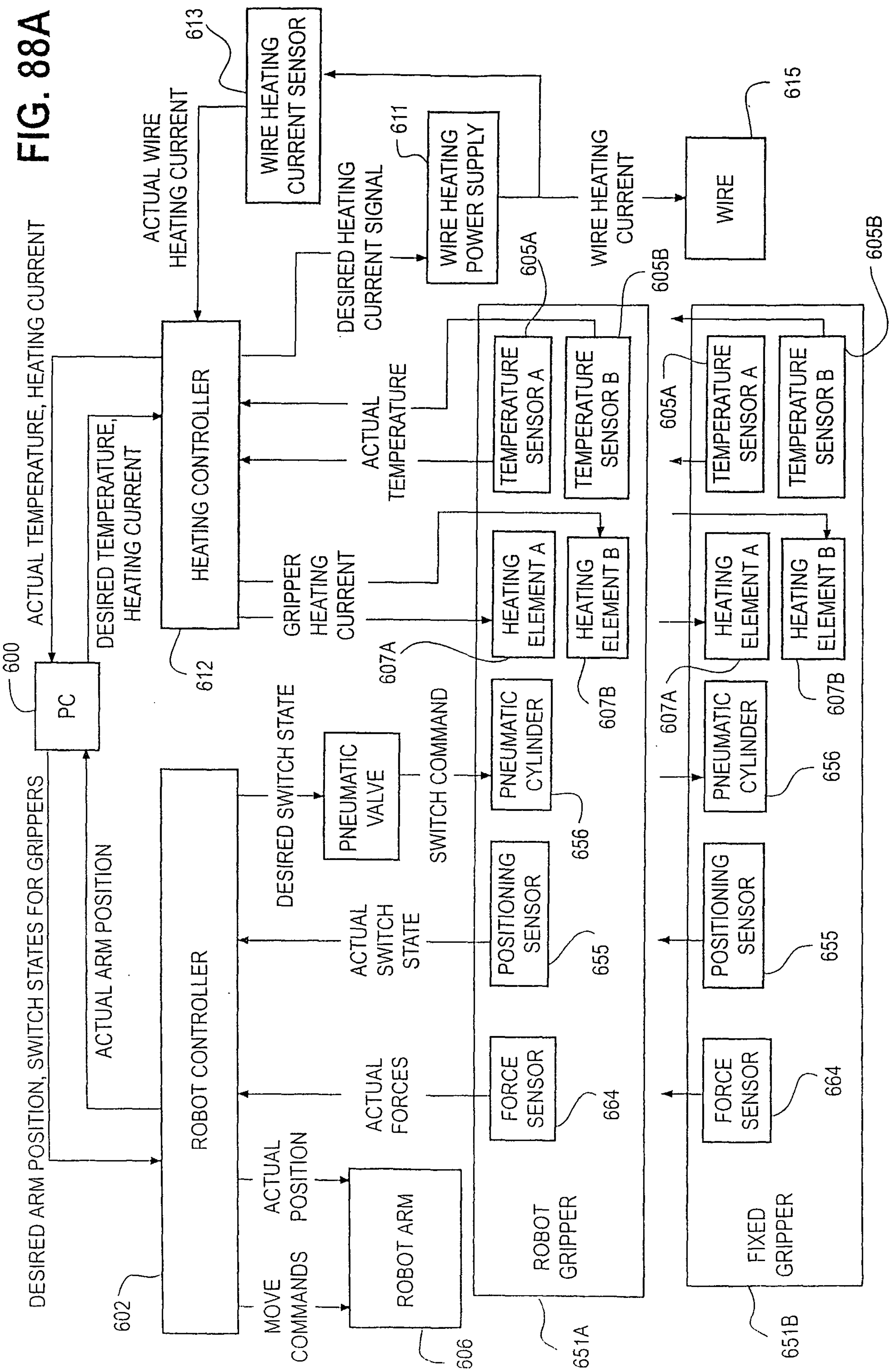

DESIRED ARM POSITION, SWITCH STATES FOR GRIPPERS
ACTUAL TEMPERATURE, HEATING CURRENT
600
PC
ACTUAL ARM POSITION
DESIRED TEMPERATURE, HEATING CURRENT
ACTUAL WIRE HEATING CURRENT
613
WIRE HEATING CURRENT SENSOR
602
ROBOT CONTROLLER
612
HEATING CONTROLLER
MOVE COMMANDS
ACTUAL POSITION
ROBOT ARM
606
ACTUAL FORCES
ACTUAL SWITCH STATE
DESIRED SWITCH STATE
PNEUMATIC VALVE
SWITCH COMMAND
GRIPPER HEATING CURRENT
ACTUAL TEMPERATURE
DESIRED HEATING CURRENT SIGNAL
611
WIRE HEATING POWER SUPPLY
WIRE HEATING CURRENT
WIRE
615
FORCE SENSOR
664
POSITIONING SENSOR
655
PNEUMATIC CYLINDER
656
HEATING ELEMENT A
607A
HEATING ELEMENT B
607B
TEMPERATURE SENSOR A
605A
TEMPERATURE SENSOR B
605B
ROBOT GRIPPER
651A
FIXED GRIPPER
651B

FIG. 88A

Fig. 89

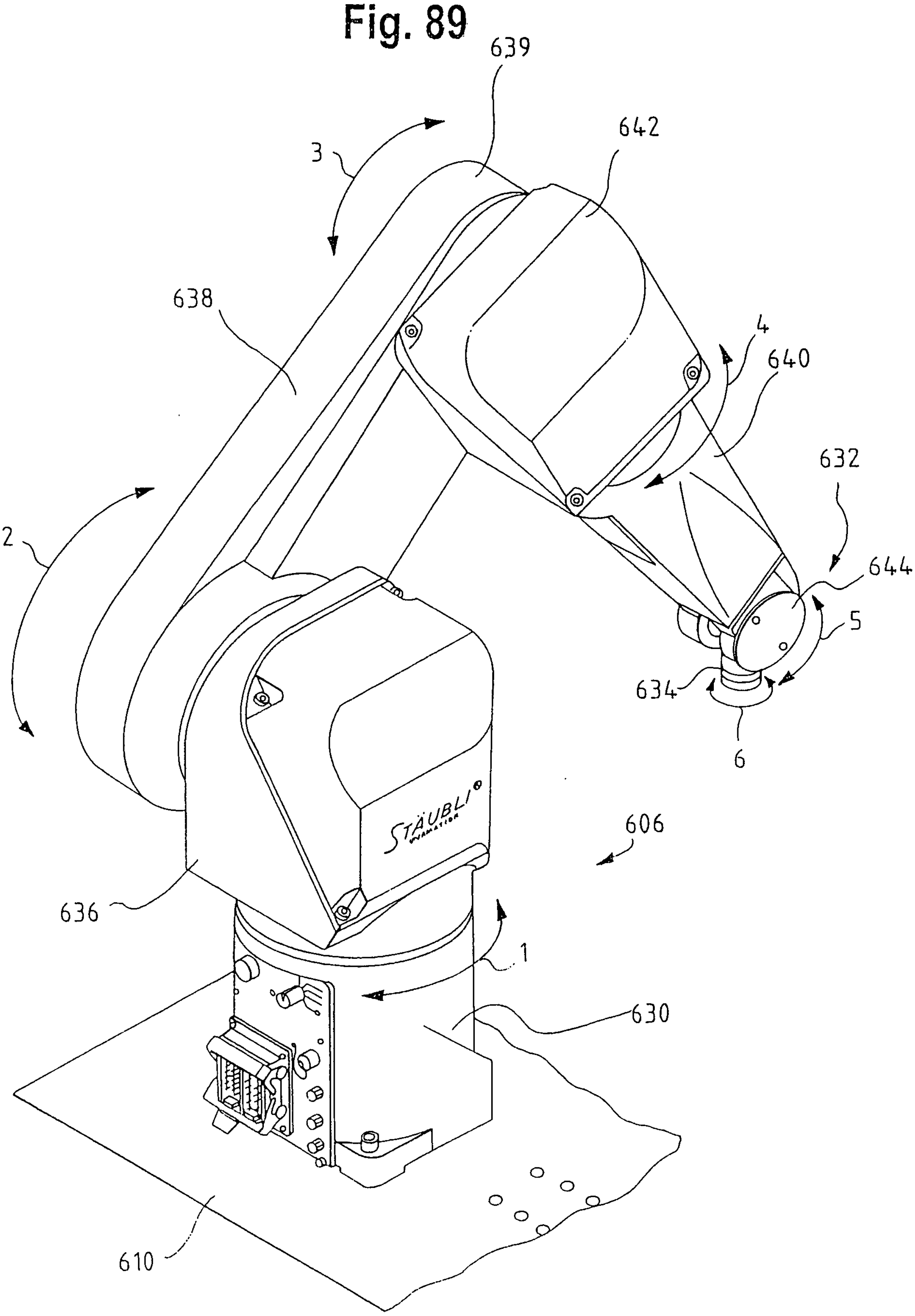

Fig. 90

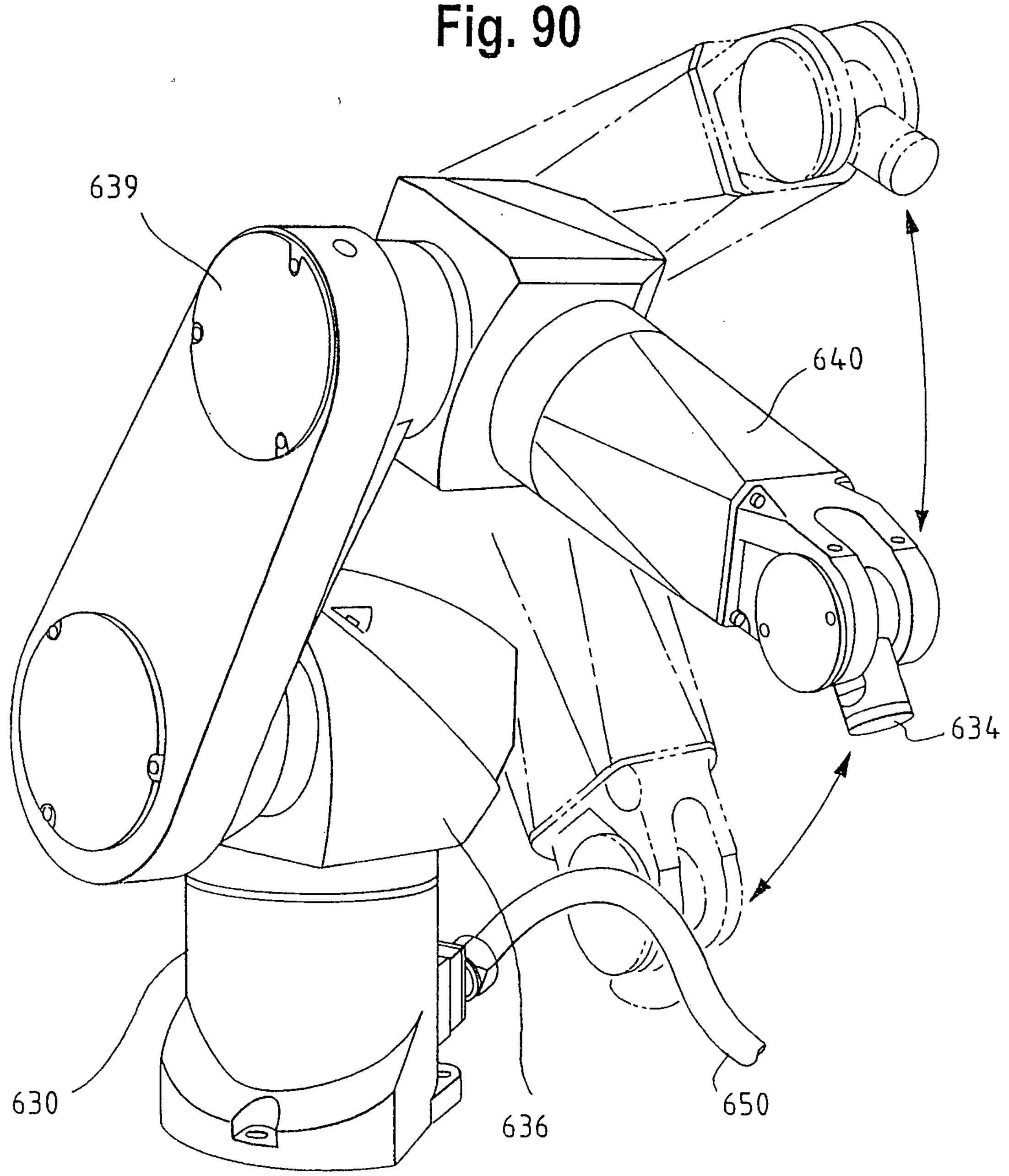

Fig. 91

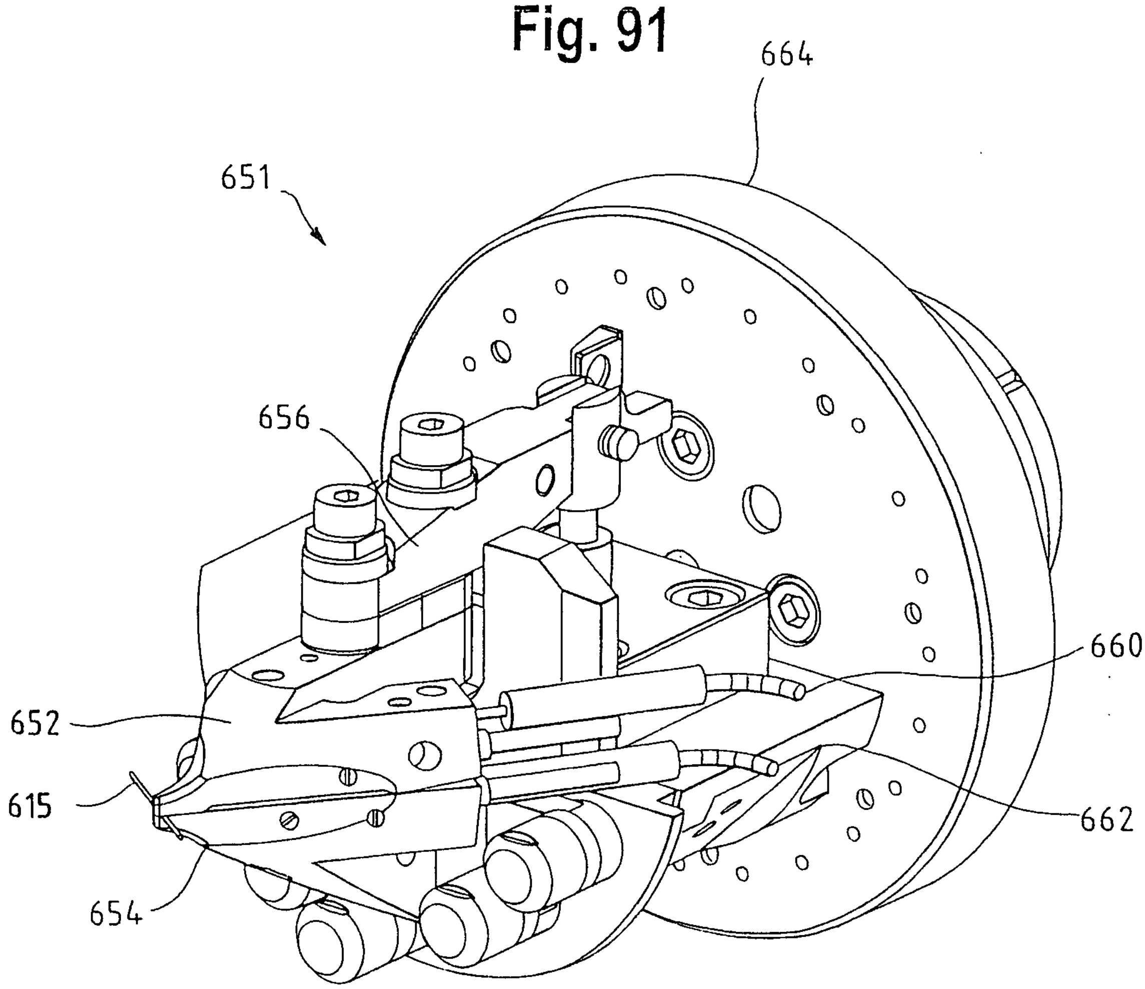

Fig. 92

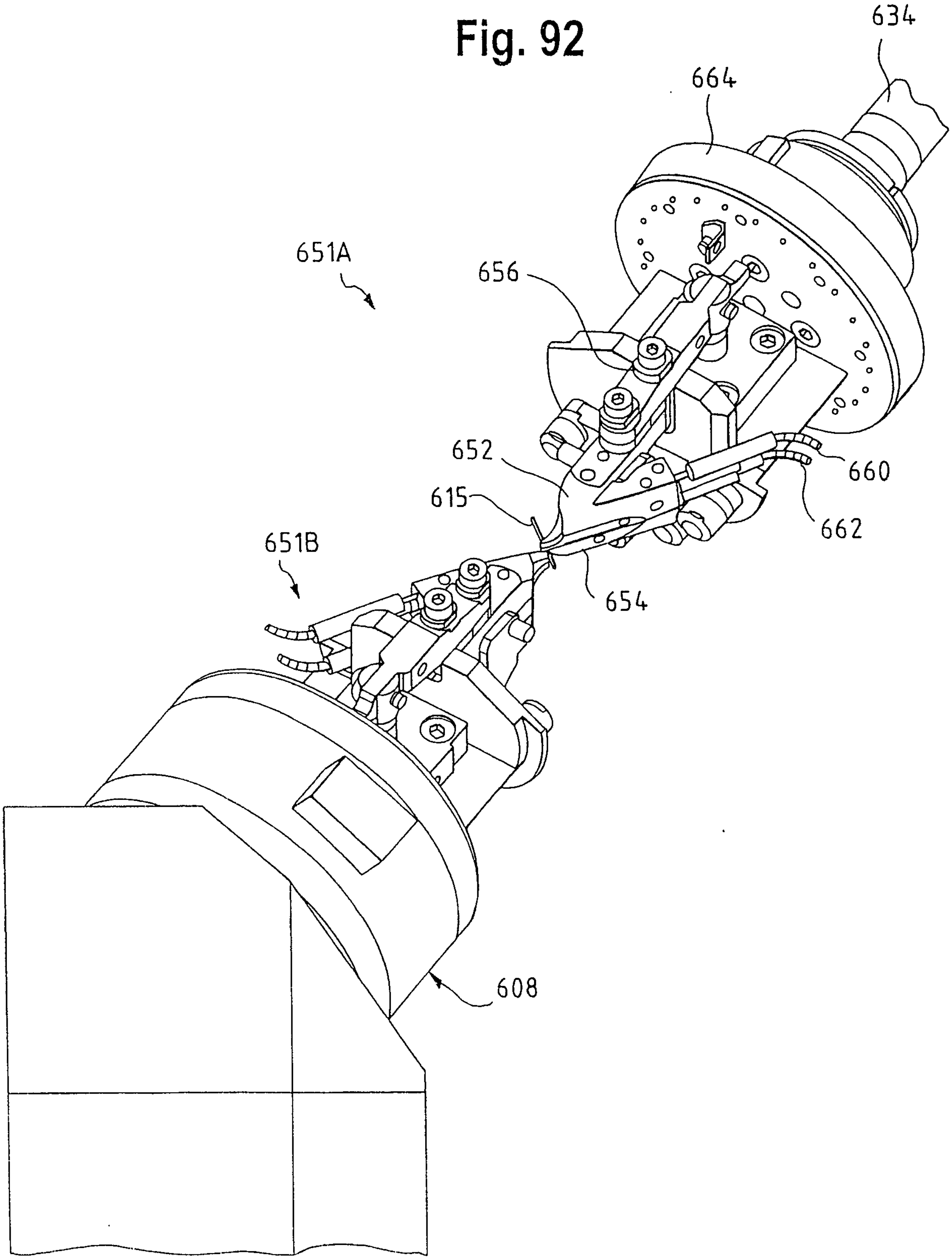

FIG. 93

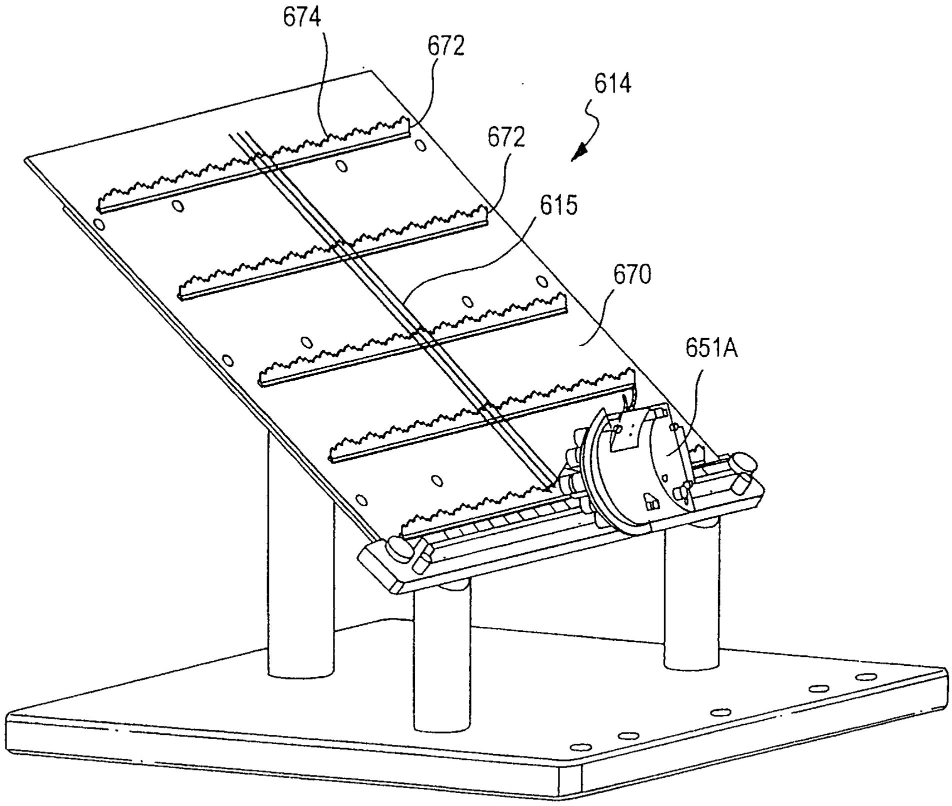

FIG. 94

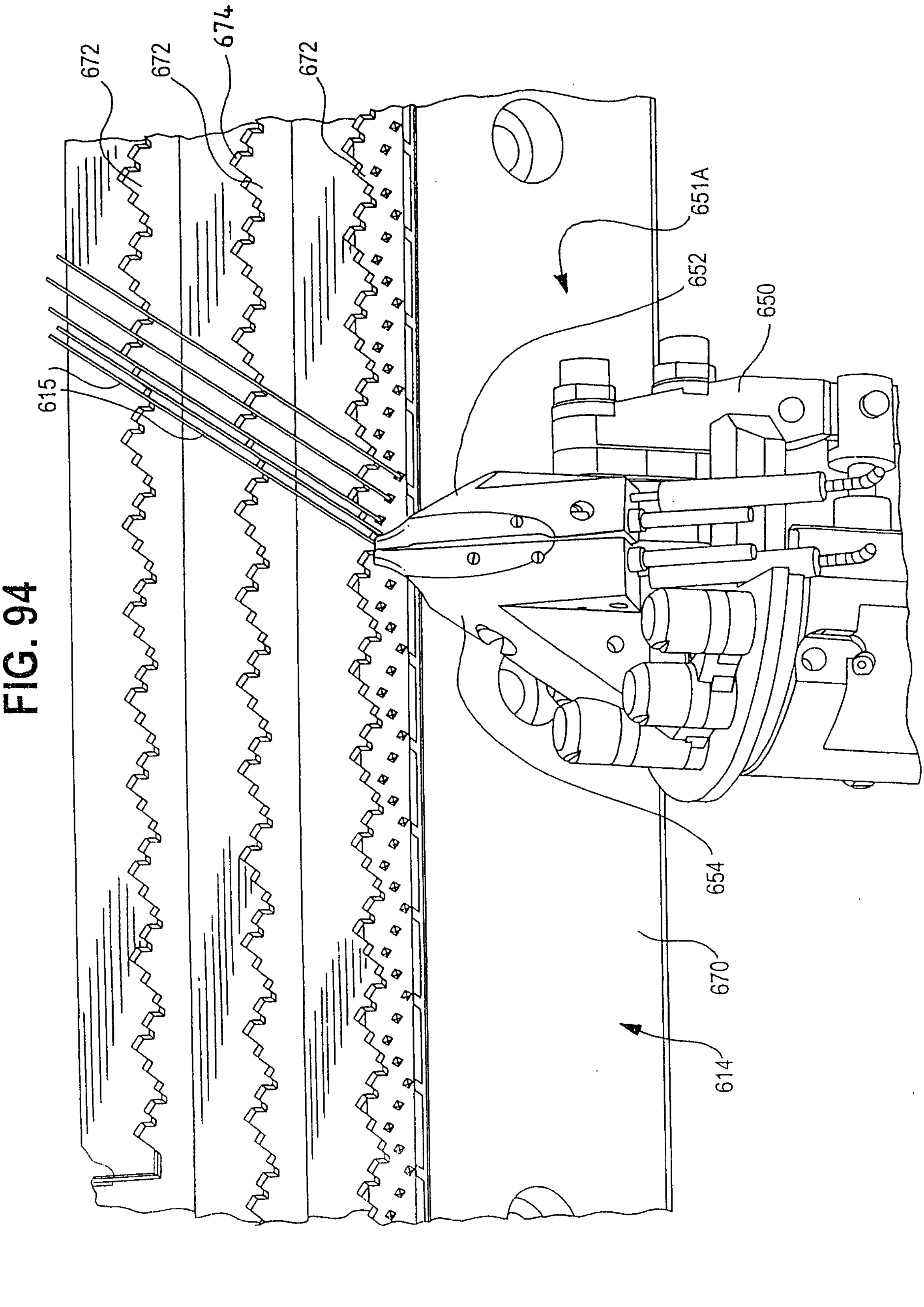

FIG. 95

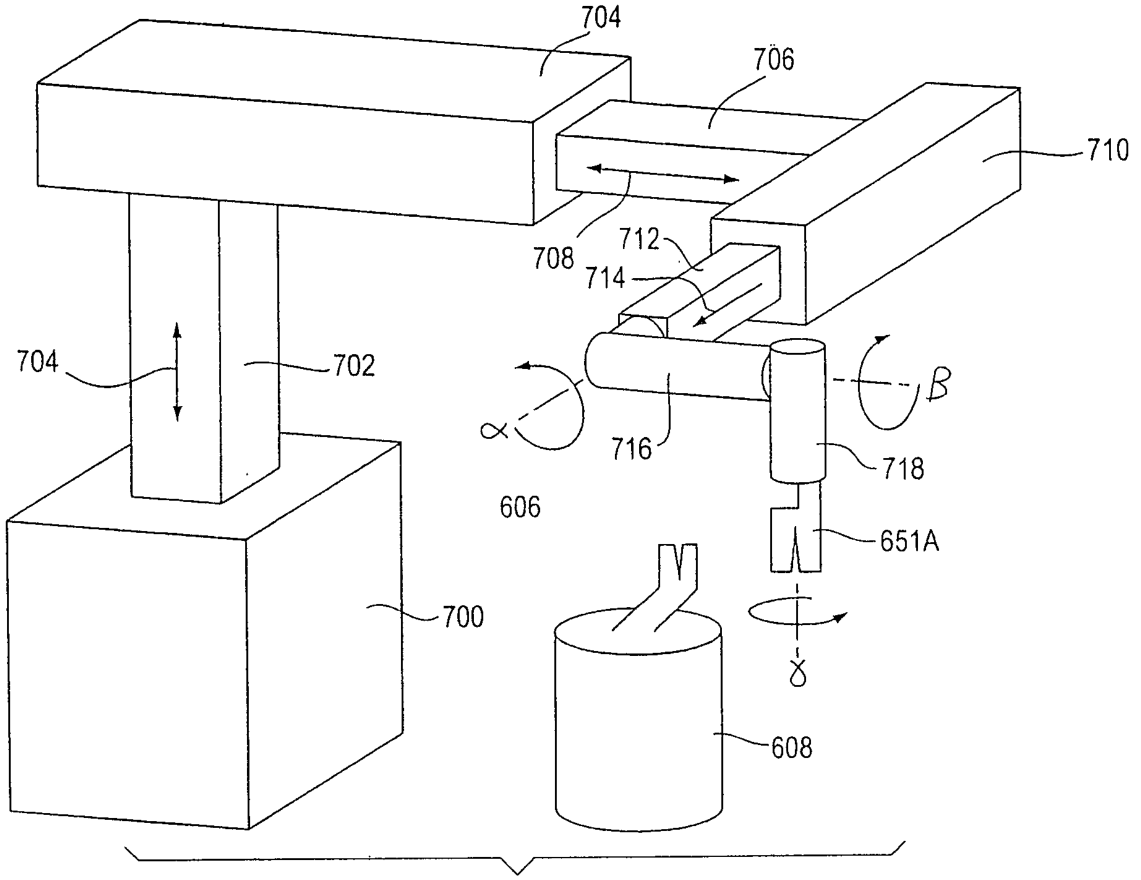


FIG. 96A

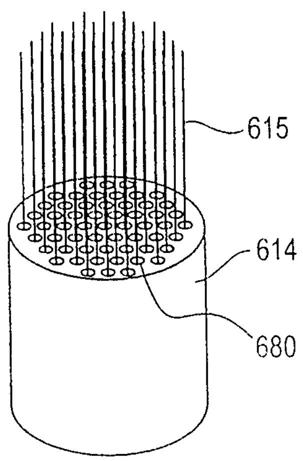


FIG. 96B

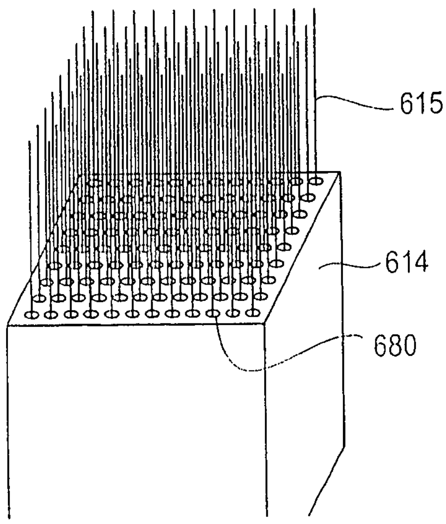

FIG. 97

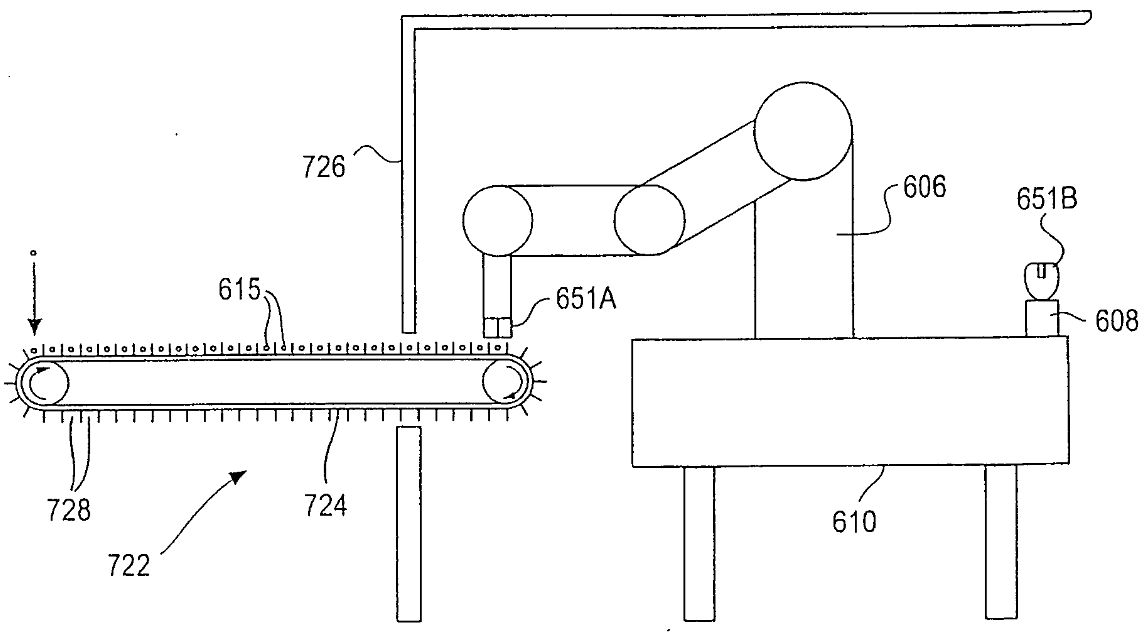

FIG. 98

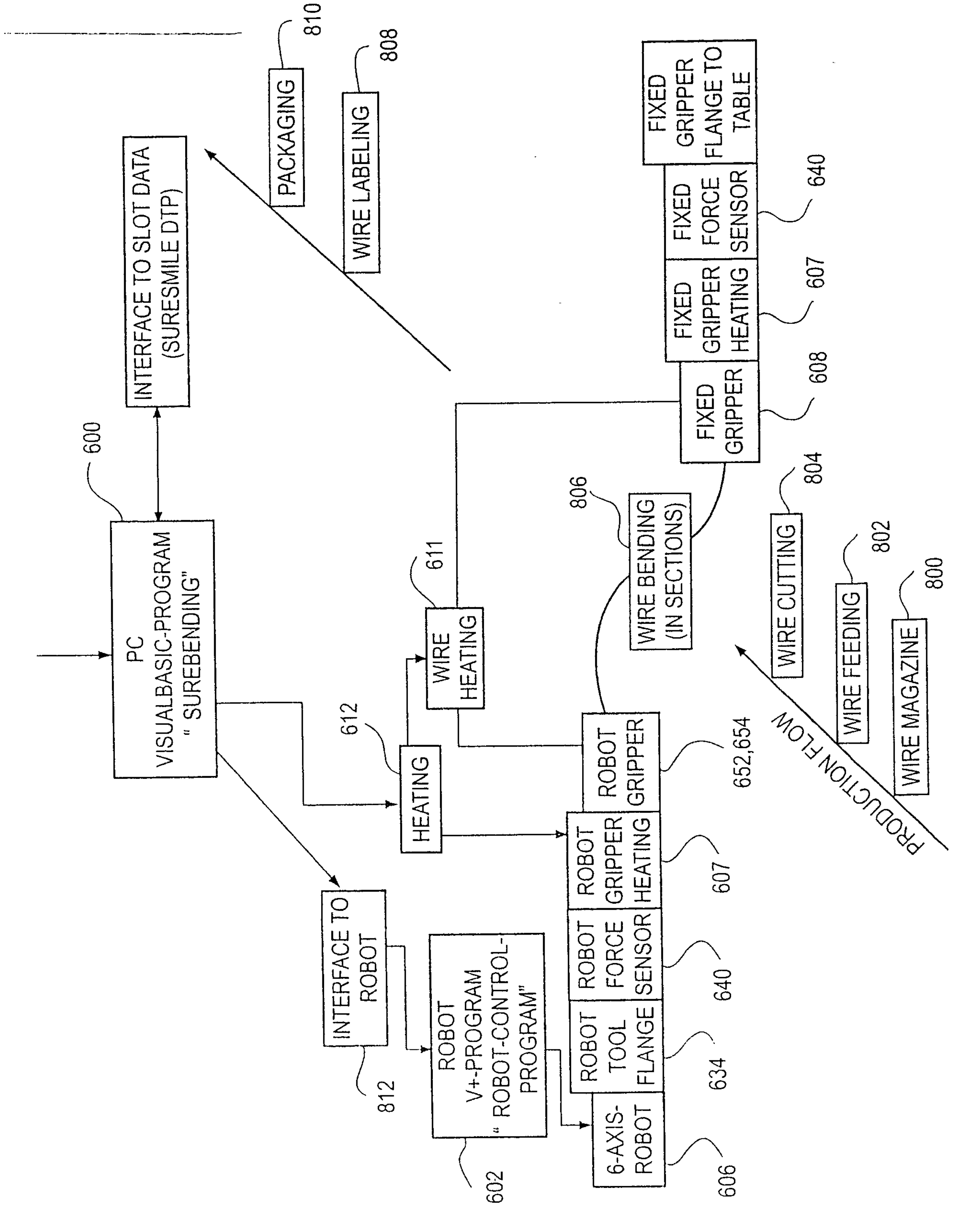
PC
VISUALBASIC-PROGRAM
" SUREBENDING"
600
INTERFACE TO SLOT DATA
(SURESMILE DTP)
PACKAGING
810
WIRE LABELING
808
INTERFACE TO
ROBOT
812
ROBOT
V+-PROGRAM
" ROBOT-CONTROL-
PROGRAM"
602
HEATING
612
WIRE
HEATING
611
6-AXIS-
ROBOT
606
ROBOT
TOOL
FLANGE
634
ROBOT
FORCE
SENSOR
640
ROBOT
GRIPPER
HEATING
607
ROBOT
GRIPPER
652,654
WIRE BENDING
(IN SECTIONS)
806
FIXED
GRIPPER
608
FIXED
GRIPPER
HEATING
607
FIXED
FORCE
SENSOR
640
FIXED
GRIPPER
FLANGE TO
TABLE
WIRE CUTTING
804
WIRE FEEDING
802
WIRE MAGAZINE
800
PRODUCTION FLOW

FIG. 99

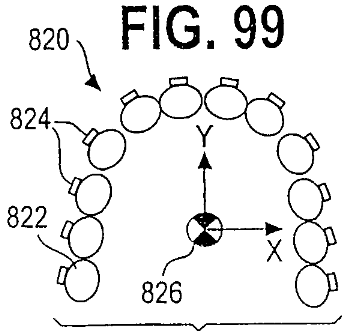


FIG. 100

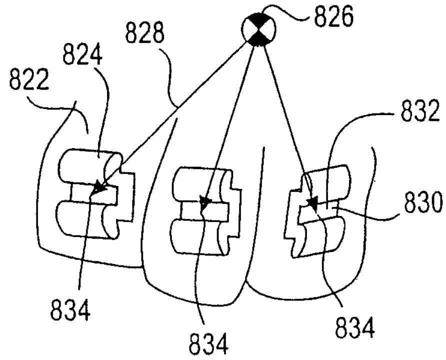


FIG. 101

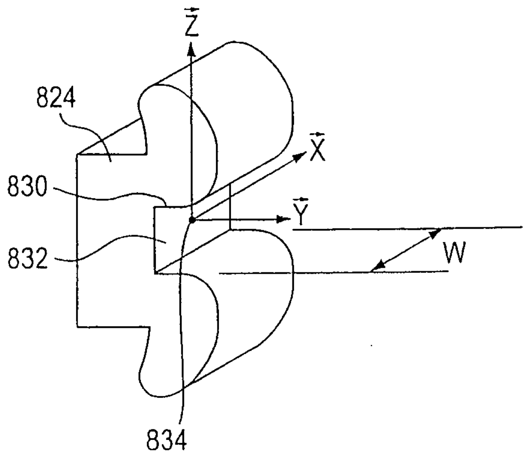


FIG. 102

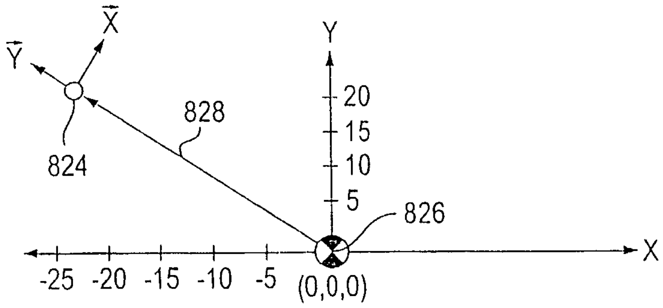


FIG. 103

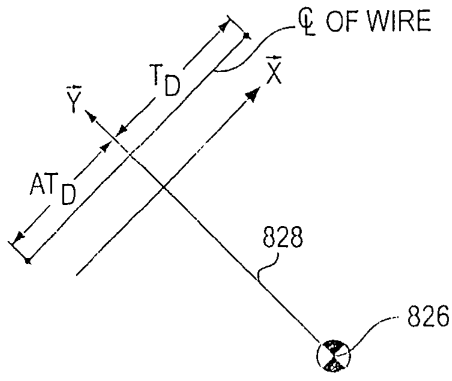

FIG. 104

$$\begin{bmatrix} 0.4 & -0.7 & 0 & -22 \\ 0.9 & 0.7 & 0 & 21 \\ 0 & 0.14 & 0 & 2 \\ 0 & 0 & 0 & 1 \end{bmatrix}$$

FIG. 105

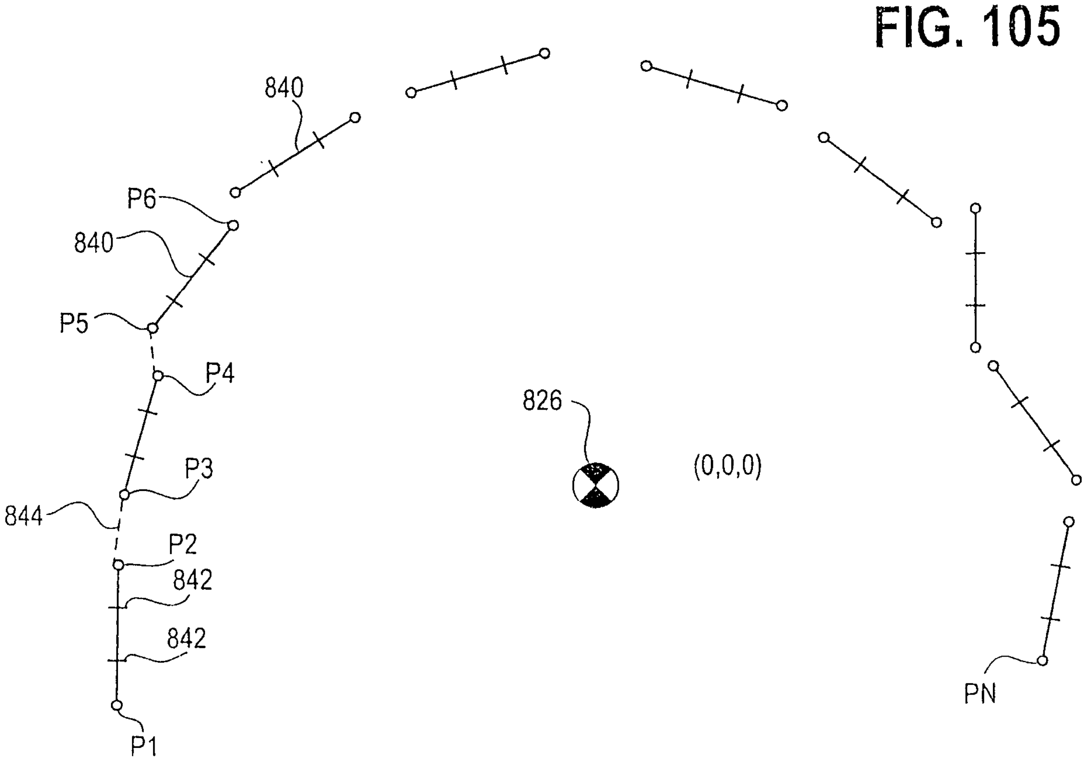


FIG. 106

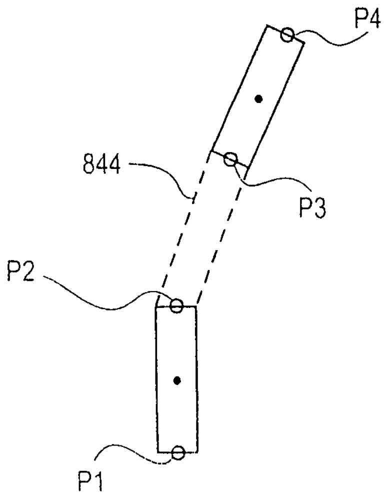

FIG. 106A

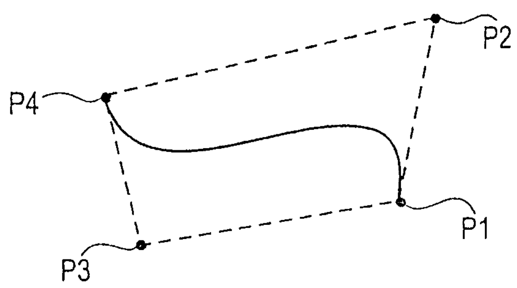


FIG. 106B

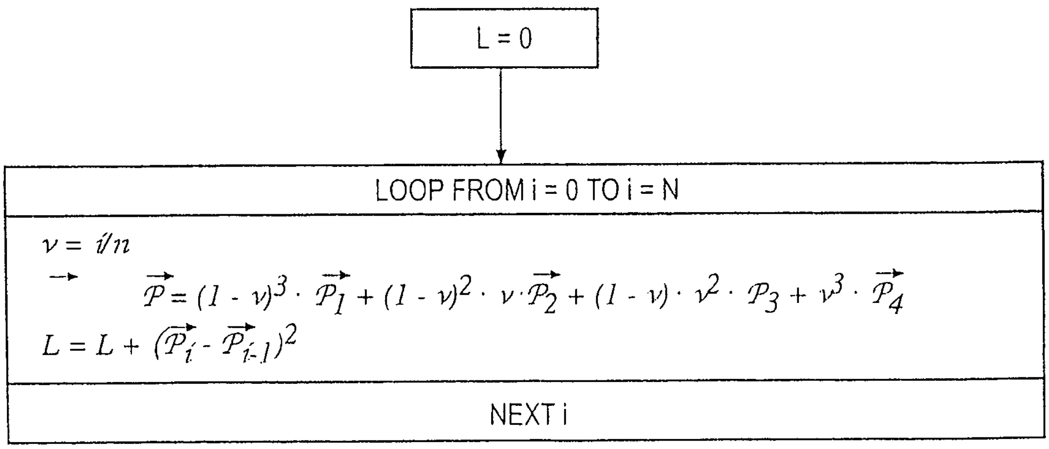

FIG. 107

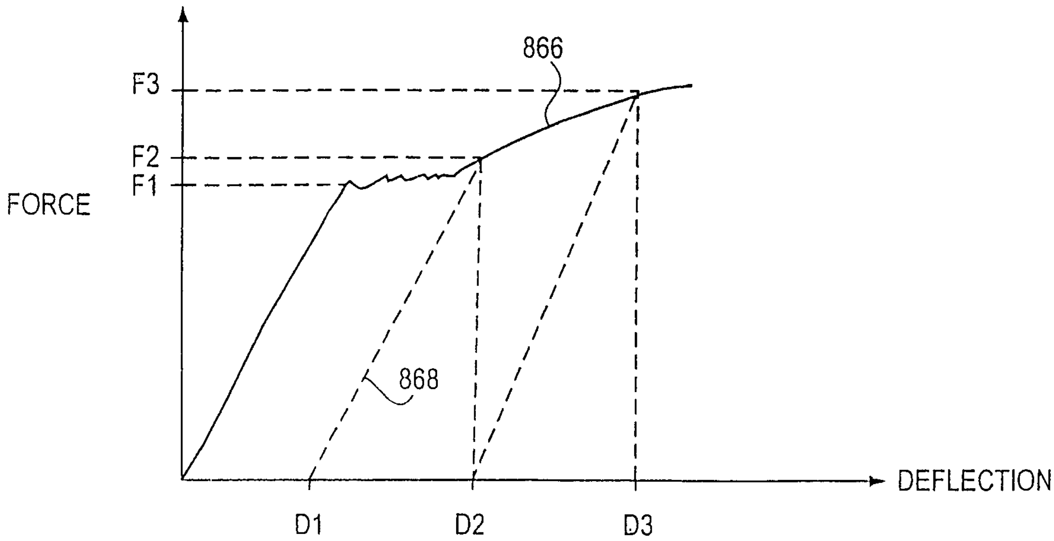


FIG. 108

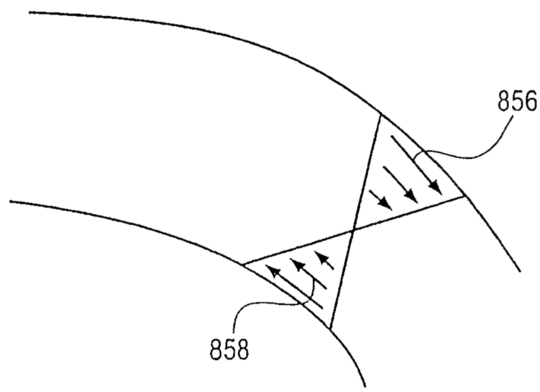


FIG. 109

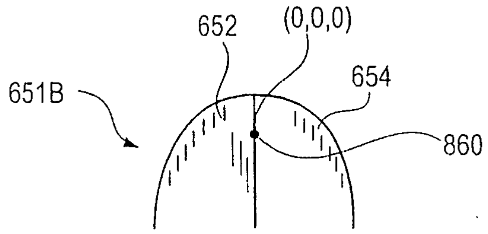


FIG. 110

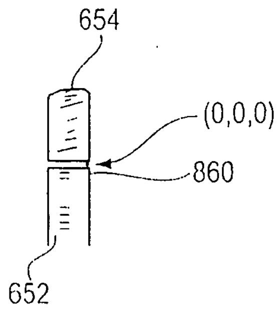

FIG. 111

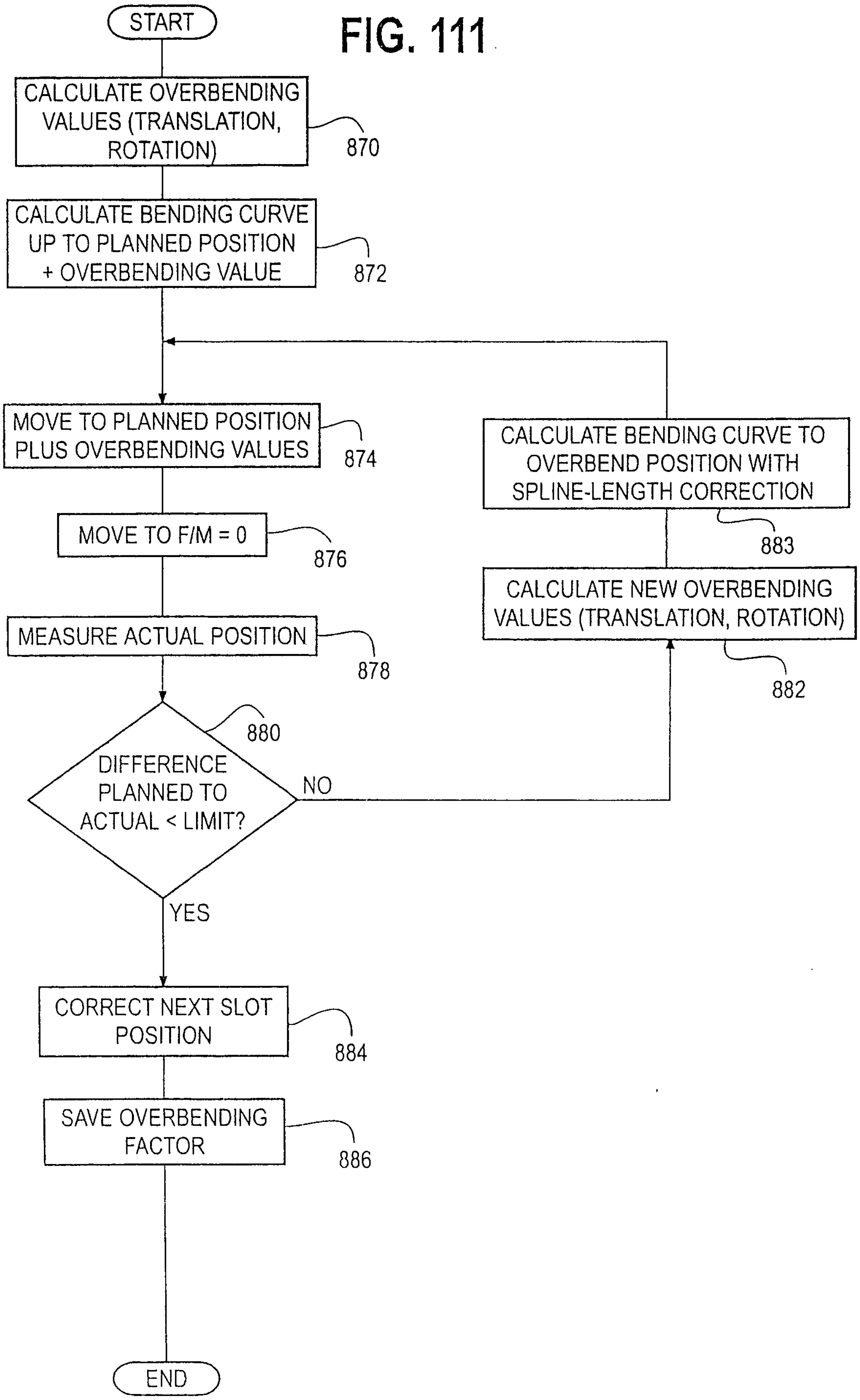
START
CALCULATE OVERBENDING VALUES (TRANSLATION, ROTATION)
870
CALCULATE BENDING CURVE UP TO PLANNED POSITION + OVERBENDING VALUE
872
MOVE TO PLANNED POSITION PLUS OVERBENDING VALUES
874
CALCULATE BENDING CURVE TO OVERBEND POSITION WITH SPLINE-LENGTH CORRECTION
883
MOVE TO F/M = 0
876
CALCULATE NEW OVERBENDING VALUES (TRANSLATION, ROTATION)
MEASURE ACTUAL POSITION
878
882
880
DIFFERENCE PLANNED TO ACTUAL < LIMIT?
NO
YES
CORRECT NEXT SLOT POSITION
884
SAVE OVERBENDING FACTOR
886
END

FIG. 112

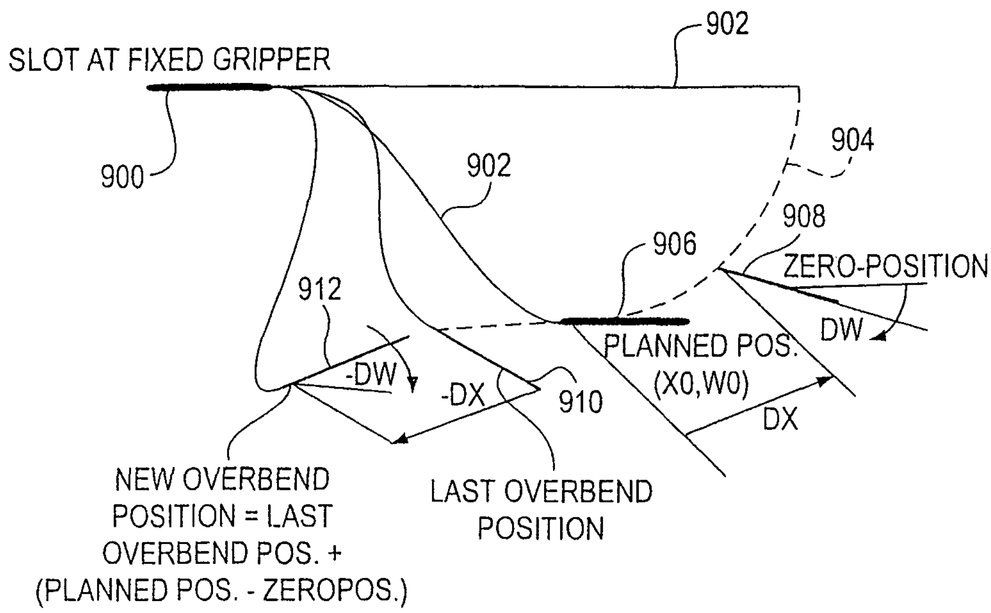
902
SLOT AT FIXED GRIPPER
904
900
902
908
906
ZERO-POSITION
912
DW
PLANNED POS.
-DW
(X0,W0)
-DX
910
DX
NEW OVERBEND
POSITION = LAST
OVERBEND POS. +
(PLANNED POS. - ZEROPOS.)
LAST OVERBEND
POSITION

FIG. 113A

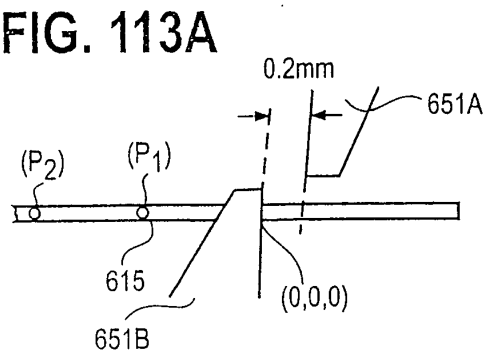


FIG. 113B

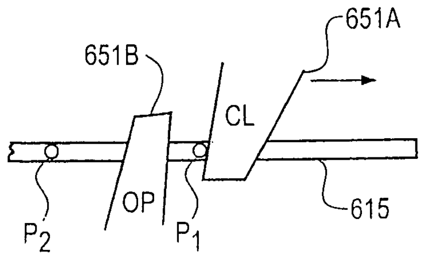


FIG. 113C

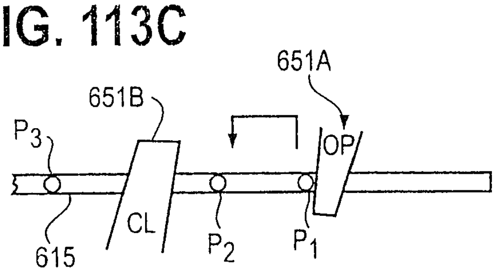


FIG. 113D

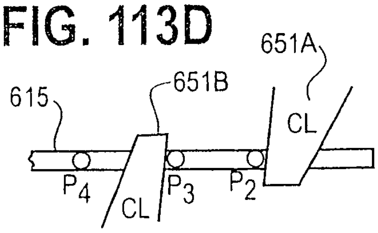


FIG. 113E

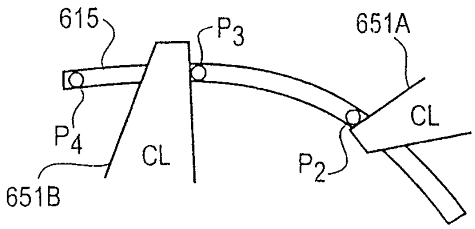


FIG. 114

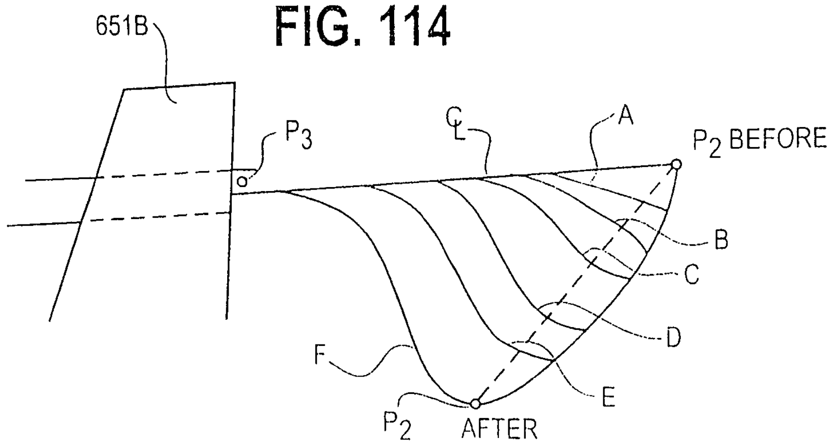

FIG. 115A

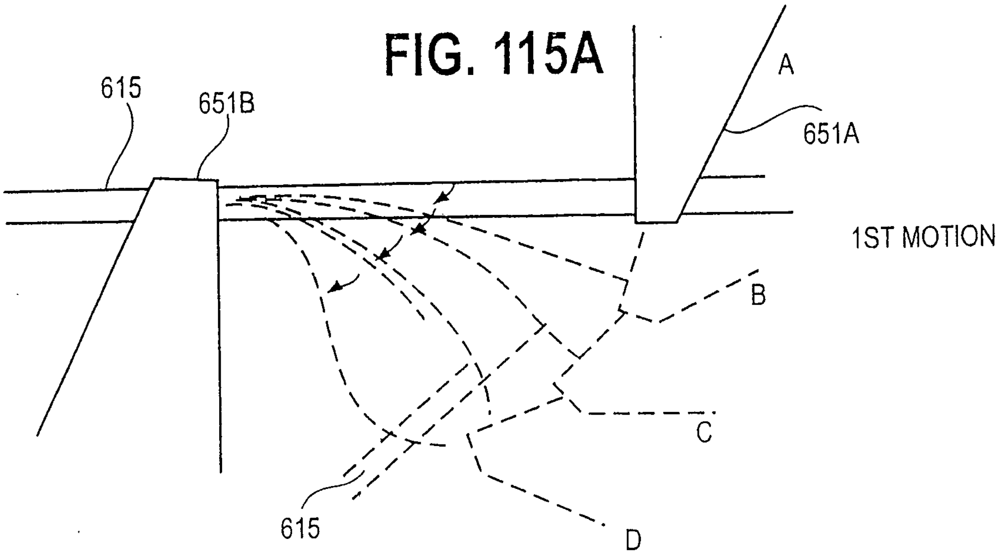


FIG. 115B

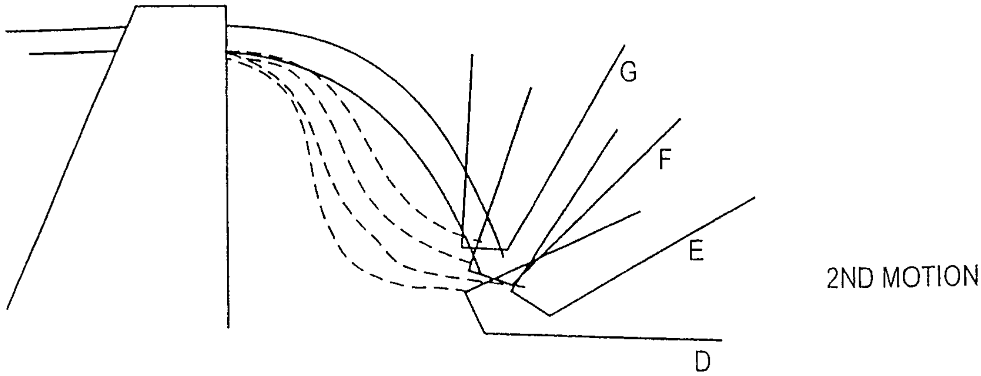


FIG. 115C

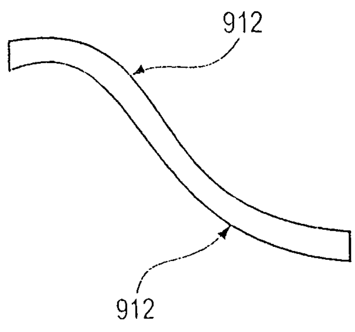

FIG. 116A

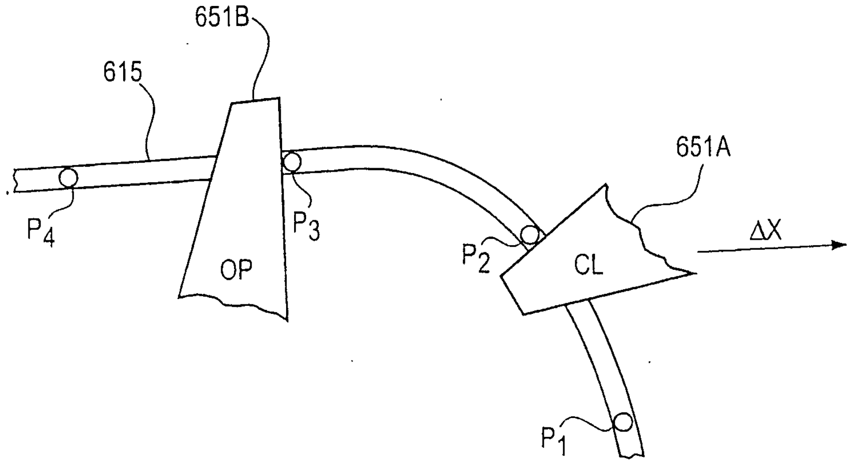


FIG. 116B

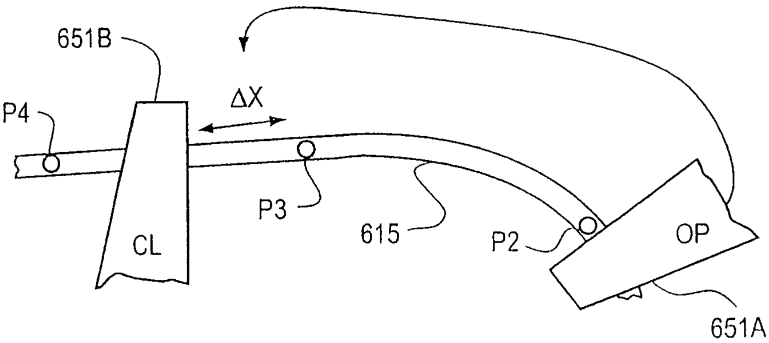


FIG. 116C

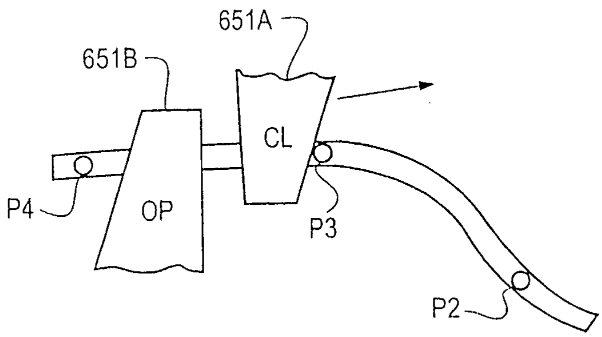


FIG. 116D

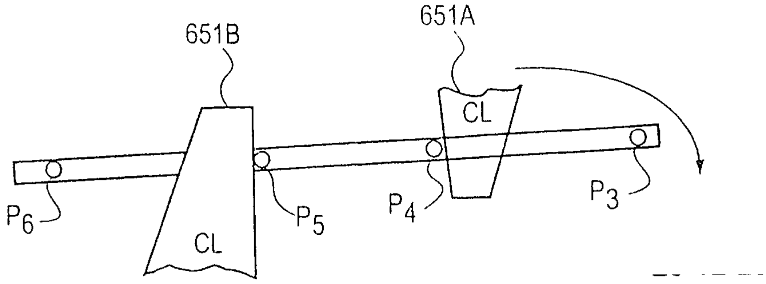

FIG. 117

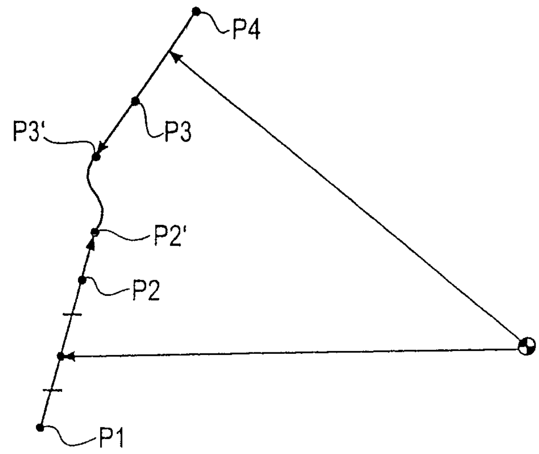


FIG. 118

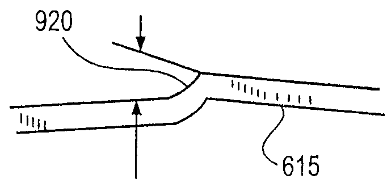


FIG. 119

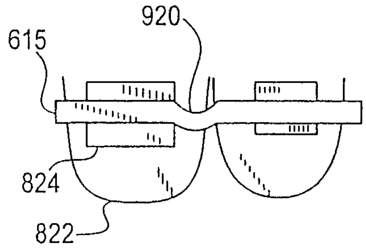


FIG. 120

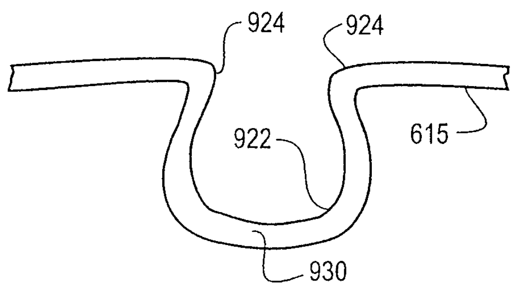


FIG. 121A

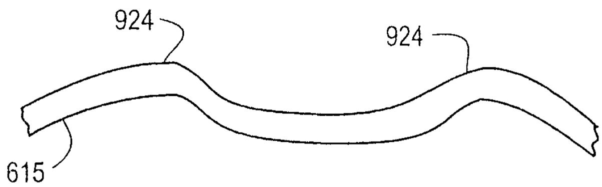


FIG. 121B

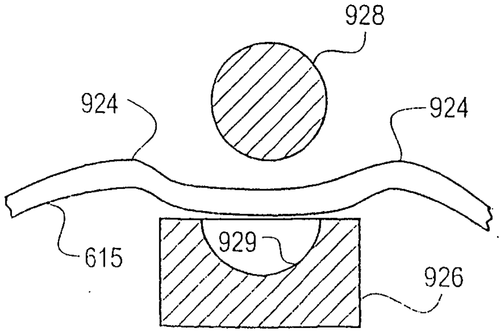

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5431562 A, Andreiko
- US 4837732 A
- US 4575805 A, Brandestini and Moermann
- US 5372502 A, Massen
- US 5027281 A, Rekow
- US 4656860 A, Orthuber
- WO 09254755 A, Rudger Rubbert
- WO 091560131 A
- US 09673863 B
- US 5155597 A, Lareau
- US 09451609 B, Sachdeva
- US 45160999 A